# EUROPEAN PATENT SPECIFICATION

(11) **EP 3 619 204 B1**
(45) Date of publication and mention of the grant of the patent: **01.10.2025**
(21) Application number: 18795097.7
(22) Date of filing: 03.05.2018
(51) Int. Cl.: C07D 403/04, C07D 403/14, C07D 401/14, A61K 31/33, A61P 11/06

(54) **COMPOUNDS FOR THE TREATMENT OF RESPIRATORY DISEASES**
VERBINDUNGEN ZUR BEHANDLUNG VON ATEMWEGSERKRANKUNGEN
COMPOSÉS POUR TRAITER DES MALADIES RESPIRATOIRES

(30) Priority: 03.05.2017 AU 2017901611; 05.05.2017 AU 2017901647
(43) Date of publication of application: 11.03.2020
(73) Proprietor: Tianli Biotech Pty Ltd, Melbourne, VIC 3010 (AU)
(72) Inventor: WILLIAMS, Spencer, The University of Melbourne, Victoria 3010 (AU); SHAH, Sayali, The University of Melbourne, Victoria 3010 (AU); HAKKI, Zalihe, The University of Melbourne, Victoria 3010 (AU); STEWART, Alastair, The University of Melbourne, Victoria 3010 (AU)
(74) Representative: HGF
(86) International application number: PCT/AU2018/050404
(87) International publication number: WO 2018/201192

(56) References cited:
- WO-A1-2014/023271
- WO-A1-2016/149756
- WO-A1-96/40143
- WO-A1-99/32121
- US-A- 5 593 991
- DATABASE REGISTRY [online] CHEMICAL ABSTRACTS SERVICE, COLUMBUS, OHIO, US; 5 October 2011 (2011-10-05), XP002800942, retrieved from STN Database accession no. 1349012-04-9
- DATABASE PubChem Compound [online] 25 October 2006 (2006-10-25), XP002800943, retrieved from NCBI Database accession no. 9959143
- KIM, DAE-KEE ET AL.: "Synthesis and biological evaluation of trisubstituted imidazole derivatives as inhibitors of p38a mitogen-activated protein kinase", BIOORGANIC & MEDICINAL CHEMISTRY LETTERS, vol. 18, 2008, pages 4006 - 4010, XP022852885

## Description

This application claims priority from Australian provisional applications 2017901611 and 2017901647.

### Field of the invention

The present invention provides imidazole-based compounds which show potential in the treatment of asthma and related conditions.

### Background of the invention

Asthma is a syndrome that encompasses various different types of diseases, which vary in their severity and in their causes and triggers. The common features of the asthma syndrome are reversible airway obstruction, airway hyper-responsiveness and airway inflammation, with infiltration of the airway wall by eosinophils and T lymphocytes the most prominent features in addition to activation of mast cells.

Current asthma medications include short- and long-acting β₂-adrenoceptor selective agonists (SABA and LABA) and inhaled corticosteroids (ICS). Ultra-LABA are now also available. Short and long-acting muscarinic receptor antagonists (SAMA and LAMA) are used in some patients, usually in combination with other bronchodilators and anti-inflammatory drugs, especially ICS. Leukotriene receptor antagonists (LTRA) may also be added to different therapeutic regimens. More recently, the monoclonal antibody mepolizumab, which neutralises a chemoattractant for eosinophils, interleukin-5, has been shown to have benefit additional to the ICS and LABA combinations in selected patients. Nevertheless, for severe asthma in particular, patients are still symptomatic and have periodic worsening of disease, referred to as exacerbations. There is a considerable unmet need in the drug treatment of severe asthma. These asthma exacerbations are considered to be caused by respiratory viral infection of the lower respiratory tract in the majority of cases. The viruses that cause these exacerbations include respiratory syncytial virus, influenza virus and rhinoviruses, which infect the respiratory epithelium. The epithelium of asthmatic individuals is considered to be especially susceptible to such infections and is implicated in the worsening of the inflammatory response.

Research has shown that TGF-β is able to compromise the effectiveness of ICS. Furthermore, the inventors have demonstrated that viral infection of the airway epithelium compromises ICS activity through induction of TGF-β activity. Drug targeting of TGF-β carries risk of autoimmune and mitral valve defects. The inventors surprisingly identified casein kinase 1δ/ε as a mediator of TGF-β induced ICS insensitivity, using the compound PF670462 (WO2016/149756), and demonstrated the utility of this agent to reverse steroid insensitivity.

Novel compounds that can treat respiratory diseases, that can be administered to the lungs, and that can show reduced systemic effects when compared to current medications would be desirable.

### Summary of the invention

The present inventors have found a series of CK1δ/ε inhibitors that have been designed to achieve an increase in LogP when compared to PF670462. Increased LogP is a feature of inhalational drugs that is strongly associated with increased pulmonary residence time, and therefore with a greater dose interval, often affording twice or once daily administration and simultaneously minimising systemic exposure. Generally speaking, and surprisingly, the series has shown similar activity to PF670462 in asthma-related assays.

These results are surprising because as is known to those skilled in the art it is impossible to predict the effect on activity of substantial differences in physical properties between an active compound and its analogues.

Further, an N-methyl analogue has been shown to be more potent than PF670462 and maintains a wide spectrum of activity, including the ability to prevent TGFβ-induced ICS insensitivity and the suppression of TGF-β and TNF-α induced fibrogenic and inflammogenic growth factors and cytokines.

Any references in the description to methods of treatment refer to the compounds and pharmaceutical compositions of the present invention for use in a method for treatment of the human (or animal) body by therapy.

Embodiments not encompassed by the claims (e.g. compound of formula la) are provided for reference purposes.

Accordingly, in one aspect, the present invention provides a compound of formula (I) or a salt, solvate, or polymorph thereof as defined in the appended claims:

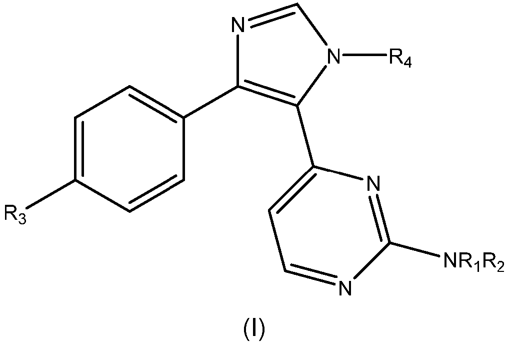

In another aspect, there is provided a method of treating or preventing a respiratory disease in a subject in need thereof, the method comprising administering to the subject a therapeutically effective amount of a compound of formula (I) or a salt, solvate, or polymorph thereof, thereby treating or preventing a respiratory disease in a subject.

There is further provided a compound of formula (I) or a salt, solvate, or polymorph thereof for use in the treatment or prevention of a respiratory disease in a subject.

The respiratory disease may be selected from asthma, chronic obstructive pulmonary disease, interstitial lung diseases (such as idiopathic pulmonary fibrosis) and other conditions relating to tissue remodelling, primary or secondary lung tumour, hayfever, chronic and acute sinusitis, and chronic and acute viral, fungal and bacterial infections of the respiratory tract.

In another aspect, there is provided a method of improving respiratory function in a subject in need thereof, the method comprising administering to the subject a therapeutically effective amount of a compound of formula (I) or a salt, solvate, or polymorph thereof, thereby improving respiratory function of the subject.

There is further provided a compound of formula (I) or a salt, solvate, or polymorph thereof for use in improving respiratory function in a subject.

The improvement in respiratory function may be selected from a decrease in the level of constriction of the lungs, a decrease in the elastic stiffness of the respiratory system, and/or an increase in the ease with which the respiratory system can be extended. Preferably, the improvement is selected from a decrease in the level of constriction of the lungs, and a decrease in the elastic stiffness of the respiratory system. In yet another aspect, there is provided a composition comprising a compound according to formula (I) or a salt, solvate, or polymorph thereof, and a pharmaceutically acceptable excipient.

The composition may be formulated for oral administration or administration by inhalation or injection.

Use of a compound or composition of the invention in the preparation of medicaments for the treatment or prevention of a respiratory disease in a subject is also described.

As used herein, except where the context requires otherwise, the term "comprise" and variations of the term, such as "comprising", "comprises" and "comprised", are not intended to exclude further additives, components, integers or steps.

Reference to any prior art in the specification is not an acknowledgment or suggestion that this prior art forms part of the common general knowledge in any jurisdiction or that this prior art could reasonably be expected to be understood, regarded as relevant, and/or combined with other pieces of prior art by a skilled person in the art.

Further aspects of the present invention and further embodiments of the aspects described in the preceding paragraphs will become apparent from the following description, given by way of example and with reference to the accompanying drawings.

### Brief description of the drawings

**Figure 1****.** The effects of SS9-010 are contrasted with those of PF670462 in an assay in BEAS-B cells measuring the induction of the glucocorticoid-regulated gene, SCNN1A encoding the protein epithelial sodium channel alpha subunit.
**Figure 2****.** In the same experiment as described in Figure 1, the expression of the fibrogen plasminogen activator inhibitor-1 (PAI-1) was measured.
**Figure 3****.** Exposure of BEAS-2B cells to TNF-α with(out) 30 min pretreatment with either SS9-010 or PF670462 (1-10µM).
**Figure 4****.** In A549 cells, pretreatment with (0.1-10 µM) of either PF670462 or SS9-010 concentration-dependently inhibited PAI-1 and CTGF mRAN levels and restored TGFβ (100 pM)-induced suppression of E cadherin mRNA levels.
**Figure 5****.** Shows the effect of the anti-fibrotic agent nintedanib by way of contrast with the extent and potency of the actions of PF670462.
**Figure 6****.** The effects of the CK1 inhibitors, PF670462 and SS9-010 (1-10 µM) on TNF-α (10ng/ml)-induced increases in expression of IL-8 mRNA and on the level of immunoreactive IL-8 in the culture supernatant are shown.
**Figure 7****.** The expression of CSF-2 (GM-CSF) and its immunoreactive level in the supernatant from the same experiment depicted in Figure 6.
**Figure 8****.** Defines a number of known compounds which are relevant to the methods of the present invention.
**Figure 9****.** Effects of inhaled SS9-010 on bleomycin-induced BAL protein leakage, BAL cell recruitment, as well as lung fibrogenic gene expression in mouse lungs. Female C57BI/6 mice were transnasally administered bleomycin or saline on day 0. SS9-010 was administered day -1 to day 3 through daily inhalation of aerosol of the concentration of 1mg/mL for a period of 15 min once daily. Bronchoalveolar lavage (BAL) protein and BAL cell number were assessed. Lung fibrogenic gene expression was also assessed on day 3. Data are presented as mean ± SEM (n=6).
**Figure 10****.** Effects of intraperitoneally injected SS9-010 on bleomycin-induced BAL protein leakage, BAL cell recruitment, as well as lung fibrogenic gene expression in mouse lungs. Female C57BI/6 mice were intraperitoneally administered bleomycin or saline on day 0. SS9-010 (3 or 10mg/kg/day, ip) was administered from day -1 to 3. Bronchoalveolar lavage (BAL) protein and BAL cell number were assessed. Lung fibrogenic gene expression was also assessed on day 3. Data are presented as mean ± SEM (n=4 sal, n=5 other groups).

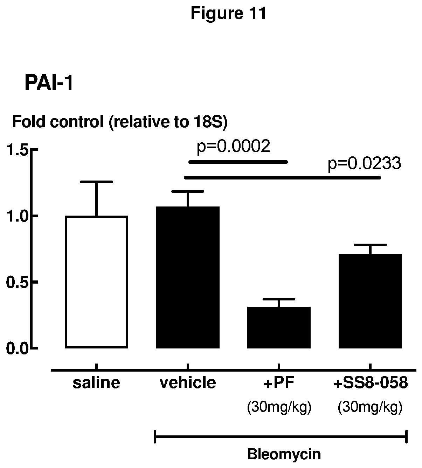

**Figure 11****.** The effects of oral (gavage) PF670462 or SS87-058 were investigated in female C57BL6 mice, with a focus on liver gene expression. Administration of 30 mg/kg/po of either PF670462 or SS8-058 throughout a 4 day period resulted in significant reductions in the baseline levels of the mRNA encoding fibrogenic gene products, PAI-1 and TMP1, 3 days after a transnasal dose of bleomycin on day 0. Data are presented as mean ± SEM (n=4).
**Figure 12****.** Airway hyperresponsiveness (AHR) in response to inhaled methacholine (MCh) measured in sham- or ovalbumin (OVA)-sensitized BALB/c mice that received inhalation of SS9-010 (estimated deposited dose of 1 µg) or saline. Resistance (Rn), elastance (E) and compliance (C) were recorded to assess lung mechanical changes. Data are presented as mean ± SEM (n=6).

### Detailed description

It will be understood that the invention disclosed and defined in this specification extends to all alternative combinations of two or more of the individual features mentioned or evident from the text or drawings. All of these different combinations constitute various alternative aspects of the invention.

Reference will now be made in detail to certain embodiments of the invention. While the invention will be described in conjunction with the embodiments, it will be understood that the intention is not to limit the invention to those embodiments.

One skilled in the art will recognize many methods and materials similar or equivalent to those described herein, which could be used in the practice of the present invention. The present invention is in no way limited to the methods and materials described. It will be understood that the invention disclosed and defined in this specification extends to all alternative combinations of two or more of the individual features mentioned or evident from the text or drawings. All of these different combinations constitute various alternative aspects of the invention.

For purposes of interpreting this specification, terms used in the singular will also include the plural and *vice versa.*

The present invention provides a compound of formula (I) or a salt, solvate, or polymorph thereof as defined in appended claim 1:

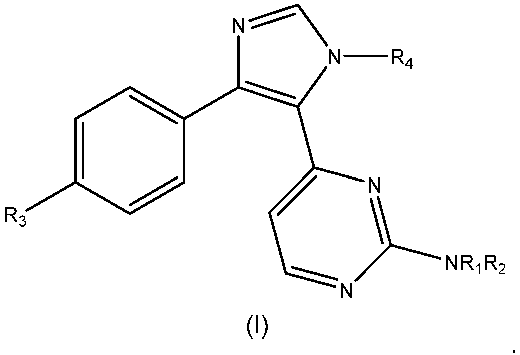

In one embodiment, the present invention provides a compound of formula (I) wherein R₁ is C₂₋₆alkynyl. R₁ may be selected from C₂alkynyl, C₃alkynyl and C₄alkynyl. In one embodiment, when R₁ is C₂₋₆alkynyl, R₂ is H.

In one embodiment, the present invention provides a compound of formula (I) wherein R₁ is C₃₋₆cycloalkyl. R₁ may be selected from cyclobutyl, cyclopentyl and cyclohexyl. In one embodiment, when R₁ is C₃₋₆cycloalkyl, R₂ is H.

In one embodiment, the present invention provides a compound of formula (I) wherein R₁ is C₃₋₅heterocyclyl. R₁ may be selected from an oxygen-containing heterocyclyl group, a nitrogen-containing heterocyclyl group, or a sulphur-containing heterocyclyl group, or a heterocyclyl group containing a combination of two or more oxygen, nitrogen and sulphur atoms. Examples include oxiranyl, thiiranyl 1,3-diazetidinyl, oxetanyl, thietanyl, pyrrolidinyl, pyrazolidinyl, imidazolidinyl, terahydrofuranyl, 1,3-dioxolanyl, tetrahydropyranyl, tetrahydrothipheneyl, and 1,2-and 1,3-oxathiolanyl. In one embodiment, when R₁ is C₃₋₅heterocyclyl, R₂ is H.

In one embodiment, R₃ is selected from halo and CH₃.

In one embodiment, R₃ is CH₃.

In one embodiment, R₃ is F, Cl, Br or I.

Preferably, R₃ is F.

In a preferred form, R₄ is selected from cyclopropyl, cyclobutyl, cyclopentyl, cyclohexyl and cycloheptyl.

In one embodiment, R₁, and R₄ are optionally substituted by one or more groups selected from OH, C₁₋₆alkoxy, halo, amino, mercapto and C₁₋₆alkyl. Also described is a compound of formula (la) or a salt, solvate, prodrug or polymorph thereof: 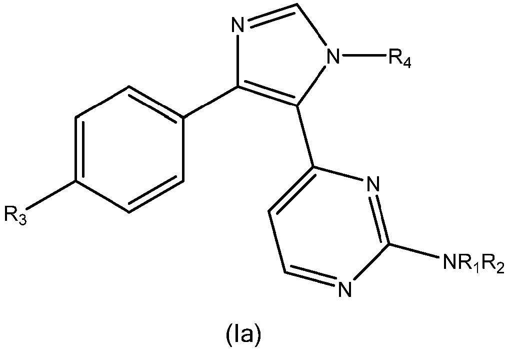 wherein:
R₁ and R₂ are
   (i) each independently selected from the group consisting of H, C₁₋₆alkyl, C₂₋₆alkynyl, C₁alkylC₆aryl, C₆aryl, C₃₋₆cycloalkyl and C₃₋₅heterocyclyl; or
   (ii) R₁ and R₂ together with the nitrogen atom to which they are attached form a heterocyclyl or heteroaryl group;
R₃ is selected from the group consisting of F, Cl, Sr, I, CH₃, OCH₃, OCF₂H, OCF₃, CO₂H, and CO₂C₁₋₁₀alkyl;
R₄ is selected from the group consisting of C₀₋₃alkylC₃₋₁₂cycloalkyl, C₁₋₁₂alkyl, C₁₋₁₀alkylC₆aryl, C₁₋₆alkylOC₁₋₆alkylC₆aryl, C₀₋₆alkylheteroaryl, and C₀₋₆alkylheterocyclyl;
wherein each of R₁, R₂, R₃ and R₄ is optionallly substituted.

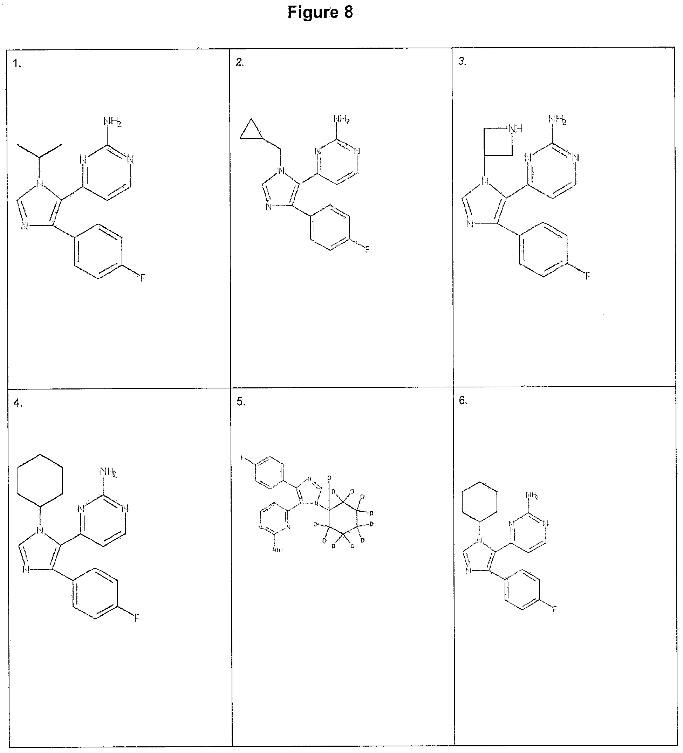

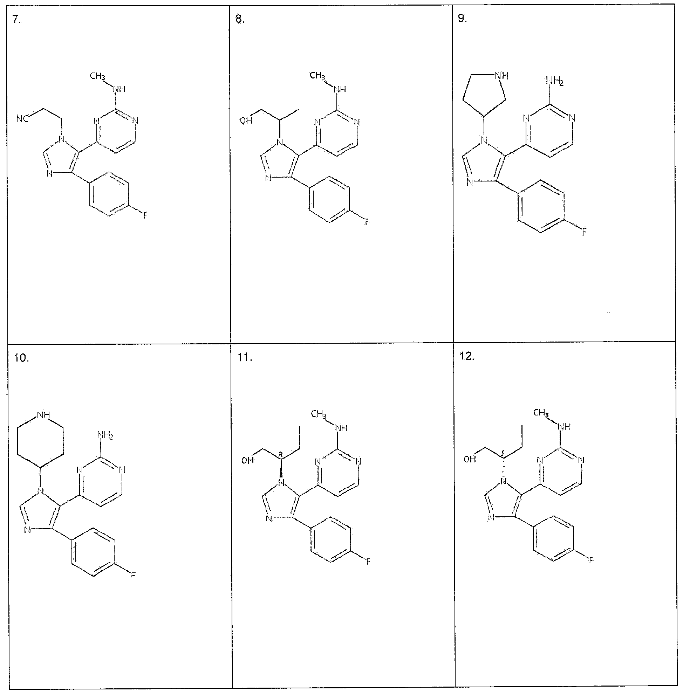

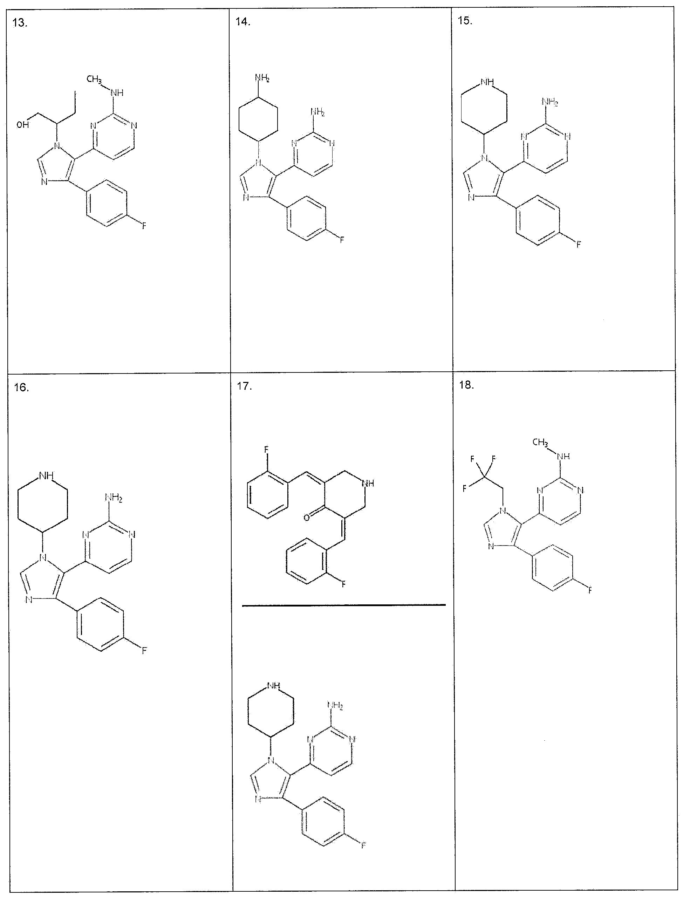

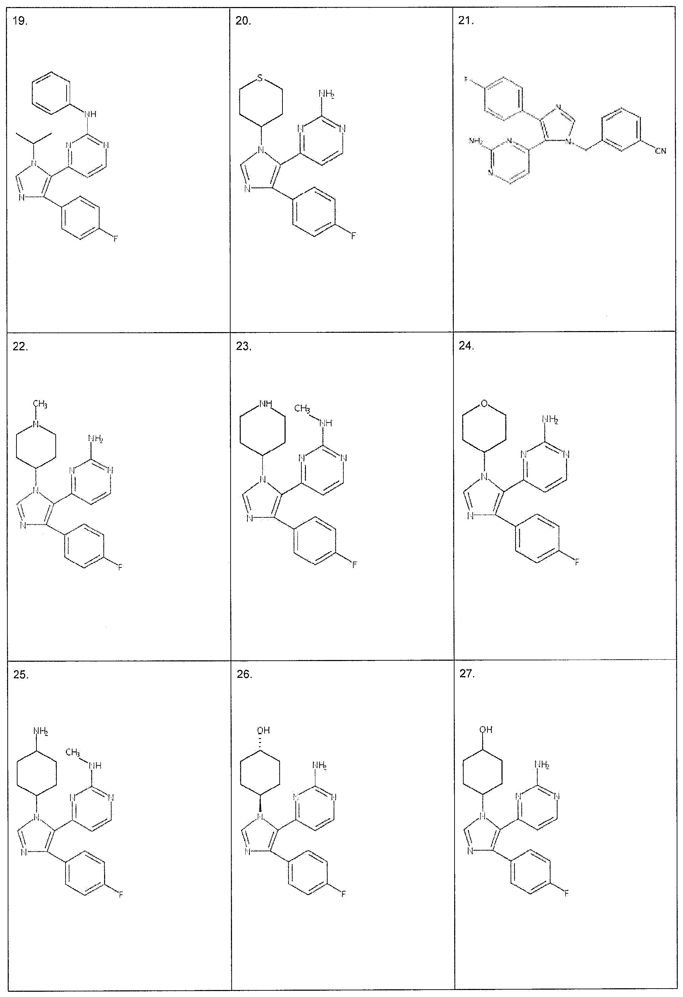

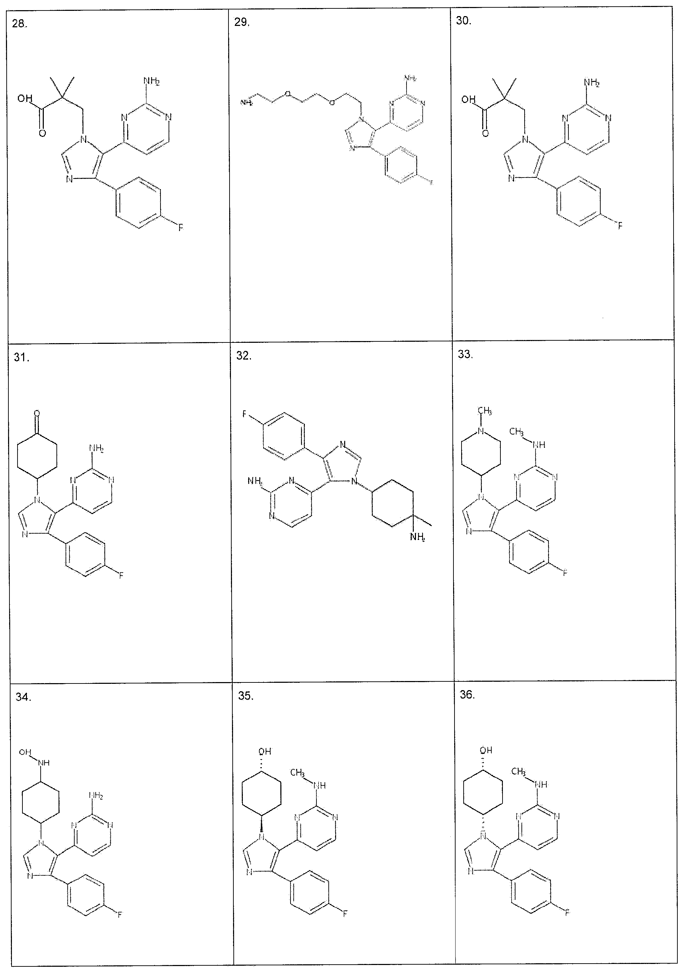

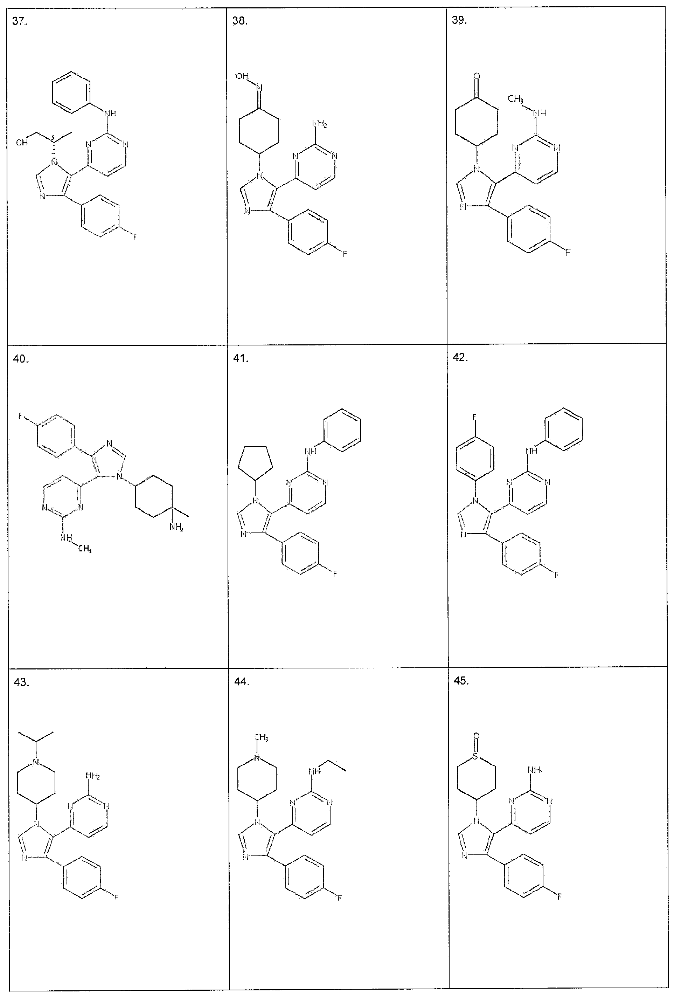

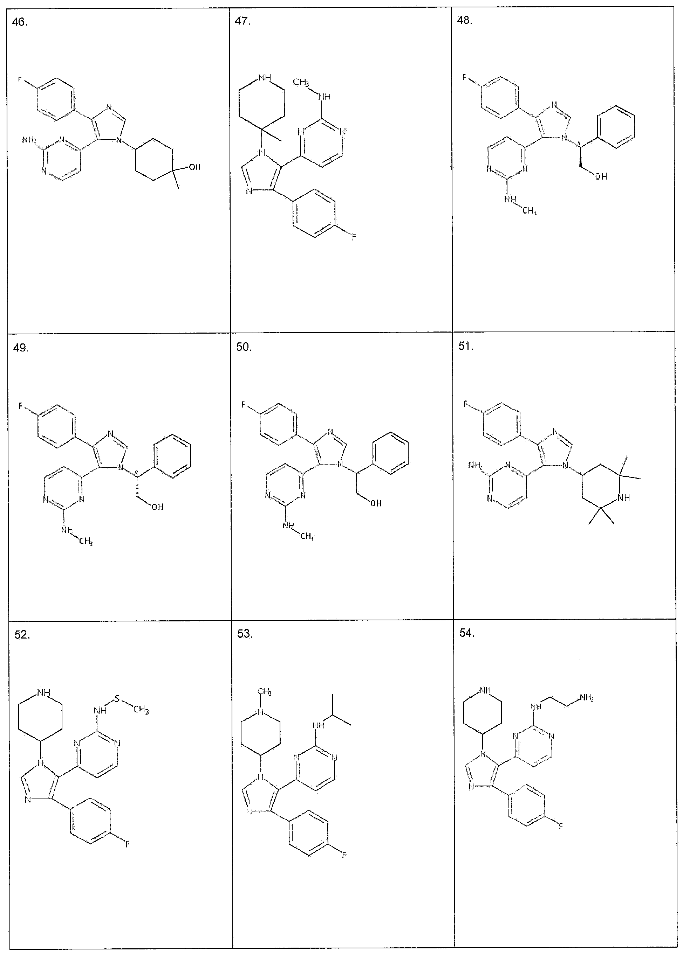

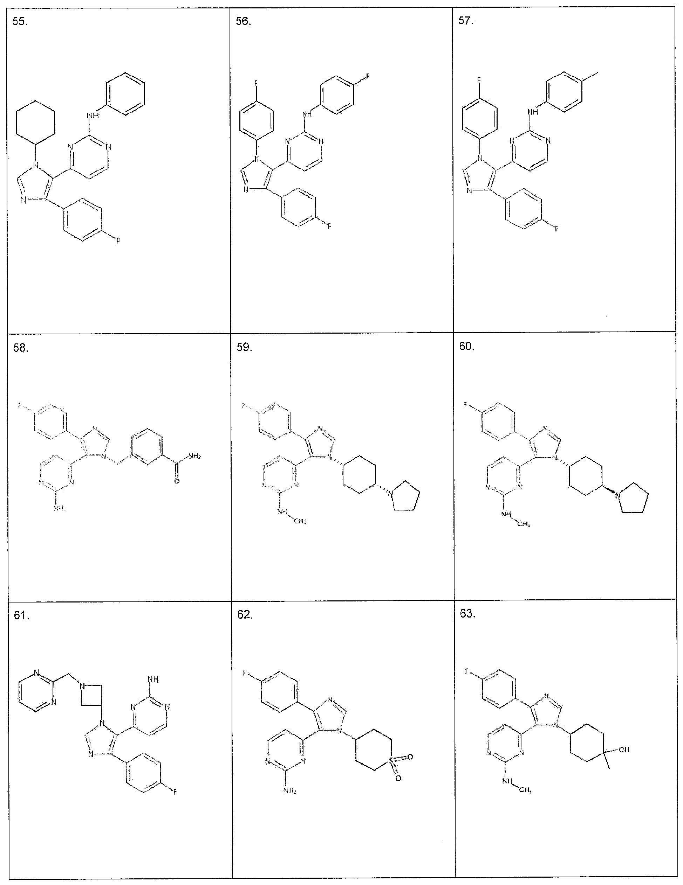

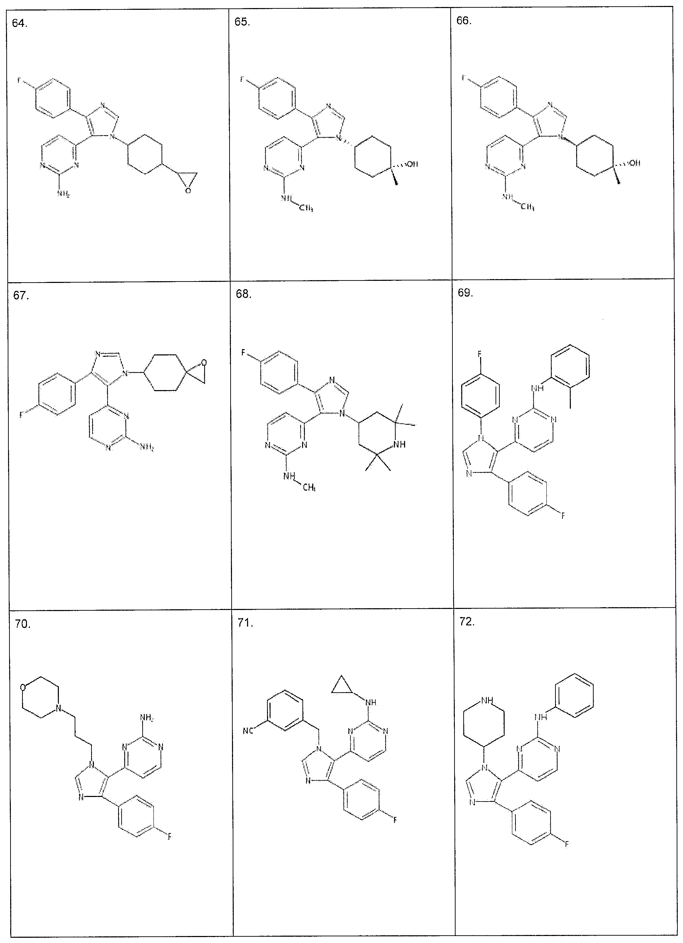

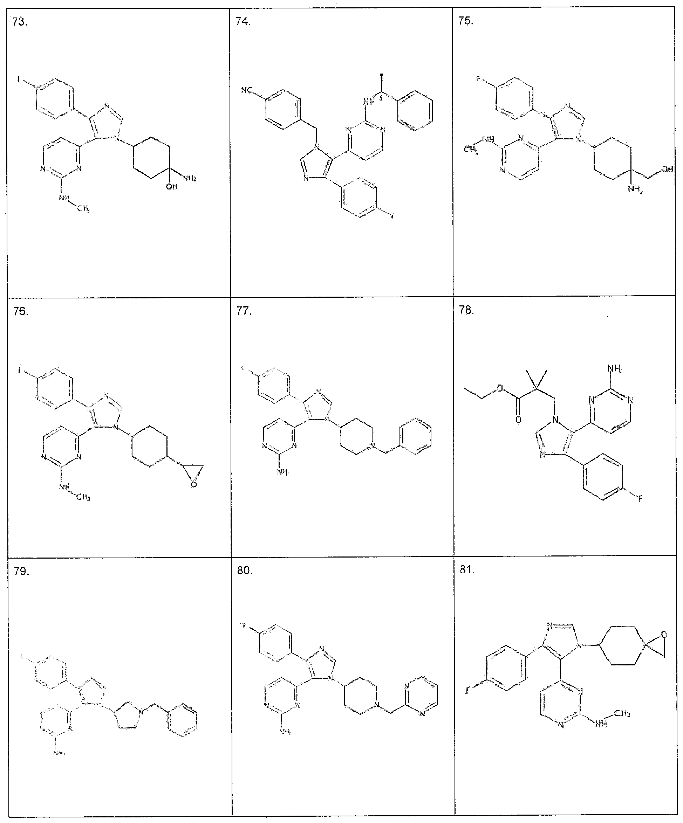

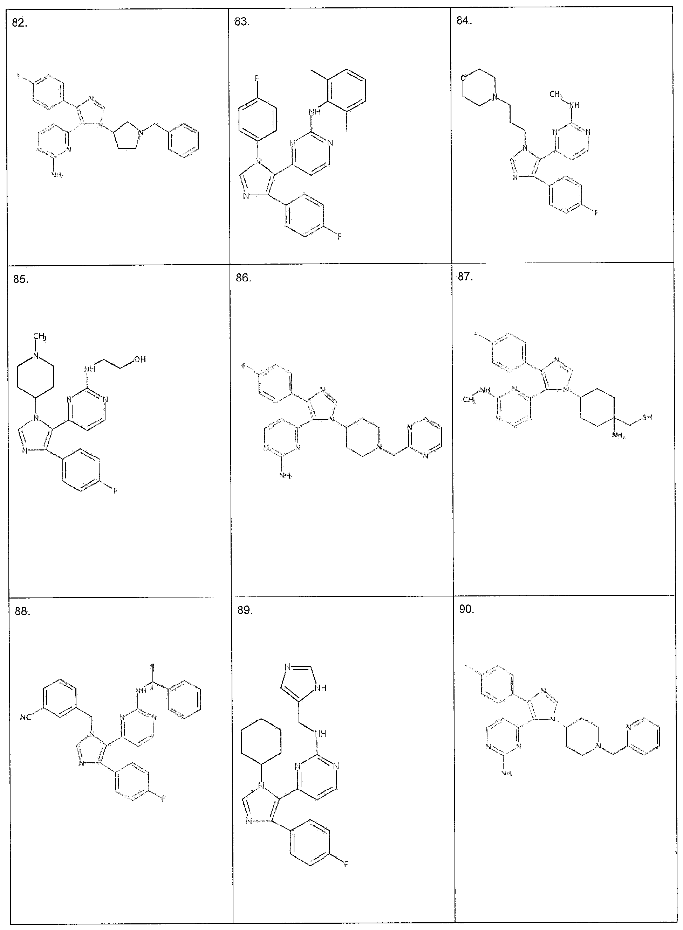

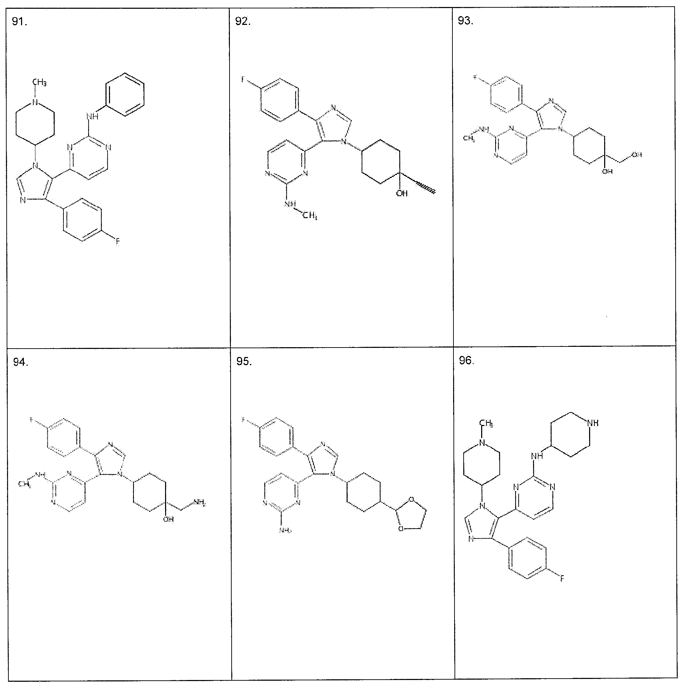

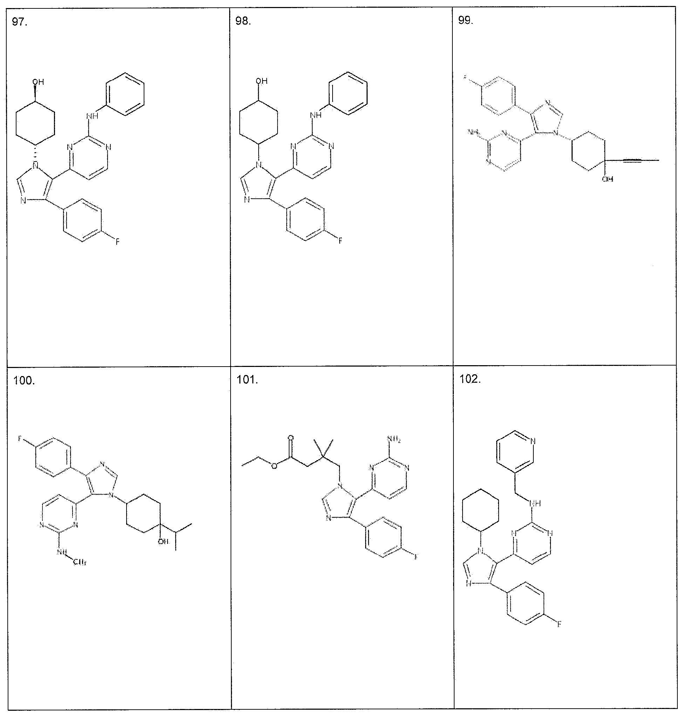

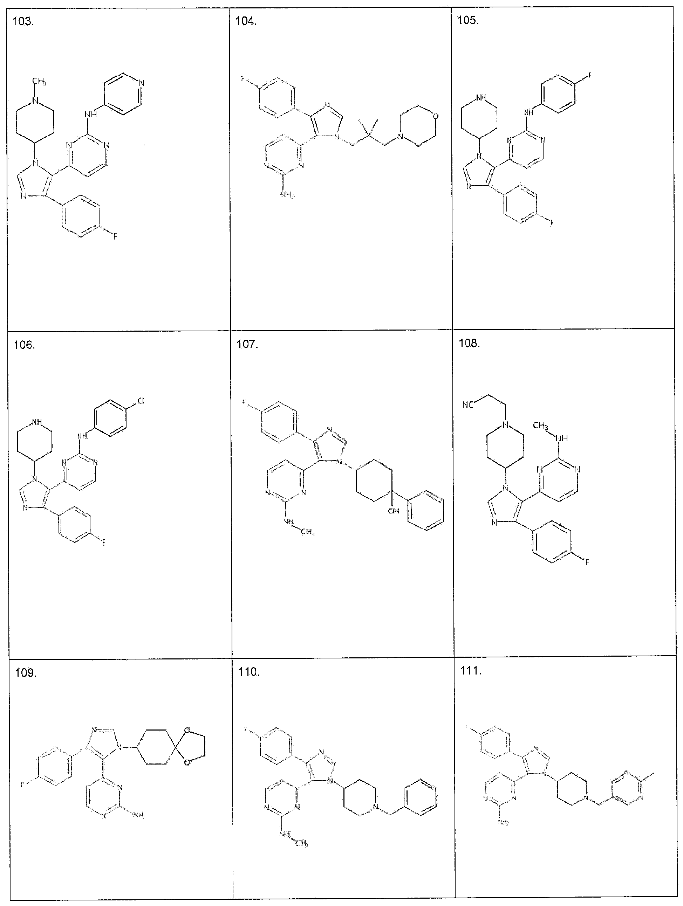

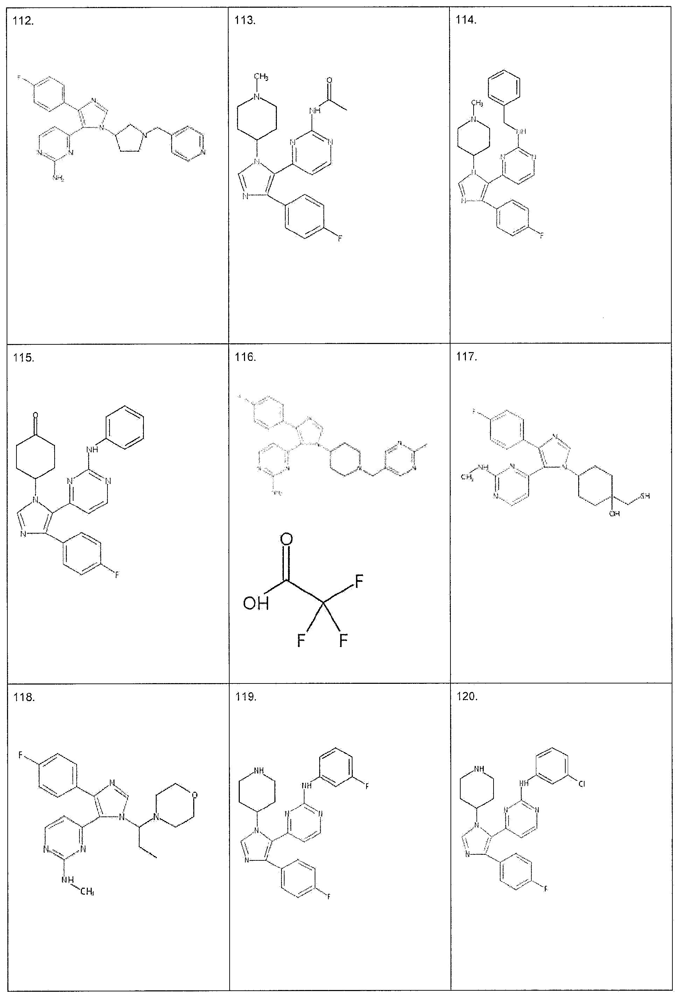

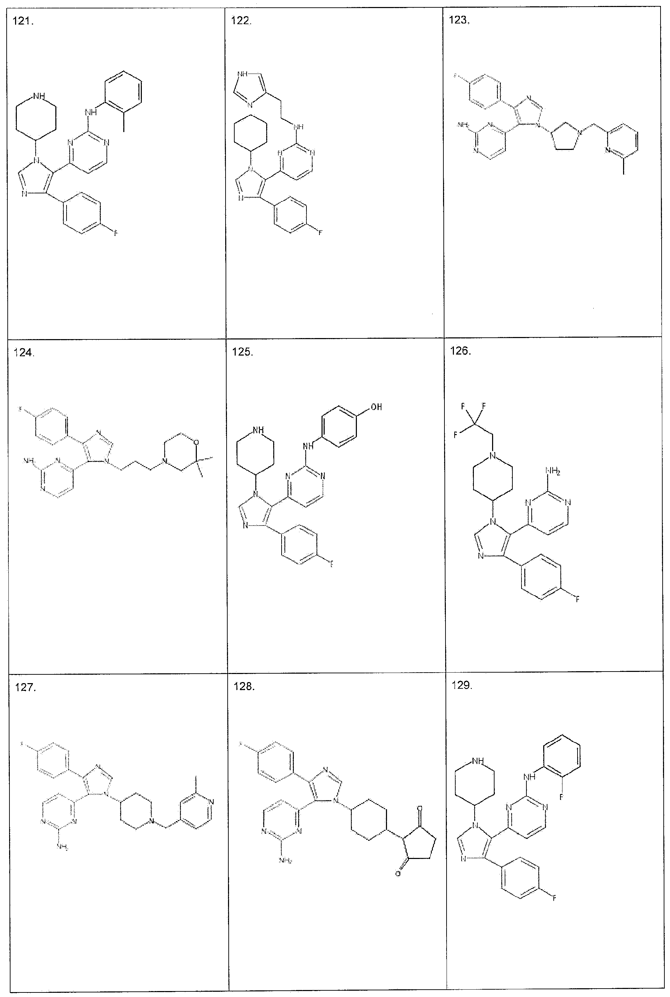

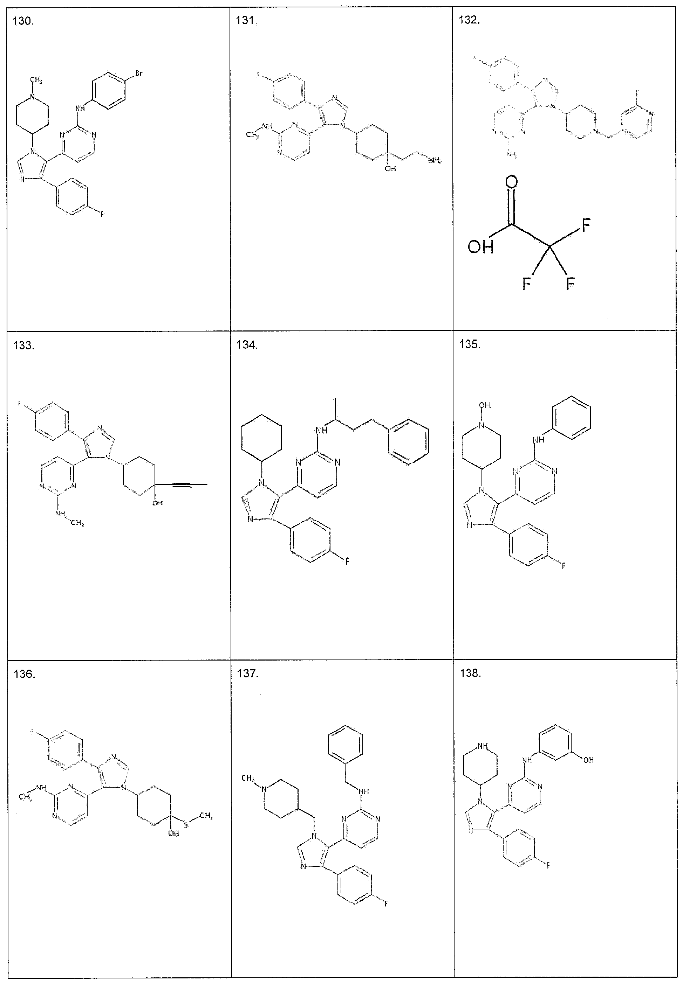

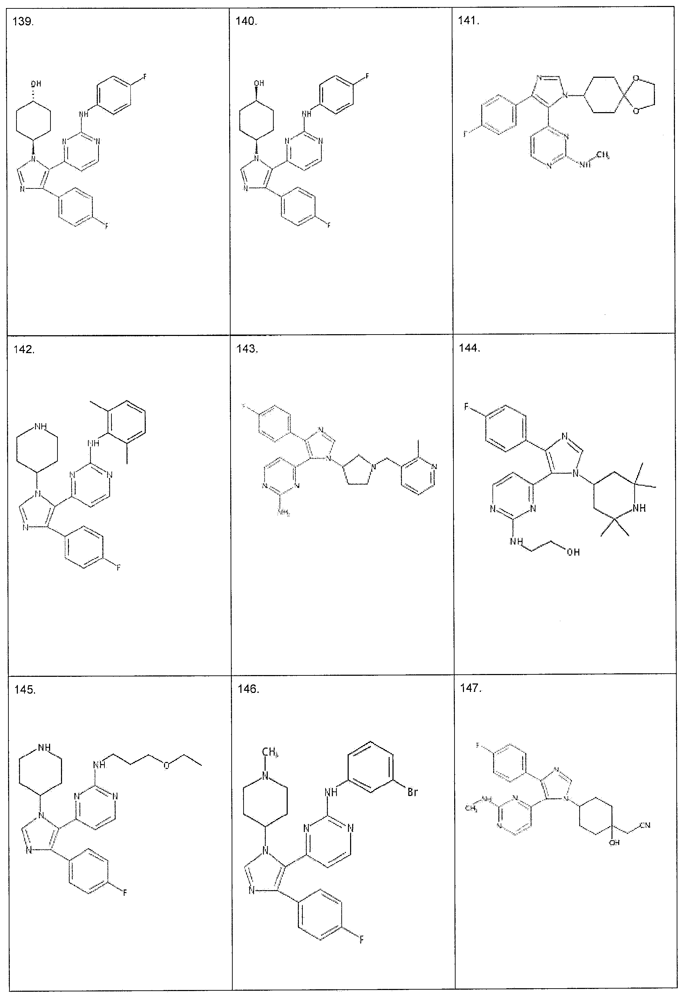

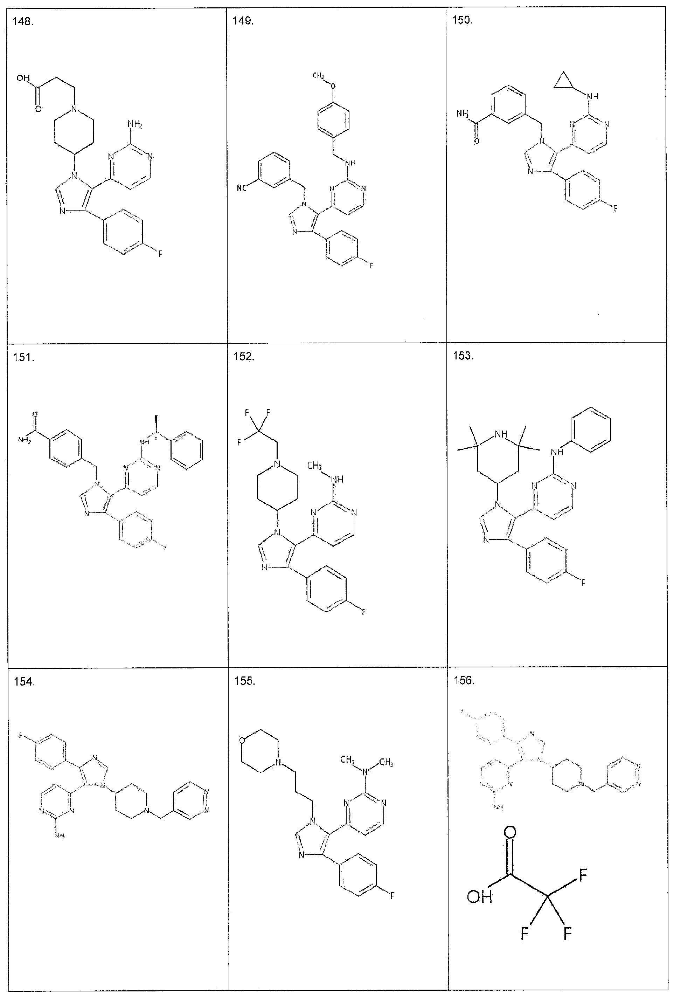

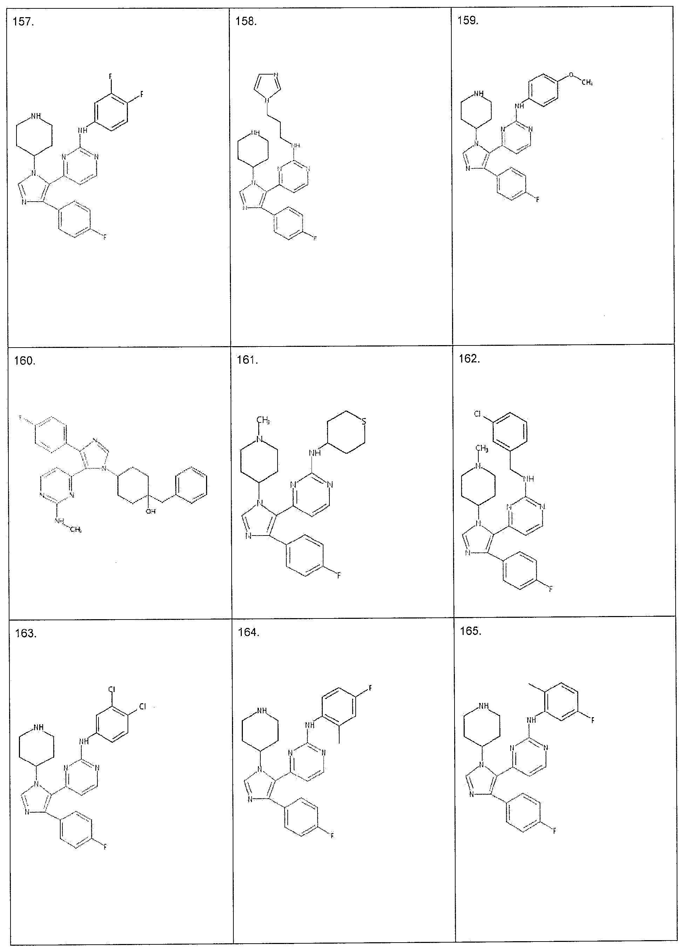

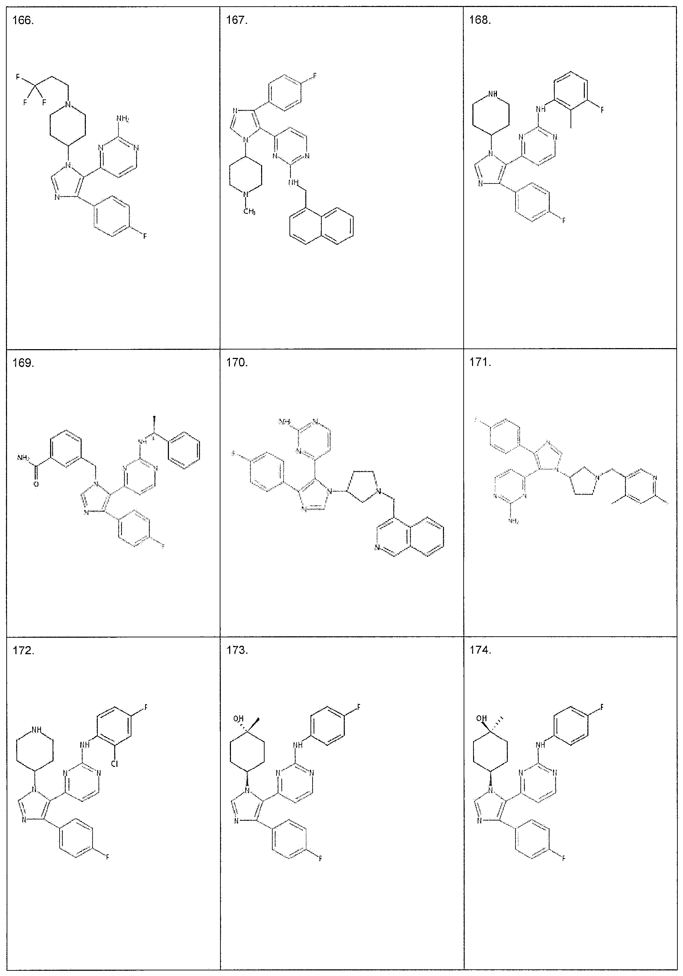

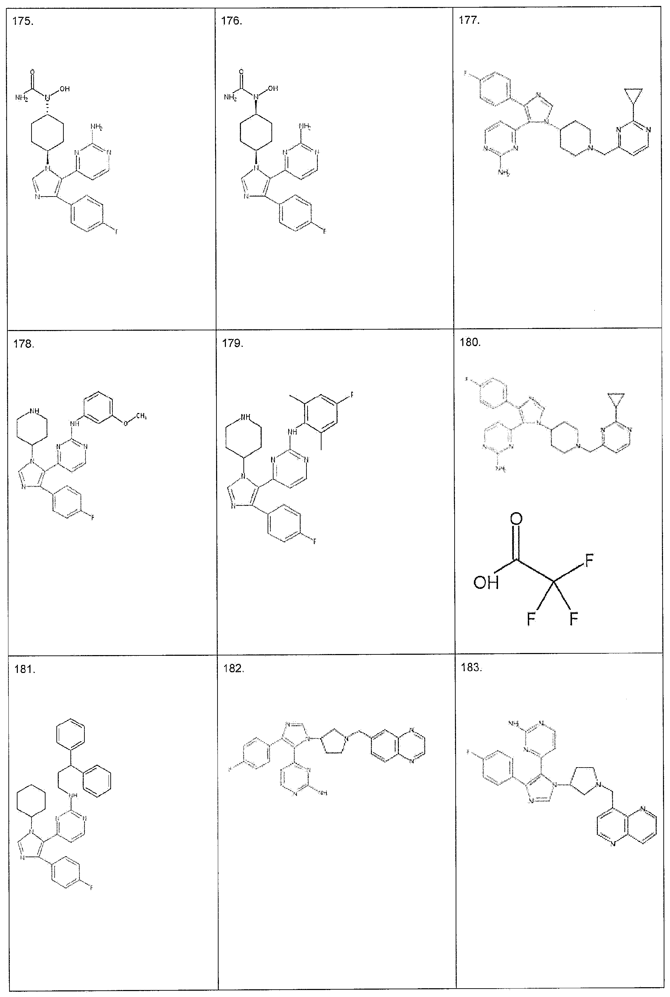

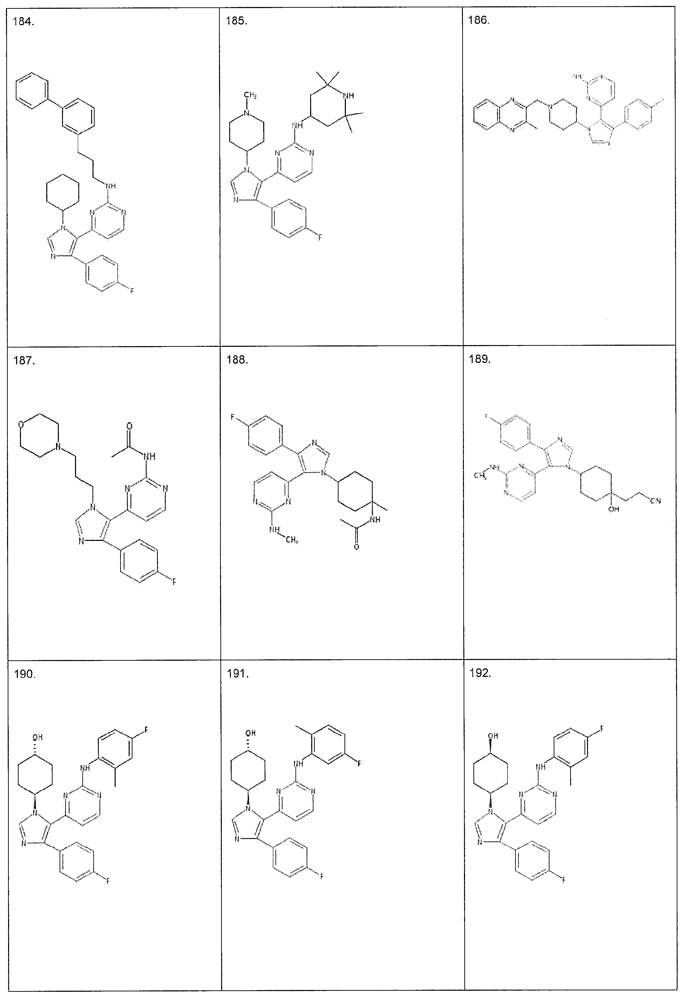

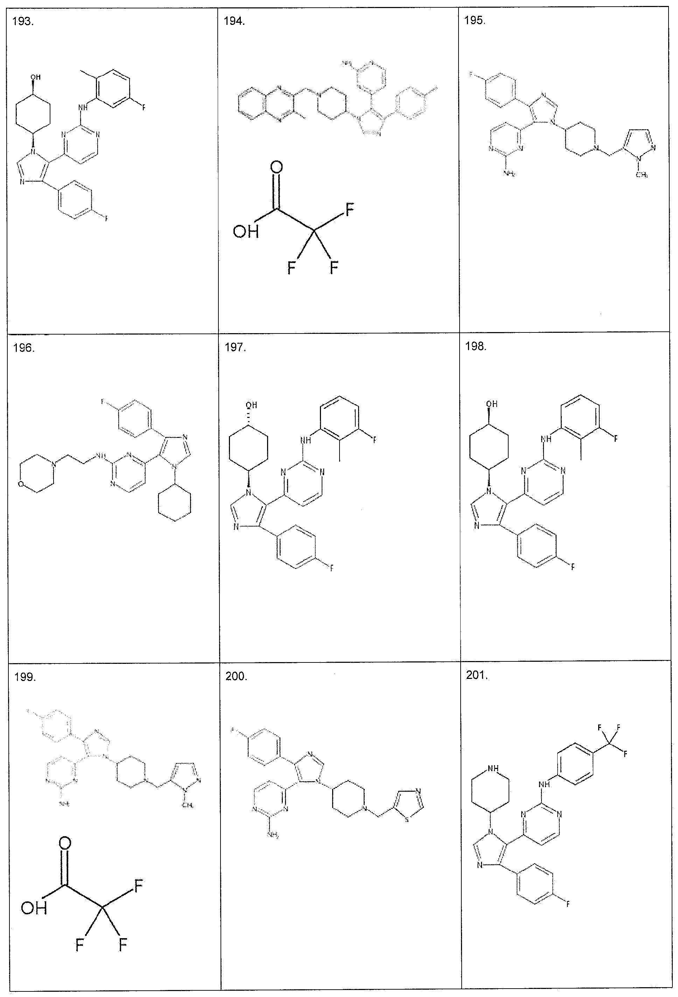

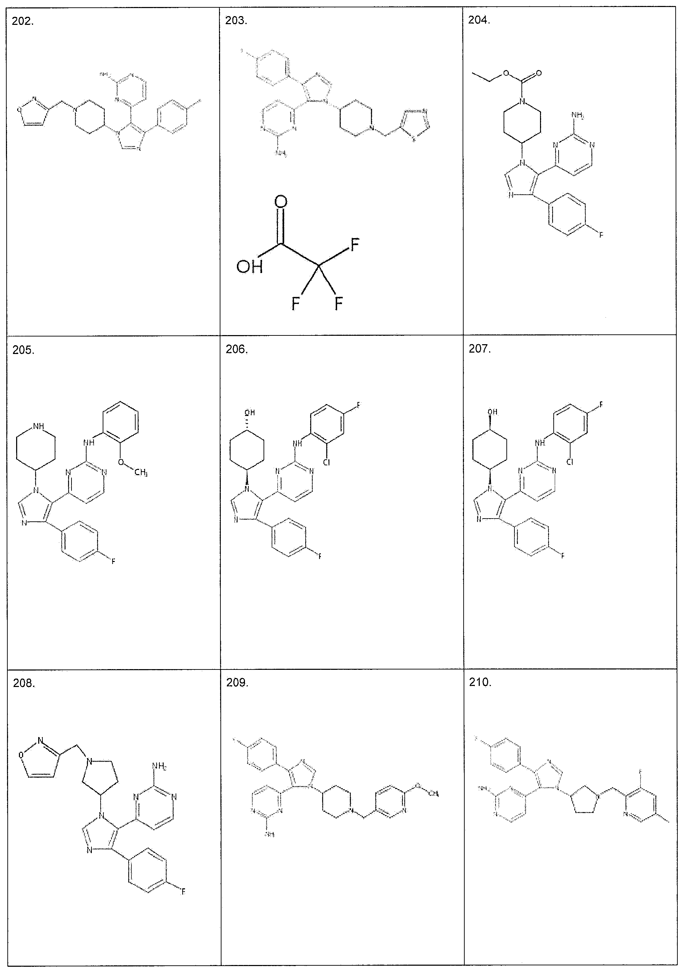

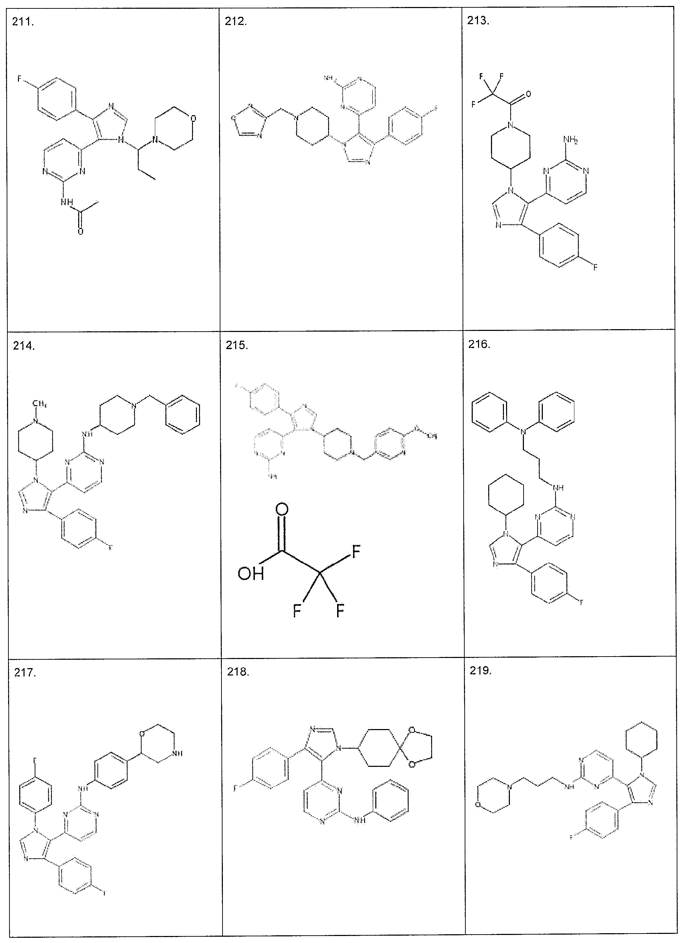

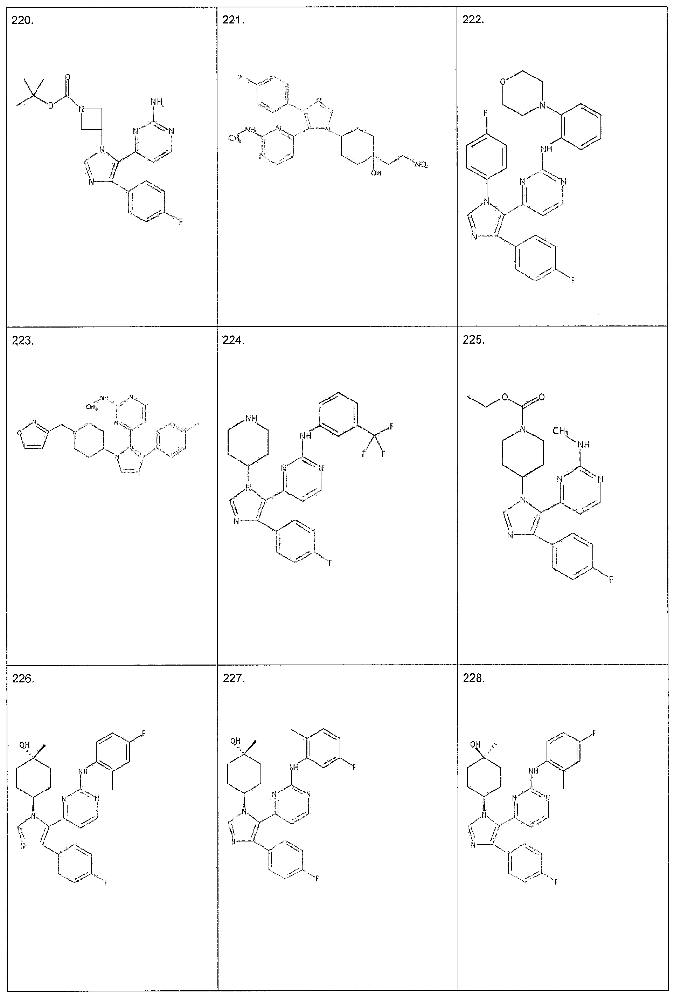

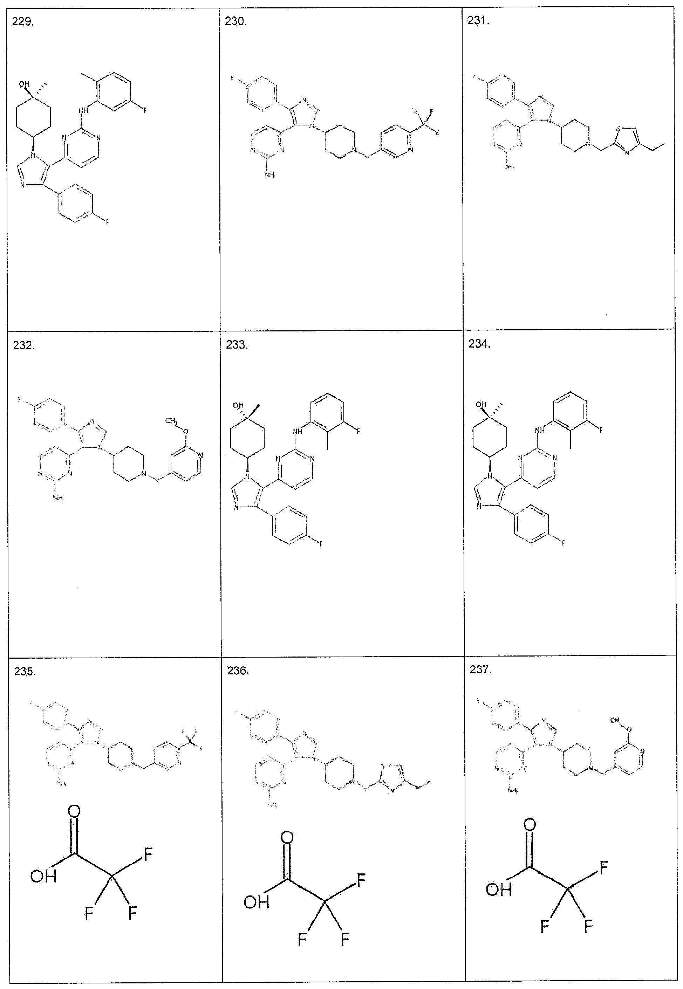

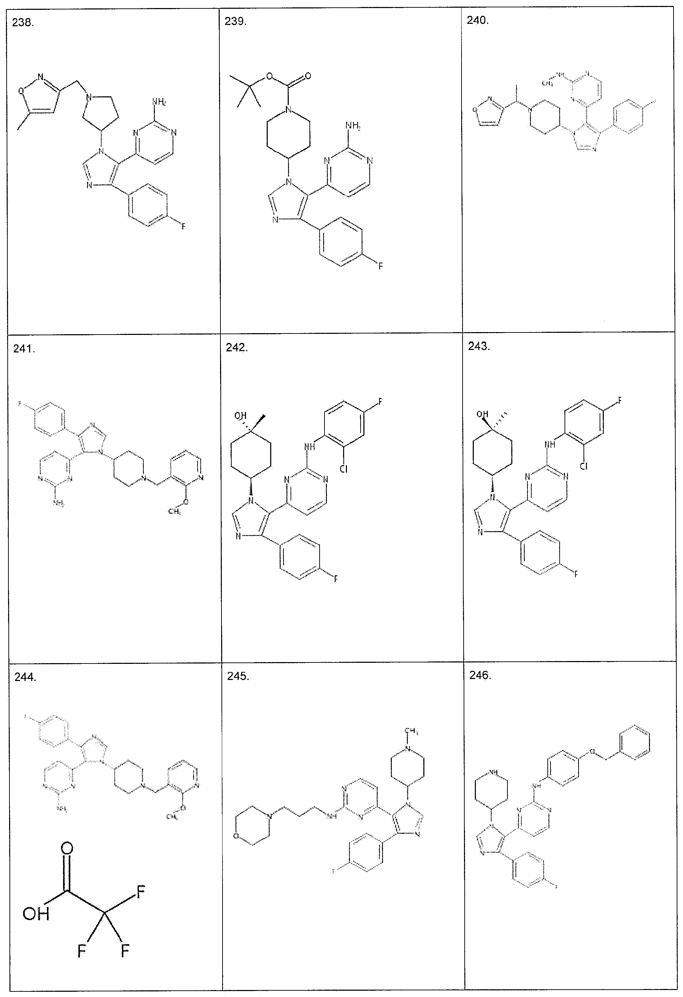

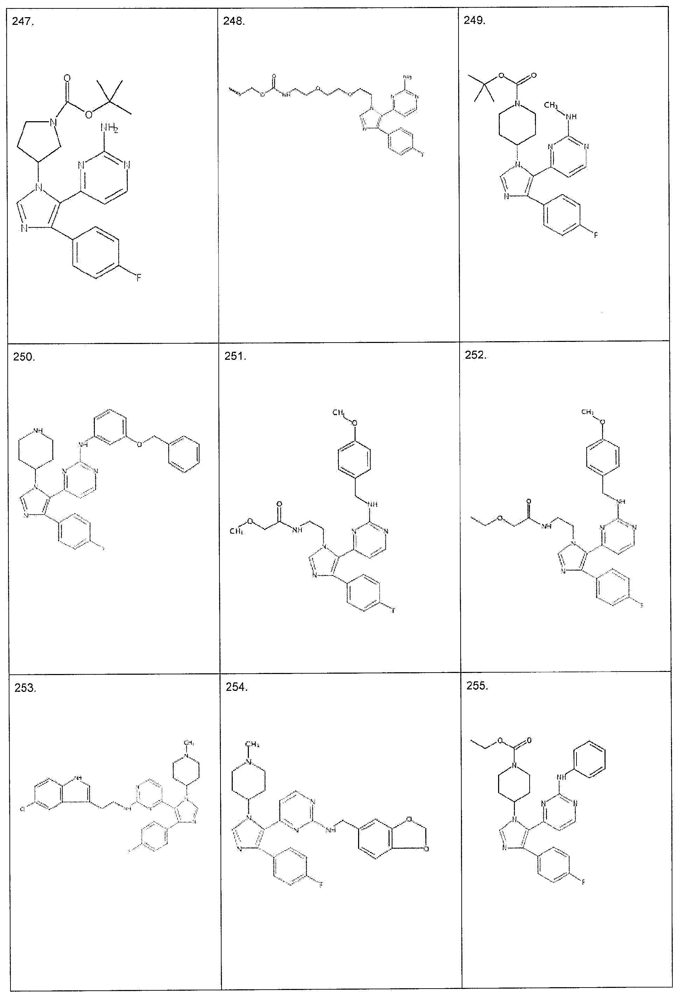

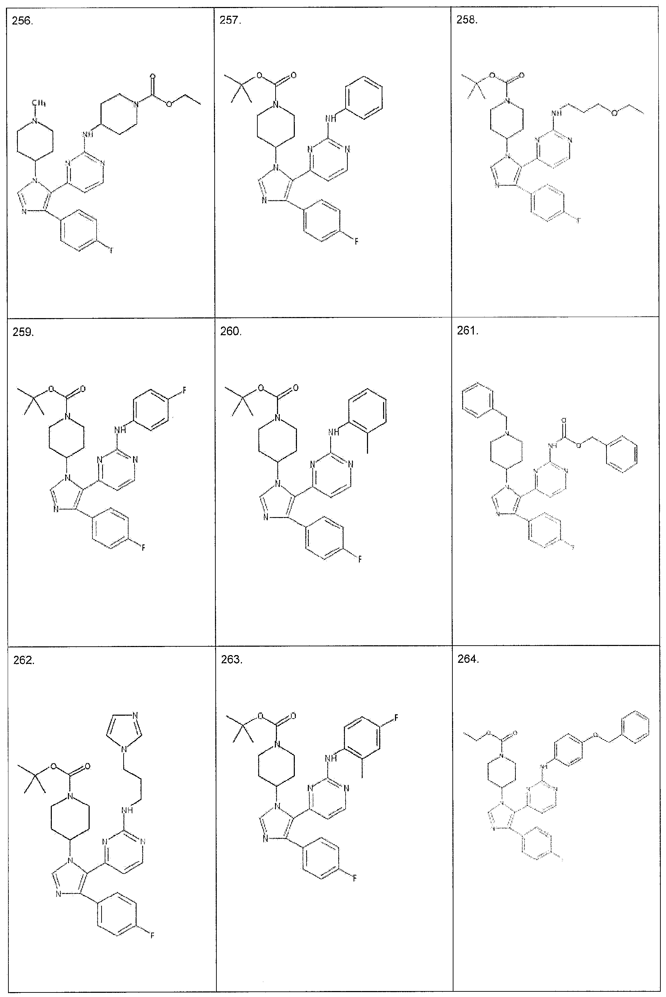

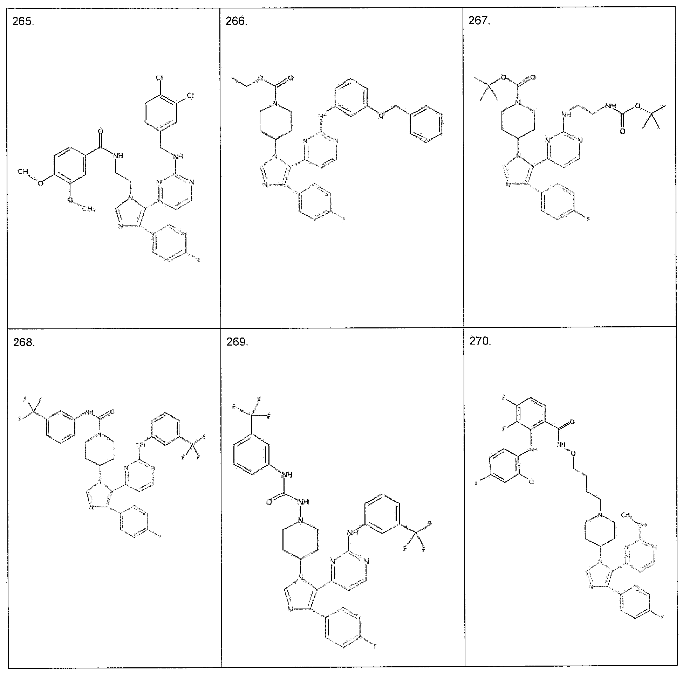

In one embodiment, the present invention provides a compound of formula (la) provided that the compound is not selected from the list of compounds in Figure 8.

In one embodiment, the present invention provides a compound of formula (Ia) wherein R₁ is not H.

In one embodiment, the present invention provides a compound of formula (Ia) wherein R₁ is C₁₋₃alkyl. R₁ may be C₁₋₃alkyl substituted with hydroxyl.

In one embodiment, the present invention provides a compound of formula (Ia) wherein R₁ is C₁₋₃alkyl and R₂ is H.

In one embodiment, the present invention provides a compound of formula (Ia) wherein R₁ is C₂₋₆alkynyl. R₁ may be selected from C₂alkynyl, C₃alkynyl and C₄alkynyl. In one embodiment, when R₁ is C₂₋₆alkynyl, R₂ is H.

In one embodiment, the present invention provides a compound of formula (Ia) wherein R₁ is C₆aryl. In one embodiment, when R₁ is C₆aryl, R₂ is H.

In one embodiment, the present invention provides a compound of formula (Ia) wherein R₁ is C₃₋₆cycloalkyl. R₁ may be selected from cyclobutyl, cyclopentyl and cyclohexyl. In one embodiment, when R₁ is C₃₋₆cycloalkyl, R₂ is H.

In one embodiment, the present invention provides a compound of formula (Ia) wherein R₁ is C₃₋₅heterocyclyl. R₁ may be selected from an oxygen-containing heterocyclyl group, a nitrogen-containing heterocyclyl group, or a sulphur-containing heterocyclyl group, or a heterocyclyl group containing a combination of two or more oxygen, nitrogen and sulphur atoms. Examples include oxiranyl, thiiranyl 1,3-diazetidinyl, oxetanyl, thietanyl, pyrrolidinyl, pyrazolidinyl, imidazolidinyl, terahydrofuranyl, 1,3-dioxolanyl, tetrahydropyranyl, tetrahydrothipheneyl, and 1,2-and 1,3-oxathiolanyl. In one embodiment, when R₁ is C₃₋₅heterocyclyl, R₂ is H.

In one embodiment, the present invention provides a compound of formula (Ia) wherein R₁ and R₂ are both the same. For example, R₁ and R₂ may both be H, or R₁ and R₂ may both be C₁₋₆alkyl (e.g. methyl, ethyl, propyl or butyl).

Preferably, R₁ and R₂ together with the nitrogen to which they are attached form a morpholino group.

In another preferred form, R₁ and R₂ together with the nitrogen to which they are attached form an N-heterocyclyl group comprising 4 to 8 carbon atoms.

In one embodiment, the present invention provides a compound of formula (Ia) wherein R₃ is CH₃. In another embodiment, R₃ is F, Cl, Br or I. Preferably, R₃ is F or Cl.

In one embodiment, the present invention provides a compound of formula (Ia) wherein R₄ is C₀₋₃alkylC₃₋₁₂cycloalkyl.

In one embodiment, R₄ is C₀₋₃alkylC₃₋₁₂cycloalkyl, wherein the C₃₋₁₂cycloalkyl group is selected from cyclopropyl, cyclobutyl, cyclopentyl, cyclohexyl and cycloheptyl.

In a preferred form, R₄ is C₃₋₁₂cycloalkyl. More preferably, R₄ is selected from cyclopropyl, cyclobutyl, cyclopentyl, cyclohexyl and cycloheptyl.

In one embodiment, R₄ is C₁₋₂alkylC₃₋₁₂cycloalkyl. R₄ may be C₁alkylC₃₋₁₂cycloalkyl. The C₃₋₁₂cycloalkyl group may be selected from cyclopropyl, cyclobutyl, cyclopentyl, cyclohexyl and cycloheptyl

In a preferred form, when R₄ is C₀₋₃alkylC₃₋₁₂cycloalkyl, the C₃₋₁₂cycloalkyl group is a bridged moiety. More preferably, R₄ is selected from the group consisting of adamantyl, norbornyl, and a spirocycloalkyl moiety.

In one embodiment, the present invention provides a compound of formula (Ia) wherein R₄ is C₁₋₁₂alkyl. R₄ may be a methyl, ethyl, propyl or butyl group.

In one embodiment, R₄ is C₀₋₆alkylheterocyclyl and comprises a nitrogen atom. The nitrogen may be substituted with the group R₅, wherein R₅ is selected from the group consisting of C₀₋₃alkylC₃₋₁₂cycloalkyl, C₁₋₁₂alkyl, C₀₋₁₀alkylC₆aryl, C₁₋₆alkylOC₁₋₆alkylC₆aryl, C₀₋₆alkylheteroaryl, and C₀₋₆alkylheterocyclyl.

In one embodiment, R₅ is selected from C₁₋₁₂alkyl and C₀₋₁₀alkylC₆aryl. In one embodiment, the C₀₋₁₀alkylC₆aryl group is C₁₋₆alkylC₆aryl.

As used herein the term "alkyl" refers to a straight or branched chain hydrocarbon radical having from one to twelve carbon atoms, or any range between, i.e. it contains 1, 2, 3, 4, 5, 6, 7, 8, 9, 10, 11 or 12 carbon atoms. The alkyl group is optionally substituted with substituents, multiple degrees of substitution being allowed. Examples of "alkyl" as used herein include, but are not limited to, methyl, ethyl, n-propyl, isopropyl, n-butyl, isobutyl, t-butyl, n-pentyl, isopentyl, and the like.

As used herein, the terms "C₁₋₃alkyl", "C₁₋₄alkyl" and "C₁₋₆alkyl" and the like refer to an alkyl group, as defined above, containing at least 1, and at most 3, 4 or 6 carbon atoms respectively, or any range in between (e.g. alkyl groups containing 2-5 carbon atoms, i.e. 2, 3, 4 or 5 carbon atoms, are also within the range of C₁₋₆). Where the term "C₀₋₂ alkyl", or the like, is used, there may be no alkyl group, or an alkyl group containing 1 or 2 carbon atoms.

The term "alkynyl" refers to an at least partially unsaturated, straight-chain or branched hydrocarbon group that contains from 2 to 6 carbon atoms i.e. 2, 3, 4, 5 or 6, carbon atoms. Specific examples of alkynyl groups are ethynyl, propynyl, butynyl, acetylenyl and propargyl groups. Preferably, alkynyl groups have one or two triple bond(s). The triple bond may be a terminal. The triple bond(s) may be internal.

As used herein, the term "halogen" refers to fluorine (F), chlorine (Cl), bromine (Br), or iodine (I) and the term "halo" refers to the halogen radicals fluoro (-F), chloro (-CI), bromo (-Br), and iodo (-I). Preferably, 'halo' is fluoro or chloro.

As used herein, the term "cycloalkyl" refers to a non-aromatic cyclic hydrocarbon ring. In a like manner the term "C₃₋₇cycloalkyl" refers to a non-aromatic cyclic hydrocarbon ring having from three to seven carbon atoms, or any range in between. For example, the C₃₋₇cycloalkyl group would also include cycloalkyl groups containing 4 to 6 (i.e. 4, 5 or 6) carbon atoms. The alkyl group is as defined above, and may be substituted. Exemplary "C₃₋₇cycloalkyl" groups useful in the present invention include, but are not limited to, cyclopropyl, cyclobutyl, cyclopentyl, cyclohexyl and cycloheptyl.

Cycloalkyl groups may optionally be fused to one or more heterocyclic or cycloalkyl rings. Cycloalkyl rings may be substituted at any of the carbon atoms on the ring with another cycloalkyl or heterocyclic moiety to form a spirocycloalkyl or spiroheteroalkyl compound.

Two non-adjacent atoms on the cycloalkyl group may be bridged by an alkyl or heteroalkyl group to form a bridged system. Preferably, the bridging group is 1-3 atoms in length.

As used herein, the terms "heterocyclic" or "heterocyclyl" refer to a non-aromatic heterocyclic ring, being saturated or having one or more degrees of unsaturation, containing one or more heteroatom substitution selected from S, S(O), S(O)₂, O, or N. The term "C₃₋₇heterocyclyl" refers to a non-aromatic cyclic hydrocarbon ring having from three to seven carbon atoms (i.e. 3, 4, 5, 6 or 7 carbon atoms) containing one or more heteroatom substitutions as referred to herein. The heterocyclic moiety may be substituted, multiple degrees of substitution being allowed. The term "C₃₋₇ heterocyclyl" also includes heterocyclyl groups containing C₄₋₅, C₅₋₇, C₆₋₇, C₄₋₇, C₄₋₆ and C₅₋₆ carbon atoms. Preferably, the heterocyclic ring contains four to six carbon atoms and one or two heteroatoms. More preferably, the heterocyclic ring contains five carbon atoms and one heteroatom, or four carbon atoms and two heteroatom substitutions, or five carbon atoms and one heteroatom. Such a ring may be optionally fused to one or more other "heterocyclic" ring(s) or cycloalkyl ring(s). Examples of "heterocyclic" moieties include, but are not limited to, tetrahydrofuran, pyran, oxetane, 1,4-dioxane, 1,3-dioxane, piperidine, piperazine, N-methylpiperazinyl, 2,4-piperazinedione, pyrrolidine, imidazolidine, pyrazolidine, morpholine, thiomorpholine, tetrahydrothiopyran, tetrahydrothiophene, and the like.

Heterocyclic groups may be substituted at any of the carbons on the ring with another heterocyclic or cycloalkyl moiety to form a spirocycloalkyl or spiroheteroalkyl compound.

Two non-adjacent atoms on the heterocyclic group may further be bridged by an alkyl or heteroalkyl group to form a bridged system. Preferably, the bridging group is 1-3 atoms in length.

As an example of substituted heterocyclic groups, the term "C₀₋₂alkylC₃₋₇heterocyclyl" includes heterocyclyl groups containing either no alkyl group as a linker between the compound and the heterocycle, or an alkyl group containing 1 or 2 carbon atoms as a linker between the compound and the heterocycle (i.e. heterocycle, -CH₂-heterocycle or -CH₂CH₂-heterocycle). These heterocycles may be further substituted.

Substituted cycloalkyl and heterocyclyl groups may be substituted with any suitable substituent as described below.

As used herein, the term "aryl" refers to an optionally substituted benzene ring or to an optionally substituted benzene ring system fused to one or more optionally substituted benzene rings to form, for example, anthracene, phenanthrene, or naphthalene ring systems. Examples of "aryl" groups include, but are not limited to, phenyl, 2-naphthyl, 1-naphthyl, biphenyl, as well as substituted derivatives thereof.

As used herein, the term "heteroaryl" refers to a monocyclic five, six or seven membered aromatic ring, or to a fused bicyclic or tricyclic aromatic ring system comprising at least one monocyclic five, six or seven membered aromatic ring. These heteroaryl rings contain one or more nitrogen, sulfur, and/or oxygen heteroatoms, where N-oxides and sulfur oxides and dioxides are permissible heteroatom substitutions and may be optionally substituted with up to three members. Examples of "heteroaryl" groups used herein include furanyl, thiophenyl, pyrrolyl, imidazolyl, pyrazolyl, triazolyl, tetrazolyl, thiazolyl, oxazolyl, isoxazolyl, oxadiazolyl, oxo-pyridyl, thiadiazolyl, isothiazolyl, pyridyl, pyridazyl, pyrazinyl, pyrimidyl, quinolinyl, isoquinolinyl, benzofuranyl, benzothiophenyl, indolyl, indazolyl, benzimidazolyl, and substituted versions thereof.

A "substituent" as used herein, refers to a molecular moiety that is covalently bonded to an atom within a molecule of interest. For example, a "ring substituent" may be a moiety such as a halogen, alkyl group, or other substituent described herein that is covalently bonded to an atom, preferably a carbon or nitrogen atom, that is a ring member. The term "substituted," as used herein, means that any one or more hydrogens on the designated atom is replaced with a selection from the indicated substituents, provided that the designated atom's normal valence is not exceeded, and that the substitution results in a stable compound, i.e., a compound that can be isolated, characterized and tested for biological activity.

The terms "optionally substituted" or "may be substituted" and the like, as used throughout the specification, denotes that the group may or may not be further substituted or fused (so as to form a polycyclic system), with one or more nonhydrogen substituent groups. Suitable chemically viable substituents for a particular functional group will be apparent to those skilled in the art.

Examples of substituents include but are not limited to:
C₁₋₆alkyl, C₁₋₆haloalkyl, C₁₋₆ haloalkoxy, C₁₋₆hydroxyalkyl, C₃₋₇heterocyclyl, C₃₋₇cycloalkyl, C₁₋₆alkoxy, C₁₋₆alkylsulfanyl, C₁₋₆alkylsulfenyl, C₁₋₆alkylsulfonyl, C₁₋₆alkylsulfonylamino, arylsulfonoamino, alkylcarboxy, alkylcarboxyamide, oxo, hydroxy, mercapto, amino, acyl, carboxy, carbamoyl, aryl, aryloxy, heteroaryl, aminosulfonyl, aroyl, aroylamino, heteroaroyl, acyloxy, aroyloxy, heteroaroyloxy, alkoxycarbonyl, nitro, cyano, halogen, ureido or C₁₋₆ perfluoroalkyl. In one embodiment, cyclic or heterocyclic substituents may form a spirocycloalkyl or spiroheteroalkyl substituent with a carbon in the moiety from which the cyclic or heterocyclic group is substituted. In another embodiment, cyclic or heterocyclic substituents may be bridged.

Any of these groups may be further substituted by any of the above-mentioned groups, where appropriate. For example, alkylamino, or dialkylamino, C₁₋₆alkoxy, etc.

The salts of the compounds of formulae (I) and (Ia) are preferably pharmaceutically acceptable, but it will be appreciated that non-pharmaceutically acceptable salts also fall within the scope of the present disclosure, since these are useful as intermediates in the preparation of pharmaceutically acceptable salts.

Suitable pharmaceutically acceptable salts include, but are not limited to, salts of pharmaceutically acceptable inorganic acids such as hydrochloric, sulphuric, phosphoric, nitric, carbonic, boric, sulfamic, and hydrobromic acids, or salts of pharmaceutically acceptable organic acids such as acetic, propionic, butyric, tartaric, maleic, hydroxymaleic, fumaric, malic, citric, lactic, mucic, gluconic, benzoic, succinic, oxalic, phenylacetic, methanesulphonic, toluenesulphonic, benzenesulphonic, salicylic, sulphanilic, aspartic, glutamic, edetic, stearic, palmitic, oleic, lauric, pantothenic, tannic, ascorbic and valeric acids.

Base salts include, but are not limited to, those formed with pharmaceutically acceptable cations, such as sodium, potassium, lithium, calcium, magnesium, zinc, ammonium, alkylammonium such as salts formed from triethylamine, alkoxyammonium such as those formed with ethanolamine and salts formed from ethylenediamine, choline or amino acids such as arginine, lysine or histidine. General information on types of pharmaceutically acceptable salts and their formation is known to those skilled in the art and is as described in general texts such as *"Handbook of Pharmaceutical salts"* P.H.Stahl, C.G.Wermuth, 1st edition, 2002, Wiley-VCH.

Basic nitrogen-containing groups may be quarternised with such agents as lower alkyl halide, such as methyl, ethyl, propyl, and butyl chlorides, bromides and iodides; dialkyl sulfates like dimethyl and diethyl sulfate; and others.

Prodrugs include compounds wherein an amino acid residue, or a polypeptide chain of two or more (e.g., two, three or four) amino acid residues which are covalently joined to free amino and/or amido groups of compounds of formula (I) or (la). The amino acid residues include the 20 naturally occurring amino acids commonly designated by three letter symbols and also include, 4-hydroxyproline, hydroxylysine, demosine, isodemosine, 3-methylhistidine, norvlin, beta-alanine, gamma-aminobutyric acid, citrulline, homocysteine, homoserine, ornithine and methionine sulfone. Prodrugs also include compounds wherein carbonates, carbamates, amides and alkyl esters which are covalently bonded to the above substituents of formula (I) or formula (Ia) through the carbonyl carbon prodrug sidechain. Prodrugs can include covalent irreversible and reversible inhibitors.

In the case of compounds that are solids, it will be understood by those skilled in the art that the inventive compounds, agents and salts may exist in different crystalline or polymorphic forms, all of which are intended to be within the scope of the present invention and specified formulae.

The term "polymorph" includes any crystalline form of compounds of formula (I) or formula (la) such as anhydrous forms, hydrous forms, solvate forms and mixed solvate forms.

In yet another aspect, there is provided a composition comprising a compound according to formula (I) or a salt, solvate, or polymorph thereof, and a pharmaceutically acceptable excipient.

In yet another aspect, there is provided a composition comprising a compound according to formula (Ia) or a salt, solvate, prodrug or polymorph thereof, and a pharmaceutically acceptable excipient.

An appropriate dosage level will generally be about 0.01 to 500 mg per kg patient body weight per day which can be administered in single or multiple doses. Preferably, the dosage level will be about 0.1 to about 250 mg/kg per day; more preferably about 0.5 to about 100 mg/kg per day. A suitable dosage level may be about 0.01 to 250 mg/kg per day, about 0.05 to 100 mg/kg per day, or about 0.1 to 50 mg/kg per day. Within this range the dosage may be 0.05 to 0.5, 0.5 to 5, or 5 to 50 mg/kg per day. For oral administration, the compositions are preferably provided in the form of tablets containing 1.0 to 1000 milligrams of the active ingredient, particularly 1.0, 5.0, 10.0, 15.0, 20.0, 25.0, 50.0, 75.0, 100.0, 150.0, 200.0, 250.0, 300.0, 400.0, 500.0, 600.0, 750.0, 800.0, 900.0, and 1000.0 milligrams of the active ingredient for the symptomatic adjustment of the dosage to the patient to be treated. The compounds may be administered on a regimen of 1 to 4 times per day, preferably once or twice per day. It will be understood, however, that the specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy. The amount of a compound of the present invention in the composition will also depend upon the particular compound in the composition.

In the case of inhaled products, the typical inhalation dose is less than with other forms of dosing starting at 1 microgram and rising to 1000 microgram for a single puff. In a preferred form, the dose ranges from 25 microgram to 250 microgram per puff. In another preferred form, the dosage ranges from 500 to 1000 micrograms per puff. In another form, the dosage is selected from the group consisting of 1, 2.5, 10.0, 25.0, 50.0, 75.0, 100.0, 150.0, 200.0, 250.0, 300.0, 400.0, 500.0, 600.0, 750.0, 800.0, 900.0, and 1000.0 micrograms per puff or any range in between and including two of these values. The medication may be one puff per day or increase up to two puffs four times a day.

The pharmaceutical composition may further comprise other therapeutically active compounds which are usually applied in the treatment of the disclosed disorders or conditions. Selection of the appropriate agents for use in combination therapy may be made by one of ordinary skill in the art, according to conventional pharmaceutical principles. The combination of therapeutic agents may act synergistically to effect the treatment or prevention of the various disorders or conditions disclosed herein. Using this approach, one may be able to achieve therapeutic efficacy with lower dosages of each agent, thus reducing the potential for adverse side effects.

Compounds and compositions of the invention may be formulated for any appropriate route of administration including, for example, topical (for example, transdermal or ocular), pulmonary, oral, buccal, nasal, vaginal, rectal or parenteral administration. The term parenteral as used herein includes subcutaneous, intradermal, intravascular (for example, intravenous), intramuscular, spinal, intracranial, intrathecal, intraocular, periocular, intraorbital, intrasynovial and intraperitoneal injection, as well as any similar injection or infusion technique. In certain embodiments, compositions in a form suitable for oral use or parenteral use are preferred. Suitable oral forms include, for example, tablets, troches, lozenges, aqueous or oily suspensions, dispersible powders or granules, emulsions, hard or soft capsules, or syrups or elixirs. Within yet other embodiments, compositions provided herein may be formulated as a lyophilizate.

In a preferred form, the composition is suitable for administration to the respiratory tract. In another form, the composition is suitable for oral administration.

The various dosage units are each preferably provided as a discrete dosage tablet, capsules, lozenge, dragee, gum, or other type of solid formulation. Capsules may encapsulate a powder, liquid, or gel. The solid formulation may be swallowed, or may be of a suckable or chewable type (either frangible or gum-like). The present invention contemplates dosage unit retaining devices other than blister packs; for example, packages such as bottles, tubes, canisters, packets. The dosage units may further include conventional excipients well-known in pharmaceutical formulation practice, such as binding agents, gellants, fillers, tableting lubricants, disintegrants, surfactants, and colorants; and for suckable or chewable formulations.

Compositions intended for oral use may further comprise one or more components such as sweetening agents, flavouring agents, colouring agents and/or preserving agents in order to provide appealing and palatable preparations. Tablets contain the active ingredient in admixture with physiologically acceptable excipients that are suitable for the manufacture of tablets. Such excipients include, for example, inert diluents such as calcium carbonate, sodium carbonate, lactose, calcium phosphate or sodium phosphate, granulating and disintegrating agents such as corn starch or alginic acid, binding agents such as starch, gelatine or acacia, and lubricating agents such as magnesium stearate, stearic acid or talc. The tablets may be uncoated or they may be coated by known techniques to delay disintegration and absorption in the gastrointestinal tract and thereby provide a sustained action over a longer period. For example, a time delay material such as glyceryl monosterate or glyceryl distearate may be employed.

Formulations for oral use may also be presented as hard gelatine capsules wherein the active ingredient is mixed with an inert solid diluent such as calcium carbonate, calcium phosphate or kaolin, or as soft gelatine capsules wherein the active ingredient is mixed with water or an oil medium such as peanut oil, liquid paraffin or olive oil.

Aqueous suspensions contain the active ingredient(s) in admixture with excipients suitable for the manufacture of aqueous suspensions. Such excipients include suspending agents such as sodium carboxymethylcellulose, methylcellulose, hydroxypropylmethylcellulose, sodium alginate, polyvinylpyrrolidone, gum tragacanth and gum acacia, and dispersing or wetting agents such as naturally-occurring phosphatides (for example, lecithin), condensation products of an alkylene oxide with fatty acids such as polyoxyethylene stearate, condensation products of ethylene oxide with long chain aliphatic alcohols such as heptadecaethyleneoxycetanol, condensation products of ethylene oxide with partial esters derived from fatty acids and a hexitol such as polyoxyethylene sorbitol mono-oleate, or condensation products of ethylene oxide with partial esters derived from fatty acids and hexitol anhydrides such as polyethylene sorbitan monooleate. Aqueous suspensions may also comprise one or more preservatives, for example ethyl or n-propyl p-hydroxybenzoate, one or more colouring agents, one or more flavouring agents, and one or more sweetening agents, such as sucrose or saccharin.

Oily suspensions may be formulated by suspending the active ingredients in a vegetable oil such as arachis oil, olive oil, sesame oil or coconut oil, or in a mineral oil such as liquid paraffin. The oily suspensions may contain a thickening agent such as beeswax, hard paraffin or cetyl alcohol. Sweetening agents such as those set forth above, and/or flavouring agents may be added to provide palatable oral preparations. Such suspensions may be preserved by the addition of an antioxidant such as ascorbic acid.

Dispersible powders and granules suitable for preparation of an aqueous suspension by the addition of water provide the active ingredient in admixture with a dispersing or wetting agent, suspending agent and one or more preservatives. Suitable dispersing or wetting agents and suspending agents are exemplified by those already mentioned above. Additional excipients, such as sweetening, flavouring and colouring agents, may also be present.

Pharmaceutical compositions may also be in the form of oil-in-water emulsions. The oily phase may be a vegetable oil such as olive oil or arachis oil, a mineral oil such as liquid paraffin, or a mixture thereof. Suitable emulsifying agents include naturally-occurring gums such as gum acacia or gum tragacanth, naturally-occurring phosphatides such as soy bean lecithin, and esters or partial esters derived from fatty acids and hexitol, anhydrides such as sorbitan monoleate, and condensation products of partial esters derived from fatty acids and hexitol with ethylene oxide such as polyoxyethylene sorbitan monoleate. An emulsion may also comprise one or more sweetening and/or flavouring agents.

Syrups and elixirs may be formulated with sweetening agents, such as glycerol, propylene glycol, sorbitol or sucrose. Such formulations may also comprise one or more demulcents, preservatives, flavouring agents and/or colouring agents.

Compositions of the invention may be formulated for local or topical administration, such as for topical application to the skin. Formulations for topical administration typically comprise a topical vehicle combined with active agent(s), with or without additional optional components.

Suitable topical vehicles and additional components are well known in the art, and it will be apparent that the choice of a vehicle will depend on the particular physical form and mode of delivery. Topical vehicles include organic solvents such as alcohols (for example, ethanol, iso-propyl alcohol or glycerine), glycols such as butylene, isoprene or propylene glycol, aliphatic alcohols such as lanolin, mixtures of water and organic solvents and mixtures of organic solvents such as alcohol and glycerine, lipid-based materials such as fatty acids, acylglycerols including oils such as mineral oil, and fats of natural or synthetic origin, phosphoglycerides, sphingolipids and waxes, protein-based materials such as collagen and gelatine, silicone-based materials (both nonvolatile and volatile), and hydrocarbon-based materials such as microsponges and polymer matrices.

A composition may further include one or more components adapted to improve the stability or effectiveness of the applied formulation, such as stabilizing agents, suspending agents, emulsifying agents, viscosity adjusters, gelling agents, preservatives, antioxidants, skin penetration enhancers, moisturizers and sustained release materials. Examples of such components are described in Martindale - The Extra Pharmacopoeia (Pharmaceutical Press, London 1993) and Martin (ed.), Remington's Pharmaceutical Sciences. Formulations may comprise microcapsules, such as hydroxymethylcellulose or gelatine-microcapsules, liposomes, albumin microspheres, microemulsions, nanoparticles or nanocapsules.

A topical formulation may be prepared in a variety of physical forms including, for example, solids, pastes, creams, foams, lotions, gels, powders, aqueous liquids, emulsions, sprays and skin patches. The physical appearance and viscosity of such forms can be governed by the presence and amount of emulsifier(s) and viscosity adjuster(s) present in the formulation. Solids are generally firm and non-pourable and commonly are formulated as bars or sticks, or in particulate form. Solids can be opaque or transparent, and optionally can contain solvents, emulsifiers, moisturizers, emollients, fragrances, dyes/colorants, preservatives and other active ingredients that increase or enhance the efficacy of the final product. Creams and lotions are often similar to one another, differing mainly in their viscosity. Both lotions and creams may be opaque, translucent or clear and often contain emulsifiers, solvents, and viscosity adjusting agents, as well as moisturizers, emollients, fragrances, dyes/colorants, preservatives and other active ingredients that increase or enhance the efficacy of the final product. Gels can be prepared with a range of viscosities, from thick or high viscosity to thin or low viscosity. These formulations, like those of lotions and creams, may also contain solvents, emulsifiers, moisturizers, emollients, fragrances, dyes/colorants, preservatives and other active ingredients that increase or enhance the efficacy of the final product. Liquids are thinner than creams, lotions, or gels, and often do not contain emulsifiers. Liquid topical products often contain solvents, emulsifiers, moisturizers, emollients, fragrances, dyes/colorants, preservatives and other active ingredients that increase or enhance the efficacy of the final product.

Emulsifiers for use in topical formulations include, but are not limited to, ionic emulsifiers, cetearyl alcohol, non-ionic emulsifiers like polyoxyethylene oleyl ether, PEG-40 stearate, ceteareth-12, ceteareth-20, ceteareth-30, ceteareth alcohol, PEG-100 stearate and glyceryl stearate. Suitable viscosity adjusting agents include, but are not limited to, protective colloids or nonionic gums such as hydroxyethylcellulose, xanthan gum, magnesium aluminum silicate, silica, microcrystalline wax, beeswax, paraffin, and cetyl palmitate. A gel composition may be formed by the addition of a gelling agent such as chitosan, methyl cellulose, ethyl cellulose, polyvinyl alcohol, polyquaterniums, hydroxyethylceilulose, hydroxypropylcellulose, hydroxypropylmethylcellulose, carbomer or ammoniated glycyrrhizinate. Suitable surfactants include, but are not limited to, nonionic, amphoteric, ionic and anionic surfactants. For example, one or more of dimethicone copolyol, polysorbate 20, polysorbate 40, polysorbate 60, polysorbate 80, lauramide DEA, cocamide DEA, and cocamide MEA, oleyl betaine, cocamidopropyl phosphatidyl PG-dimonium chloride, and ammonium laureth sulfate may be used within topical formulations.

Preservatives include, but are not limited to, antimicrobials such as methylparaben, propylparaben, sorbic acid, benzoic acid, and formaldehyde, as well as physical stabilizers and antioxidants such as vitamin E, sodium ascorbate/ascorbic acid and propyl gallate. Suitable moisturizers include, but are not limited to, lactic acid and other hydroxy acids and their salts, glycerine, propylene glycol, and butylene glycol. Suitable emollients include lanolin alcohol, lanolin, lanolin derivatives, cholesterol, petrolatum, isostearyl neopentanoate and mineral oils. Suitable fragrances and colours include, but are not limited to, FD&C Red No. 40 and FD&C Yellow No. 5. Other suitable additional ingredients that may be included in a topical formulation include, but are not limited to, abrasives, absorbents, anticaking agents, antifoaming agents, antistatic agents, astringents (such as witch hazel), alcohol and herbal extracts such as chamomile extract, binders/excipients, buffering agents, chelating agents, film forming agents, conditioning agents, propellants, opacifying agents, pH adjusters and protectants.

Typical modes of delivery for topical compositions include application using the fingers, application using a physical applicator such as a cloth, tissue, swab, stick or brush, spraying including mist, aerosol or foam spraying, dropper application, sprinkling, soaking, and rinsing. Controlled release vehicles can also be used, and compositions may be formulated for transdermal administration (for example, as a transdermal patch).

Pharmaceutical compositions may be formulated as sustained release formulations such as a capsule that creates a slow release of modulator following administration. Such formulations may generally be prepared using well-known technology and administered by, for example, oral, rectal or subcutaneous implantation, or by implantation at the desired target site. Carriers for use within such formulations are biocompatible, and may also be biodegradable. Preferably, the formulation provides a relatively constant level of modulator release. The amount of modulator contained within a sustained release formulation depends upon, for example, the site of implantation, the rate and expected duration of release and the nature of the disorder to be treated or prevented.

A pharmaceutical composition may be formulated as inhaled formulations, including sprays, mists, or aerosols. For example, for administration to the respiratory tract. This may be particularly preferred for treatment of a respiratory disease, a condition of the airway or lung involving fibrosis as described herein. The inhaled formulation may be for application to the upper (including the nasal cavity, pharynx and larynx) and lower respiratory tract (including trachea, bronchi and lungs). For inhalation formulations, the composition or combination provided herein may be delivered via any inhalation methods known to a person skilled in the art. Such inhalation methods and devices include, but are not limited to, metered dose inhalers with propellants such as HFA or propellants that are physiologically and environmentally acceptable. Other suitable devices are breath operated inhalers, multidose dry powder inhalers and aerosol nebulizers. Aerosol formulations for use in the subject method typically include propellants, surfactants and co-solvents and may be filled into conventional aerosol containers that are closed by a suitable metering valve. Different devices and excipients can be used depending on whether the application is to the upper (including the nasal cavity, pharynx and larynx) or lower respiratory tract (including trachea, bronchi and lungs) and can be determined by those skilled in the art. Further, processes for micronisation and nanoparticle formation for the preparation of compounds described herein for use in an inhaler, such as a dry powder inhaler, are also known by those skilled in the art.

Inhalant compositions may comprise liquid or powdered compositions containing the active ingredient that are suitable for nebulization and intrabronchial use, or aerosol compositions administered via an aerosol unit dispensing metered doses. Suitable liquid compositions comprise the active ingredient in an aqueous, pharmaceutically acceptable inhalant solvent such as isotonic saline or bacteriostatic water. The solutions are administered by means of a pump or squeeze-actuated nebulized spray dispenser, or by any other conventional means for causing or enabling the requisite dosage amount of the liquid composition to be inhaled into the patient's lungs. Suitable formulations, wherein the carrier is a liquid, for administration, as for example, a nasal spray or as nasal drops, include aqueous or oily solutions of the active ingredient. Examples of inhalation drug delivery devices are described in Ibrahim et al. Medical Devices: Evidence and Research 2015:8 131-139, are contemplated for use in the present invention.

In another aspect, there is provided a method of treating or preventing a respiratory disease in a subject in need thereof, the method comprising administering to the subject a therapeutically effective amount of a compound of formula (I) or a salt, solvate, or polymorph thereof, thereby treating or preventing a respiratory disease in a subject.

There is further provided a compound of formula (I) or a salt, solvate or polymorph thereof for use in the treatment or prevention of a respiratory disease in a subject in need thereof.

Use of a compound of formula (I) or a salt, solvate, or polymorph thereof in the preparation of a medicament for the treatment or prevention of a respiratory disease in a subject in need thereof is also described.

In another aspect, there is provided a method of treating or preventing a respiratory disease in a subject in need thereof, the method comprising administering to the subject a therapeutically effective amount of a compound of formula (Ia) or a salt, solvate, enantiomer, prodrug or polymorph thereof, thereby treating or preventing a respiratory disease in a subject.

There is further provided a compound of formula (Ia) or a salt, solvate, prodrug or polymorph thereof for use in the treatment or prevention of a respiratory disease in a subject in need thereof.

Use of a compound of formula (Ia) or a salt, solvate, prodrug or polymorph thereof in the preparation of a medicament for the treatment or prevention of a respiratory disease in a subject in need thereof is also described.

As used herein, 'preventing' or 'prevention' is intended to refer to at least the reduction of likelihood of the risk of (or susceptibility to) acquiring a disease or disorder (i.e., causing at least one of the clinical symptoms of the disease not to develop in a patient that may be exposed to or predisposed to the disease but does not yet experience or display symptoms of the disease). Biological and physiological parameters for identifying such patients are provided herein and are also well known by physicians.

The terms 'treatment' or 'treating' of a subject includes the application or administration of a compound of the invention to a subject (or application or administration of a compound of the invention to a cell or tissue from a subject) with the purpose of delaying, slowing, stabilizing, curing, healing, alleviating, relieving, altering, remedying, lessening, worsening, ameliorating, improving, or affecting the disease or condition, the symptom of the disease or condition, or the risk of (or susceptibility to) the disease or condition. The term 'treating' refers to any indication of success in the treatment or amelioration of an injury, pathology or condition, including any objective or subjective parameter such as abatement; remission; lessening of the rate of worsening; lessening severity of the disease; stabilization, diminishing of symptoms or making the injury, pathology or condition more tolerable to the subject; slowing in the rate of degeneration or decline; making the final point of degeneration less debilitating; or improving a subject's physical or mental well-being.

The term 'antagonizing' used herein is intended to mean 'decreasing' or 'reducing'. A sufficient period of time can be during one week, or between 1 week to 1 month, or between 1 to 2 months, or 2 months or more. For chronic conditions, the compound of the present invention can be advantageously administered for life time period.

The term 'respiratory' refers to the process by which oxygen is taken into the body and carbon dioxide is discharged, through the bodily system including the nose, throat, larynx, trachea, bronchi and lungs.

The term 'respiratory disease' or 'respiratory condition' refers to any one of several ailments that may involve inflammation and/or tissue remodelling affecting a component of the respiratory system including the upper (including the nasal cavity, pharynx and larynx) and lower respiratory tract (including trachea, bronchi and lungs). Such ailments include pulmonary fibrosis (interstitial lung diseases), rhino sinusitis, influenza, sarcoidosis, bronchial carcinoma (including but not limited to nonsmall cell and small cell carcinoma of the lung, and lung metastases from tumours of other organs), silicosis, pneumoconiosis, acute lung injury, ventilation-induced lung injury, congenital emphysema, bronchopulmonary dysplasia, bronchiectasis, atelectasis, nasal polyps, asbestosis, mesothelioma, pulmonary eosinophilia, diffuse pulmonary haemorrhage syndromes, bronchiolitis obliterans, alveolar proteinosis, collagen and vascular disorders affecting the lung, and cough. Preferably, the respiratory disease is an obstructive airway disease, such ailments include asthmatic conditions including hay fever, allergen-induced asthma, exercise-induced asthma, pollution- induced asthma, cold-induced asthma, stress-induced asthma and viral-induced- asthma, obesity-related asthma, occupational asthma, thunderstorminduced asthma, asthma COPD overlap syndrome (ACOS) chronic obstructive pulmonary diseases including chronic bronchitis with normal airflow, chronic bronchitis with airway obstruction (chronic obstructive bronchitis), emphysema, asthmatic bronchitis, and bullous disease, and other pulmonary diseases involving inflammation including cystic fibrosis, pigeon fancier's disease, farmer's lung, acute respiratory distress syndrome, pneumonia of fungal, viral, bacterial, mixed or unknown aetiology, aspiration or inhalation injury, fat embolism in the lung, acidosis inflammation of the lung, acute pulmonary edema, acute mountain sickness, postcardiac surgery, acute pulmonary hypertension, persistent pulmonary hypertension of the newborn, perinatal aspiration syndrome, hyaline membrane disease, acute pulmonary thromboembolism, heparin-protamine reactions, sepsis, status asthmaticus and hypoxia. The inflammation in the upper and lower respiratory tract may be associated with or caused by viral infection or an allergen. It is expected that the anti-inflammatory activity of the compounds either alone or when co-administered with a glucocorticoid would make them particularly suitable for treatment of these disease or conditions.

The respiratory disease or condition may be associated with or caused by an allergen, such as house dust mite. The respiratory disease or condition may be the result of an allergen-induced inflammation. The present invention finds particular application to allergic disease of the airway or lung and exacerbations of that disease, such as exacerbations resulting from viral infection (e.g. RSV infection).

A symptom of respiratory disease may include cough, excess sputum production, a sense of breathlessness or chest tightness with audible wheeze. Exercise capacity may be quite limited. In asthma the FEV1.0 (forced expiratory volume in one second) as a percentage of that predicted nomographically based on weight, height and age, may be decreased as may the peak expiratory flow rate in a forced expiration. In COPD the FEV1.0 as a ratio of the forced vital capacity (FVC) is typically reduced to less than 0.7. In IPF there is a progressive fall in FVC. The impact of each of these conditions may also be measured by days of lost work/school, disturbed sleep, requirement for bronchodilator drugs, requirement for glucocorticoids including oral glucocorticoids. Further measures of the impact of these conditions include validated health-related quality of life measurements. Medical imaging procedures including but not limited to X-ray, high resolution computed tomography, magnetic resonance imaging, positron emission tomography, ultra sound, optical coherence tomography and fluoroscopy may also be used to assess disease and therapeutic response.

The existence of, improvement in, treatment of or prevention of a respiratory disease may be by any clinically or biochemically relevant method of the subject or a biopsy therefrom. For example, a parameter measured may be the presence or degree of lung function, signs and symptoms of obstruction; exercise tolerance; night time awakenings; days lost to school or work; bronchodilator usage; ICS dose; oral GC usage; need for other medications; need for medical treatment; hospital admission.

As used herein, the term 'asthma' refers to a respiratory disorder characterized by episodic difficulty in breathing brought on by any one or a combination of three primary factors including: 1) bronchospasm (i.e., variable and reversible airway obstruction due to airway muscle contraction), 2) inflammation of the airway lining, and 3) bronchial hyper-responsiveness resulting in excessive mucous in the airways, which may be triggered by exposure to an allergen or combination of allergens (i.e., dust mites and mold), viral or bacterial infection (i.e., common cold virus), environmental pollutants (i.e., chemical fumes or smoke), physical exertion (i.e., during exercise), stress, or inhalation of cold air. The term 'asthmatic condition,' as used herein, refers to the characteristic of an individual to suffer from an attack of asthma upon exposure to any one or a number of asthma triggers for that individual. An individual may be characterized as suffering from, for example, allergen-induced asthma, exercise-induced asthma, pollution-induced asthma, viral-induced asthma, or cold-induced asthma.

The efficacy of a treatment for asthma may be measured by methods well-known in the art, for example, increase in pulmonary function (spirometry), decrease in asthma exacerbations, increase in morning peak expiratory flow rate, decrease in rescue medication use, decrease in daytime and night-time asthma symptoms, increase in asthma-free days, increase in time to asthma exacerbation, and increase in forced expiratory volume in one second (FEV1.0).

The terms 'chronic obstructive pulmonary disease' and 'COPD' as used interchangeably herein refers to a chronic disorder or combination of disorders characterized by reduced maximal expiratory flow and slow forced emptying of the lungs that does not change markedly over several months and is not, or is only minimally, reversible with traditional bronchodilators. Most commonly, COPD is a combination of chronic bronchitis, i.e. the presence of cough and sputum for more than three months for about two consecutive years, and emphysema, i.e. alveolar damage. However, COPD can involve chronic bronchitis with normal airflow, chronic bronchitis with airway obstruction (chronic obstructive bronchitis), emphysema, asthmatic bronchitis, and bullous disease, and combinations thereof. Chronic obstructive pulmonary disease is a condition usually but not exclusively resulting from chronic lung damage induced by exposure to tobacco smoke. Other noxious airborne pollutants, such as indoor cooking exhaust and car exhaust may over the long-term cause or increase the risk of COPD, as does ageing.

The phrase 'a condition of the airway or lung involving fibrosis' or 'a condition of the airway or lung having a fibrotic component' includes any disease or condition where there is the formation or development of excess fibrous connective tissue (fibrosis) in the airway or lung thereby resulting in the development of scarred (fibrotic) tissue. This includes interstitial lung diseases such as pulmonary fibrosis, lung fibrosis or Idiopathic pulmonary fibrosis (IPF). More precisely, pulmonary fibrosis is a chronic disease that causes swelling and scarring of the alveoli and interstitial tissues of the lungs. The scar tissue replaces healthy tissue and causes inflammation. This damage to the lung tissue causes stiffness of the lungs which subsequently makes breathing more and more difficult. Lung fibrosis may result from radiation injury or from exposure to therapeutic agents such as bleomycin.

'Idiopathic pulmonary fibrosis (IPF)' is a specific manifestation of idiopathic interstitial pneumonia (IIP), a type of interstitial lung disease. Interstitial lung disease, also known as diffuse parenchymal lung disease (DPLD), refers to a group of lung diseases affecting the interstitium. Microscopically, lung tissue from IPF patients shows a characteristic set of histological features known as usual interstitial pneumonia (UIP). UIP is therefore the pathologic presentation of IPF.

The existence of, improvement in, treatment of or prevention of a condition of the airway or lung involving fibrosis, particularly pulmonary fibrosis / lung fibrosis or Idiopathic pulmonary fibrosis may be by any clinically or biochemically relevant method of the subject or a biopsy therefrom. For example, the rate of decline in FVC or the appearance of high resolution computed tomographic images of the lung may be useful in diagnosing IPF. Further, a parameter measured may be the presence or degree of fibrosis, the content of collagen, fibronectin, or another extracellular matrix protein, the proliferation rate of the cells or any extracellular matrix components in the cells or transdifferentiation of the cells to myofibroblasts.

In one embodiment, the respiratory disease is selected from asthma, chronic obstructive pulmonary disease, interstitial lung diseases (such as idiopathic pulmonary fibrosis) and other conditions relating to tissue remodelling, primary or secondary lung tumour, hayfever, chronic and acute sinusitis, and chronic and acute viral, fungal and bacterial infections of the respiratory tract.

In one embodiment, the improvement in respiratory function may be selected from a decrease in the level of constriction of the lungs, a decrease in the elastic stiffness of the respiratory system, and/or an increase in the ease with which the respiratory system can be extended. Preferably, the improvement is selected from a decrease in the level of constriction of the lungs, and a decrease in the elastic stiffness of the respiratory system. In yet another aspect, there is provided a composition comprising a compound according to formula (I) or a salt, solvate, or polymorph thereof, and a pharmaceutically acceptable excipient.

The therapeutically effective amount of the formulation depends on the severity of the specific respiratory disease indication (e.g. severe chronic asthma), the patient's clinical history and response, and the discretion of the attending physician. The formulation may be administered to the patient at one time or over a series of treatments. An initial candidate dosage may be administered to a patient and the proper dosage and treatment regimen established by monitoring the progress of this patient using conventional techniques well known to those of ordinary skill in the art. Preferably, the therapeutically effective concentration of the active compound will be in the range 0.1 nM to 100 µM. More preferably the range will be 0.1 - 10 µM. However, it will be appreciated that delivery by inhalation can result in cells within the airway being exposed for short periods of time to concentrations exceeding those quoted above, for a period of time whilst the drug is being diluted in the airway surface fluid and also being absorbed from the airway and lung surfaces.

In one aspect, the method of treatment of the present invention further comprises administering a concomitant medication for the target disease indication. For example, concomitant asthma medications (for both chronic and acute) that may be used with the method of the present invention include but are not limited to: inhaled and oral steroids (e.g. beclomethasone, budesonide, flunisolide, fluticasone, triamcinolone, mometasone); systemic corticosteroids (e.g. methylprednisolone, prednisolone, prednisone, dexamethasone, and deflazacort); inhaled or oral β₂-adrenoceptor agonists (e.g. salmeterol, formoterol, bitolterol, pirbuterol, vilanterol, terbutaline, bambuterol and albuterol); cromolyn and nedocromil; anti-allergic opthalmic medications (e.g. dexamethasone); agents that modulate the production and action of transforming growth factor-beta, including pirfenidone and nintedanib; methylxanthines and other phosphodiesterase inhibitors (e,g. theophylline and mepyramine-theophylline acetate, roflumilast); leukotriene modifying agents (e.g. zafirlukast, zileuton, montekulast and pranlukast); anticholinergics (e.g. ipatropium bromide); other therapeutic antibodies of any format (e.g. antibodies directed against interleukin 5, such as mepolizumab, or against IgE, such as omalizumab, those antibodies in monoclonal form, Fab, scFV, multivalent compositions, xenoantibodies etc), natural or engineered antibody mimetics (e.g. anticalin) or natural, engineered or synthetic peptides; thromboxane A₂ synthetase inhibitors; thromboxane prostanoid receptor antagonists; other eicosanoid modifiers (e.g. alprostadil vs. PGE₁, dinoprostone vs. PGE₂, epoprostenol vs. prostacyclin and PGI₂ analogues (e.g. PG1₂ beraprost), seratrodast, phosphodiesterase 4 isoenzyme inhibitors, thromboxane A₂ synthetase inhibitors (e.g. ozmagrel, dazmegrel or ozagrel); ditec (low dose disodium cromoglycate and fenoterol); platelet activating factor receptor antagonists; antihistamines or histamine antagonists: promethazine, chlorpheniramine, loratadine, cetirazine, azelastine; thromboxane A₂ receptor antagonists; bradykinin receptor antagonists (e.g. icatibant); agents that inhibit activated eosinophils and T-cell recruitment (e.g. ketotifen), IL-13 blockers (e.g. soluble IL-13 receptor fragments), IL-4 blockers (e.g. soluble IL-4 receptor fragments); ligands that bind and block the activity of IL-13 or IL-4, and xanthine derivatives (e.g. pentoxifylline); chemokine receptor antagonists and antagonists of the CRTH2 receptor.

In certain embodiments, the method of treatment of the present invention includes the concomitant provision to the subject of inhibitory RNA molecules (RNA interference molecules), for the purpose of reducing, inhibiting or preventing the expression of genes which encode target proteins. For example, the inhibitory RNA molecules may be used for reducing or inhibiting the expression of one or more of: proteins associated with pathogens (viral, bacterial, fungal) or mammalian cells, including but not limited to casein kinase 1 isoforms and other components of the CLOCK regulatory network (eg ARNT1, period 1-3) and other proteins that contribute to the inflammatory response in the respiratory system such as interleukin-5 and the NALP inflammasome.

The skilled person will be familiar with various means for utilising inhibitory RNA molecules for the purpose of interfering with gene expression in the subject. For example, the inhibitory RNA molecules may be any one of: short interfering RNA (siRNA), microRNA mimetic (miRNA), short hairpin RNA (shRNA) or long double stranded RNA (long dsRNA) molecules. The inhibitory RNA molecule may be administered directly to the subject requiring treatment (for example by inhalation, intratracheal, oral or nasal administration or by parenteral administration), or alternatively, be formed in the subject receiving treatment, following the administration of a polynucleotide (vector) construct which encodes a double stranded RNA (dsRNA) molecule which is capable of forming an inhibitory RNA molecule. The skilled person will also be familiar with various methods known in the art for formulating inhibitory RNA molecules for administration (for example, in liposomes, nanoparticles and the like). The invention also includes the administration of an inhibitor of casein kinase 1 and a medication for the target disease indication as described above where either or both are administered by inhalation or formulated for oral administration.

Although the invention finds application in humans, the invention is also useful for therapeutic veterinary purposes. The invention is useful for domestic or farm animals such as cattle, sheep, horses and poultry; for companion animals such as cats and dogs; and for zoo animals.

As used herein, a 'subject' refers to an animal, such as a mammalian or an avian species, including a human, an ape, a horse, a cow, a sheep, a goat, a dog, a cat, a guinea pig, a rat, a mouse, a chicken etc.

In another aspect the present invention provides a kit or article of manufacture including a compound of formula (I) or pharmaceutical compositions including a compound of formula (I) as described herein.

In other embodiments there is provided a kit for use in a therapeutic or prophylactic application mentioned herein, the kit including: a container holding a compound of formula (I) or pharmaceutical composition including a compound of formula (I); and a label or package insert with instructions for use.

In another aspect the present invention provides a kit or article of manufacture including a compound of formula (la) or pharmaceutical compositions including a compound of formula (la) as described herein.

In other embodiments there is provided a kit for use in a therapeutic or prophylactic application mentioned herein, the kit including: a container holding a compound of formula (la) or pharmaceutical composition including a compound of formula (la); and a label or package insert with instructions for use.

The kit or 'article of manufacture' may comprise a container and a label or package insert on or associated with the container. Suitable containers include, for example, bottles, vials, syringes, blister pack, etc. The containers may be formed from a variety of materials such as glass or plastic. The container holds a compound of formula (I) or formula (Ia), or composition which is effective for treating the condition and may have a sterile access port (for example the container may be an intravenous solution bag or a vial having a stopper pierceable by a hypodermic injection needle). The label or package insert indicates that the composition is used for treating a disorder. In one embodiment, the label or package insert includes instructions for use and indicates that the therapeutic or prophylactic composition can be used to treat a disorder described herein.

The kit may comprise (a) a therapeutic or prophylactic composition; and (b) a second container with a second active principle or ingredient contained therein. The kit in this embodiment of the invention may further comprise a package insert indicating the composition and other active principle can be used to treat a disorder or prevent a complication stemming from a disorder described herein. Alternatively, or additionally, the kit may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

In certain embodiments the therapeutic composition may be provided in the form of a device, disposable or reusable, including a receptacle for holding the compound of formula (I) or formula (la) or therapeutic or prophylactic pharmaceutical composition including a compound of formula (I) or formula (la). In one embodiment, the device is a syringe. The therapeutic or prophylactic composition may be provided in the device in a state that is ready for use or in a state requiring mixing or addition of further components.

### Examples (Compounds 74 and 81-83 are embodiments of the invention, the remaining compounds are provided for reference purposes)

### In vitro assays

The primary assay for the activity of the novel CK1δ/ε inhibitors is the modulation of gene expression in the airway epithelial cell line, the BEAS-2B cells. The assay is conducted under several conditions. Generally, TGF-β or TNF-α is used to elicit fibrogenic or inflammatory genes, respectively. The agents are added at a concentration of 10 µM, or at the indicated concentrations for a period of 30 min prior to the addition of TGF-β/TNF-α which are incubated for a further 20 hours before the addition, in some experiments of the glucocorticoid, dexamethasone (Dex, 30 nM) for the final 4 hours of the experiment, prior to harvest of cellular RNA to measure by RT-qPCR the levels of mRNA of marker genes for inflammation and fibrogenesis. The inhaled corticosteroid (ICS), budesonide is also used in some experiments. Furthermore, the supernatants are harvested at the end of the incubation periods in selected experiments to measure the levels of CSF-2 or IL-8 by ELISA as described in (Tran et al. (2005) British Journal of Pharmacology, 145, 123-131).

The selected potential candidate was also assayed in A549 alveolar epithelial cells, a commonly-used cell model for epithelial-mesenchymal transition (EMT) investigation. The agents were added at the indicated concentrations 30 min prior to the addition of TGF-β which was incubated for a further 24 hours. Then, the cellular RNA was harvested to measure the regulation of the EMT-associated genes and fibrogenic genes.

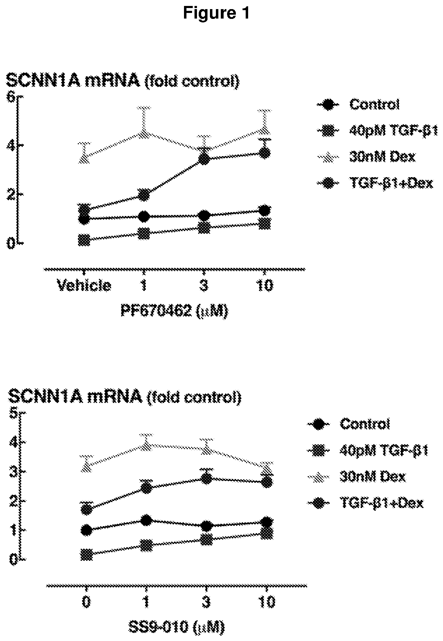

The data contained in Figures 1-7 and in Tables 1-4 suggest that the novel compound SS9-010 surprisingly shows higher potency than PF670462 with a similarly broad spectrum of activity on TGF-β-mediated actions in epithelial cells. This compound also has a higher cLogP than PF670462.

### Methods

### Cell culture

A549 alveolar epithelial cells (ATCC, Manasas, VA, USA) were cultured in DMEM containing 5% (v/v) FCS, 15mM HEPES, 0.2% (v/v) sodium bicarbonate, 2 mM L-glutamine, 1% (v/v) non-essential amino acids, 1% (v/v) sodium pyruvate, 5 IU/mL penicillin and 50 µg/mL streptomycin as previously described (S. Salem, T. Harris, J. Mok Shiueh Lian, M. Yuen Sin Li, C.R. Keenan, M.J. Schuliga, and A.G. Stewart (2012). Transforming growth factor-β impairs glucocorticoid activity in the A549 lung adenocarcinoma cell line. Br J Pharmacol. 166: 2036-2048).

Prior to experimentation, BEAS-2B and A549 cells were incubated in serumfree DMEM containing 0.25% bovine serum albumin (BSA) and insulin-transferrin-selenium-containing supplement (Monomed A; CSL, Parkville, Melbourne, Australia). Where indicated, cells were treated with one of the series of synthesised compounds (0.3 - 10 µM) prior to exposure to 100 pM TGF-β1 (R&D Systems, Minneapolis, MN) or 300 pM bFGF (Promega, Madison, WI).

### Analysis of gene expression

Total RNA was extracted from cultured cells using illustra RNAspin Mini RNA Isolation Kit (GE Healthcare). RNA extracts were reverse-transcribed into cDNA using High-Capacity RNA-to-cDNA Kit (Applied Biosystems). Real-time PCR was then performed using a QuantStudio 6 Flex Real-Time PCR System using iTaq Universal SYBR green supermix and the following thermal protocol: 50°C (2 min), 95°C (10 min), then 40 cycles of 95°C (15 sec), 60°C (1 min). The threshold cycle (CT) values determined for target genes were normalized against those obtained for 18S ribosomal RNA (18S rRNA), which was included as internal control. The generation of specific PCR products was confirmed by dissociation curve analysis.

### Immunofluorescence

A549 cells for immunofluorescence staining were seeded in ibiTreat 8 chamber slides (ibidi) and left to adhere overnight. Cells were then serum-starved for 16 h prior to pre-incubation with PF670462 (0.3 - 10 µM) for 30 min then TGF-β (100 pM) for 48 h. Cells were fixed in 10% neutral buffered formalin (Grale Scientific) for 15 min and non-specific binding sites were blocked by incubation with 5% normal goat serum /0.3% Triton X-100 in PBS for 1 h. E-Cadherin expression was detected using a rabbit monoclonal antibody (Clone 24E10; Cat#3195, Cell Signaling) followed by an AlexaFluor-488 conjugated anti-Rabbit F(ab')2 fragment secondary (Cat#4412, Cell Signaling). Specific binding was confirmed using an isotype control antibody (Clone DA1E rabbit IgG; Cat#3900, Cell Signaling). Cell nuclei were then stained with DAPI. Cells were imaged using a Leica SP5 confocal microscope (Biological Optical Microscopy Platform, University of Melbourne). Cell morphology and immunofluoresent staining was quantified using the Operetta High Content Imaging System (Biological Optical Microscopy Platform, University of Melbourne).

### Statistical analysis

All data are presented as mean ± SEM. Statistical comparisons among multiple groups were made by 1-way ANOVA with Dunnett's *post-hoc* test or 2-way ANOVA with Bonferroni *post-h*oc test. A P value of less than 0.05 was considered to be statistically significant.

### Discussion of results

In Figure 1, the effects of SS9-010 are contrasted with those of PF670462 in an assay in BEAS-B cells measuring the induction of the glucocorticoid-regulated gene, SCNN1A encoding the protein epithelial sodium channel alpha subunit. Serum-starved BEAS-2B cells were treated with CK1δ/ε inhibitor PF670462 (1, 3, 10 µM) or SS9-010 (1, 3, 10 µM) 30 min prior to TGF-β1 (40 pM) for 24 hours, then stimulated with dexamethasone (30 nM) for 4 hours. RNA was then extracted and analyzed by RT-qPCR. Data are presented as mean±SEM for n=4 independent experiments. The potency of PF670462 and SS9-010 were similar.

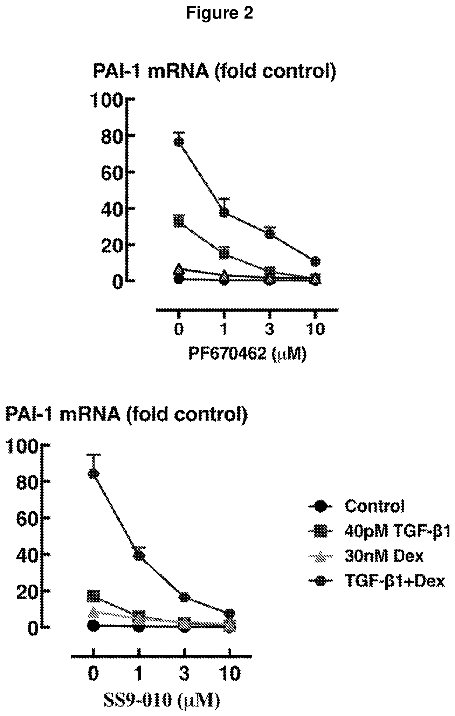

In the same experiment as described in Figure 1, the expression of the fibrogen plasminogen activator inhibitor-1 was measured (see Figure 2). The levels of this gene are increased by both TGF-β and by Dex and they synergise when added together. The CK1 inhibitors concentration-dependently reduce the effects of each of the stimuli and further reduce baseline expression. Their potency in this effect is similar, regardless of the condition being considered.

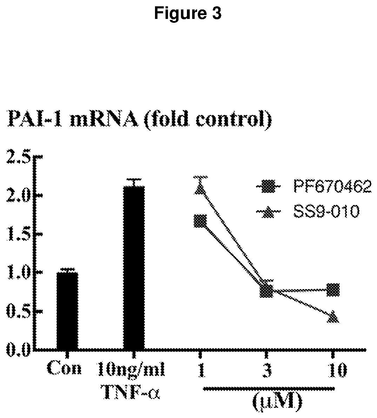

In a separate experiment BEAS-2B cells were exposed to TNF-α with(out) 30 min pretreatment with either SS9-010 or PF670462 (1-10µM). RNA was then extracted and analyzed by RT-qPCR. Each agent inhibited TNFα stimulated PAI-1 expression with similar potency and maxima (see Figure 3). Data are presented as mean±SD for n=1 (three technical replicates).

In serum-starved A549 cells, pretreatment with (0.1-10 µM) of either PF670462 or SS9-010, prior to TGF-β (100 pM) for further 24 hr, concentration-dependently inhibited PAI-1 and CTGF expression and restored TGF-β (100 pM)-induced suppression of E-cadherin. SS9-010 was significantly more potent that PF670462 in its actions on PAI-1 and ECad, but not on CTGF, as documented in Table 1 below.

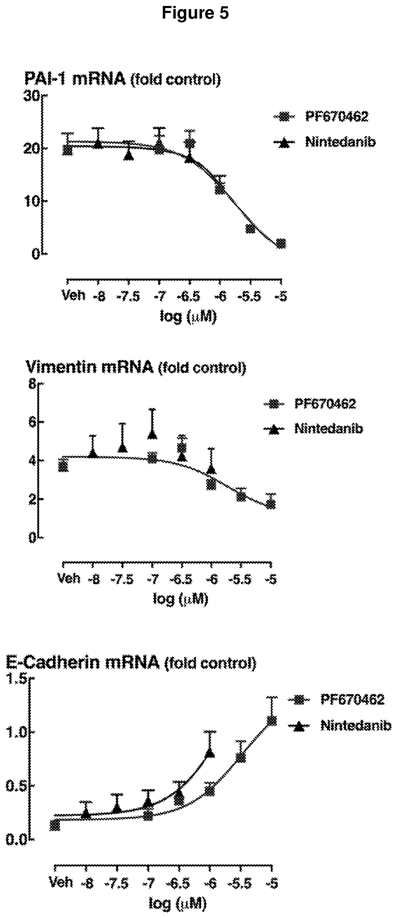

The effect of the anti-fibrotic agent nintedanib by way of contrast with the extent and potency of the actions of PF670462 is shown in Figure 5. Serum-starved A549 cells were pretreated with either PF670462 (0.1-10 µM) or nintedanib (0.01-1 µM) prior to TGF-β (100 pM) for a further 24h. RNA was then extracted and measured of PAI-1, Vimentin and E-Cadherin by RT-qPCR. Data are presented as mean±SEM for n=4 independent experiments.

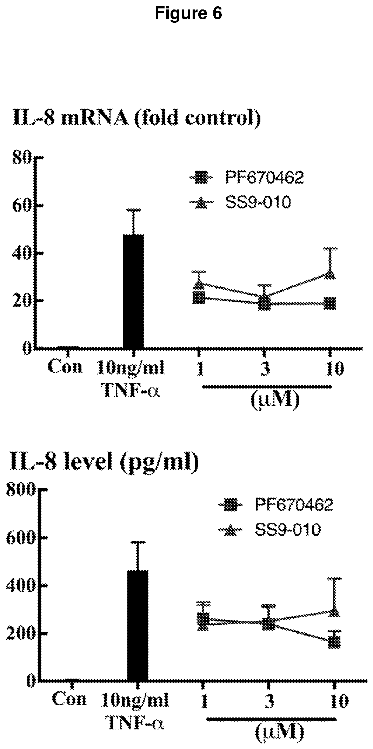

The effects of the CK1 inhibitors, PF670462 and SS9-010 (1-10 µM) on TNF-α (10 ng/mL)-induced increase in expression of IL-8 and on the level of immunoreactive IL-8 in the culture supernatant are shown in Figure 6. Serum-starved BEAS-2B cells were pretreated either SS9-010 or PF670462 (1-10 µM) 30 min prior to the exposure to TNF-α (10 ng/ml) for a further 24h. RNA was extracted and measured by RT-qPCR. Pro-inflammatory cytokines in the supernatants were measured by ELISA. Data in are presented as mean±SEM for n=3 independent experiments. The effects of each of the CK1 inhibitors was maximal at the lowest concentration used in this assay so there is no conclusion as to relative potency but it is clear that both agents are active inhibitors of the TNF-stimulated BEAS-2B cells.

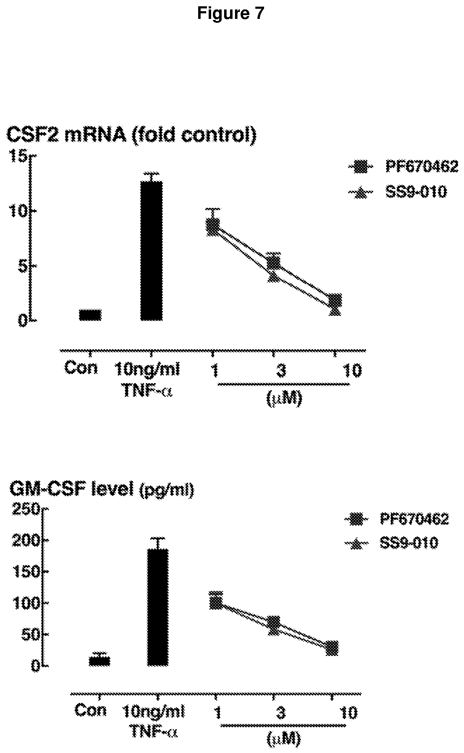

Figure 7 shows the expression of CSF-2 (GM-CSF) and its level in the supernatant from the same experiment depicted in Figure 6. In the case of CSF-2, a clear concentration-related decrease in the expression and protein levels of this colony stimulating factor are evident, but the two agents do not appear to differ in potency.

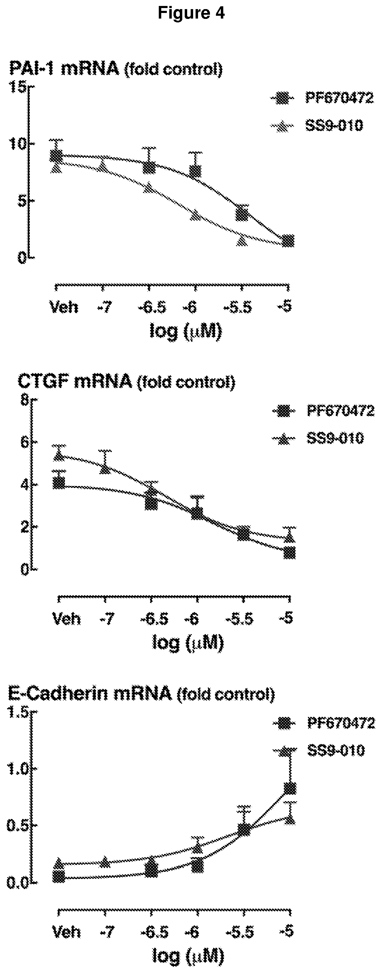

Table 1 shows the relative potency of PF670462 and SS9-010 in modulating TGFβ-induced gene expression changes linked to epithelial mesenchymal transition (EMT). EMT is a feature of the pathogenesis of fibrosis that is relevant to epithelial tumour metastases and fibrogenesis in organs such as heart, kidney liver and lung, and is known to contribute to idiopathic pulmonary fibrosis (IPF) as well as being thought to make a contribution to the fibrosis known to occur in asthma and COPD. SS9-010 shows significantly greater potency than PF670462 in attenuating the induction of the fibrogen, plasminogen-activator inhibitor-1 (PAI-1) and in preventing the repression of the E-cadherin gene which encodes a protein involved in maintaining the barrier function and polarity of differentiated epithelial cells. The potencies of PF670462 and SS9-010 in modulating induction of CTGF are not statistically distinguishable. SS9-010 is significantly more potent in regulating induction of the gene encoding one of the major collagen tissue remodelling genes, Col1A. The potency of these two agents in regulating vimentin induction by TGF-β could not be assessed, as the concentration-response relationship for PF670462 could not be fitted. However, the effects of SS9-010 were significant at 3 µM, whereas PF670462 (3 µM) had no effect. All of these data represent an analysis of the concentration-response relationships presented in Figure 4.

**Table 1.**

| **TGFβ-activated A549 gene expression** | **-Log IC₅₀ ± SEM (modulation of mRNA level)** | | **P value*** |
|---|---|---|---|
| **mRNA encoding** | **PF670462** | **SS9-010 Compound 34** | |
| PAI-1 induction | 5.41+0.25 | 6.18±0.14* | 0.023 |
| E-Cadherin repression | 5.08±0.19 | 5.67±0.14* | 0.047 |
| CTGF induction | 5.84±0.19 | 6.35±0.09 | 0.059 |
| N-Cadherin induction | 5.35±0.27 | 5.70±0.34 | 0.450 |
| Vimentin induction | Inactive at 3µM | 5.41±0.14 | Not applicable |
| Col 1A induction | 5.42±0.19 | 6.35±0.10* | 0.004 |

| | | | |
|---|---|---|---|
| *P value denotes the probability of the IC₅₀ values for PF670462 and compound SS9-010 as being equivalent. | | | |

**Table 2. Effects of PF670462 and analogues on TNF-α and TGF-β induced PAI-1 gene expression in BEAS-2B cells. mRNA levels of PAI-1 are expressed as fold control of n=4 independent experiments (or as indicated).**

| **Agent (µM)** | **vehicle** | **TNF-α (n=2)** | **TGF-β** |
|---|---|---|---|
| Vehicle | 1.00 | 2.07 | 31.5 ± 3.1 |
| PF670462 (3 µM) | | 0.74 | 1.30 ± 0.18 |
| PF670462 (10 µM) | 0.26 ± 0.04 | 0.47 | 1.64 ± 0.27 |
| SS9-010 (10 µM) | 0.18 + 0.00 | 0.43 | 0.64 ± 0.05 |
| SS8-058 Cmpd 7 (10 µM) | 0.37 ± 0.04 | 0.91 | 9.14 ± 1.23 |
| SS8-070 Cmpd 9 (10 µM) | 0.41 | 0.79 | 6.57 (n=2) |
| SS9-074 Cmpd 10 (10 µM) | 0.68 | 1.83 | 12.3 (n=2) |
| SS8-122 Cmpd 17 (10 µM) (n=2) | 1.45 | 2.76 | |
| SS8-186 Cmpd 27 (10 µM) (n=4) | 0.75 ± 0.07 | 1.54 | 6.58 ± 1.04 |

**Table 3. Effects of PF670462 and analogues on TNFα and TGFβ induced Col 1A gene expression in BEAS-2B cells. mRNA levels of Col1A are expressed as fold control of n=4 independent experiments (or as indicated).**

| **Agent (µM)** | **vehicle** | **TNF-α (n=2)** | **TGF-β** |
|---|---|---|---|
| Vehicle | 1.00 | 0.78 | 10.3 ± 1.4 |
| PF670462 (3µM) | 0.47 ± .04 | 0.52 | 1.91 ± 0.32 |
| PF670462 (10µM) | 0.65 ± 0.02 | 0.65 | 1.53 ± 0.33 |
| SS9-010 (10 µM) | 0.30 ±0.02 | 0.37 | 1.09 ± 0.10 |
| SS8-058 Cmpd 7 (10 µM) | 0.91 ± 0.19 | 1.11 | 9.68 ± 1.25 |
| SS8-070 Cmpd 9 (10 µM) | 1.17 | 0.88 | 8.52 (n=2) |
| SS9-074 Cmpd 10(10 µM) | 1.61 | 2.14 | 12.2 (n=2) |
| SS8-122 Cmpd 17 (10 µM) (n=2) | 1.39 | 1.56 | 8.07 |
| SS8-186 Cmpd 27 (10 µM) (n=4) | 0.50 ± 0.12 | 0.56 | 3.14 ± 0.20 |

**Table 4. Effects of PF670462 and analogues on TGF-β-induced suppression of dexamethasone-induced SCNN1A gene expression in BEAS-2B cells. mRNA levels of SCNN1A are expressed as fold control of n=4 independent experiments for PF670462, SS9-010 and SS8-186 or n=1 (in triplicate) for the other cmpds.**

| **Agent (µM)** | **vehicle** | **TGF-β** | **Dex** | **TGF-β/Dex** |
|---|---|---|---|---|
| Vehicle | 1.00 | 0.29 ± 0.04 | 2.86 ± 0.13 | 1.62 ± 0.09 |
| PF670462 (3µM) | 1.11± 0.10 | 0.91±0.08 | 3.36 ± 0.25 | 2.56 ± 0.08 |
| SS9-010 (3 µM) | 1.14 ± 0.05 | 0.67 ± 0.03 | 3.77 ± 0.33 | 2.77 ± 0.31 |
| SS8-058 Cmpd 7 (10µM) | 2.62 | 0.91 | 6.57 | 3.77 |
| SS8-070 Cmpd 9 (10µM) | 1.63 | 0.42 | 3.79 | 2.15 |
| SS9-074 Cmpd 10 (10µM) | 1.22 | 0.36 | 3.46 | 3.27 |
| SS8-12 Cmpd 17 (10µM) | 0.86 | 0.15 | 4.26 | 1.67 |
| SS8-186 Cmpd 27 (10µM) | 1.36 ± 0.17 | 0.42 ± 0.06 | 3.06 ± 0.34 | 1.84 ± 0.25 |

### In vivo experiments

The relative activity of agents shown *in vitro* assays are further tested in bleomycin-model of pulmonary fibrosis.

### Bleomycin model of pulmonary fibrosis

All animal experiments were carried out in accordance with ethical guidelines from the University of Melbourne Animal Ethics Committee (AEEC#1513736.1). Sixto 8-week old 20-25 g C57BI/6 mice (ARC, Perth, Australia) received treatment with 35µL of saline or bleomycin (105mU per mouse) on day 0 through intranasal administration, as previously described (Langenbach et al., (2007). Can J Physiol Pharmacol, 85, 727-738). Acute and chronic bleomycin mouse models were used to assess the effect of SS9-010 on pulmonary fibrosis *in vivo.* SS9-010 was administered systemically through intraperitoneal injection or locally to the lungs through inhalation of an aerosol generated using an oxygen concentrator connected to a hudson nebuliser operating at 5 L/min for 15 minutes. Mice that did not receive SS9-010 treatment were administered vehicle (10% DMSO, 90% peanut oil). Mice were allowed food and water *ad libitum* for the duration of all studies. At the end of each study, bronchoalveolar lavage (BAL) was performed from which leukocyte cell types were enumerated and acelluar protein content was determined as previously described (Langenbach, *et al.,* 2007), lungs were dissected and snap frozen. Gene expression were determined from pulverised frozen lung tissue.

### BAL protein content measurement

Total protein content in acellular BAL fluid was assessed using the BioRad protein assay method.

### BAL cell number measurement

BAL cells in BAL fluid were stained with Erythrosin B (EB) and counted manually with the aid of a hemocytometer. Total BAL cell number was calculated by accounting for the return volume of BAL fluid.

### Discussion of results

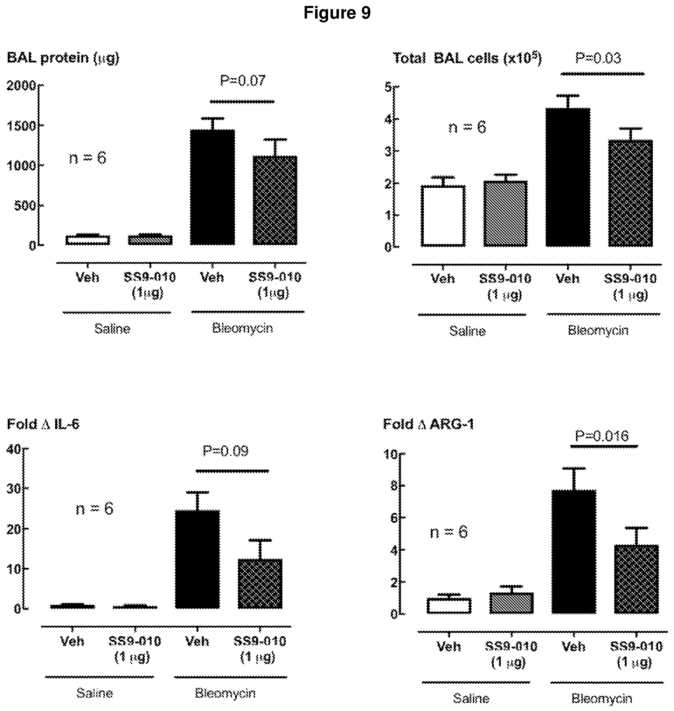

The effects of inhaled SS9-010 on bleomycin-induced BAL protein leakage, BAL cell recruitment, lung weight, as well as lung fibrogenic gene expression in mouse lungs are shown in Figure 9. Female C57BI/6 mice received a transnasal dose of bleomycin or saline on day 0. SS9-010 was administered day -1 to day 3 by daily inhalation of aerosol generated by nebulizing from a concentration of 1 mg/mL for a period of 15 min once daily. Bronchoalveolar lavage (BAL) protein and BAL cell number were assessed. Lung fibrogenic gene expression was also assessed on day 3. Data are presented as mean ± SEM (n=6). SS9-010 showed an effect on BAL protein and lung fibrogenic gene expression, and modest suppression on BAL cell number.

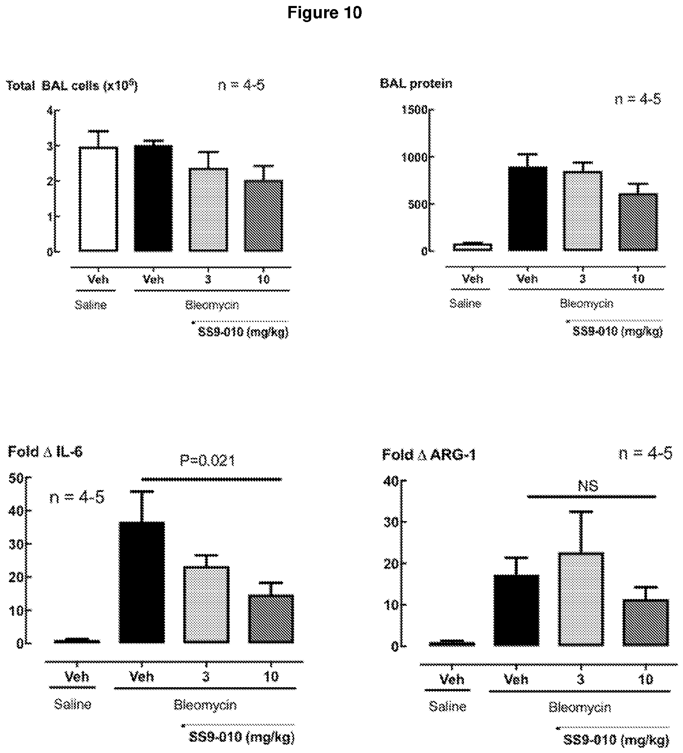

The effects of intraperitoneal injection of SS9-010 on bleomycin-induced BAL protein leakage, BAL cell recruitment, and lung fibrogenic gene expression in mouse lungs are shown in Figure 10. Female C57BI/6 mice were intraperitoneally administered bleomycin or saline on day 0. SS9-010 (3 or 10 mg/kg/day, ip) was administered from day -1 to 3. Bronchoalveolar lavage (BAL) protein and BAL cell number were assessed, and showed a positive trend. Lung fibrogenic gene expression was also assessed on day 3. Data are presented as mean ± SEM (n=4 sal, n=5 other groups). Systemic SS9-010 (3 or 10 mg/kg/day, ip) had no effect on BAL protein or BAL cell number. Induction of IL-6 was reduced by SS9-010 treatment, whereas arginase-1 was not.

In studies to ascertain the effect of oral administration of either PF670462 or SS8-058 we harvested the liver on day 3 from mice that had been treated transnasally with bleomycin on day 0 to measure gene expression of products that are implicated in fibrogenesis. Mice were treated once daily by gavage from day -1 to day 3 with either PF670462 or SS8-058 (30 mg/kg, po). Each of the treatments significantly reduced the level of each of the fibrogenic genes.

### Measurement of airway hyper-responsiveness in vivo

***Acute ovalbumin-induced asthma model*:** BALB/c Mice were sensitised with a combination of ovalbumin (50 µg), aluminium hydroxide (20 mg/mL of adjuvant) and saline by an intraperitoneal injection. 200 µL of the ovalbumin mixture was administered to each mouse on day 0, followed by a repeat dose on day 14 via an intraperitoneal injection using a 22 gauge needle. On days 21 to 28 mice were exposed daily to 1% aerosolised ovalbumin in saline for 30 minutes at a rate of 2 ml/minute by a nebuliser (deVilbiss). SS9-010 was administered locally to the lungs through inhalation of an aerosol generated using an oxygen concentrator connected to a hudson nebuliser operating at 5 L/min for 15 minutes from day 21 to 28. The estimated deposited dose was 1 µg.

***Lung function measurement:*** Lung function was measured using a modification of the low-frequency forced oscillation technique and a small-animal ventilator (flexiVent; Scireq, Montreal, QC, Canada) as described previously (Bozanich et al., (2008) Respir Physiol Neurobiol, 162, 190-196). On the day of necropsy, mice were anesthetised under ketamine and xylazine, and placed onto a lung function testing apparatus (FlexiVent). To measure airway hyperresponsiveness mice were administered methacholine by an aerosol through the tracheal cannula at a dose range of 0.1 to 30 mg/ml for 5 minutes. The lung function was performed as a terminal procedure; each mouse was deeply anaesthetised with a combination of ketamine (150 mg/kg) and xylazine (15 mg/kg) in saline intraperitoneally with a 27 gauge needle. Once anaesthesia was achieved a surgical incision, 2 cm long using a scalpel blade was made sagittally in a distal cutting motion through the subcutaneous tissue before the tracheal muscle layer. Using blunt dissection, the muscle layer around the trachea was cut longitudinally to expose the trachea. A 1.27 mm intratracheal tube, 1 cm long was inserted into the trachea and tied off with silk sutures and the mouse was ventilated on the flexiVent. Mice were placed on a SCIREQ FlexiVent system and ventilated to measure lung function at a constant breath rate (150 breaths per minute) with the tracheal cannula attached to the system. Respiratory mechanics were assessed on one mouse for approximately 60 minutes. Mice were administered either via intratracheal cannula or aerosol increasing doses of methacholine to establish airway hypperresponsiveness. Respiratory impedance (Zᵣₛ) was measured and partitioned into airway and parenchymal components to allow calculation of Newtonian resistance (Rn, equivalent to airway resistance (Raw) because of the high compliance of the chest wall, tissue damping (G) and elastance (H), as previously described (Larcombe et al., (2011) Influenza Other Respir Viruses, 5, 334-342).

### Discussion of results

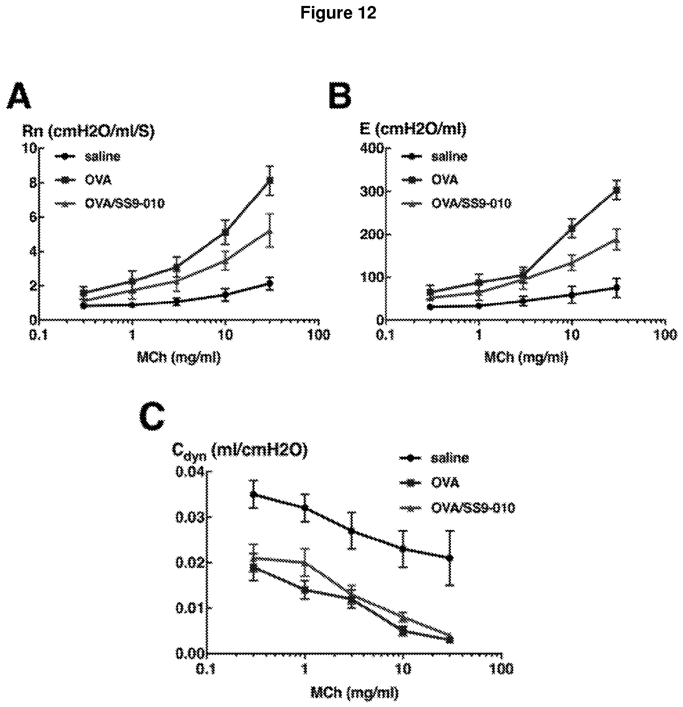

Airway hyper-responsiveness (AHR) in response to inhaled methacholine (MCh) measured in sham- or ovalbumin (OVA)-sensitized BALB/c mice that received inhalation of SS9-010 (estimated deposited dose of 1 µg) or saline is shown in Figure 12. Resistance (Rn), which is representative of the level of constriction in the lungs was assessed. Elastance (E) captures the elastic stiffness of the respiratory system at the ventilation frequency. Compliance (C) describe the ease with which the respiratory system can be extended. Data are presented as mean ± SEM (n=6). The data demonstrated a significant increase in central airway resistance (Rn) and elastance in OVA-challenged mice. This induction was attenuated in mice received SS9-010 treatment. No significant difference was observed in OVA/SS9-010 group compared to OVA alone in terms of compliance changes.

### Synthesis

### General

Proton nuclear magnetic resonance spectra (¹H NMR, 400, 600 MHz) and proton decoupled carbon-13 nuclear magnetic resonance spectra (¹³C NMR, 100, 150 MHz) were obtained in deuterated solvents, with residual protiated solvent as internal standard. Chemical shifts are followed by multiplicity, coupling constant(s) (J, Hz), integration and assignments where possible. Flash chromatography was carried out according to the procedure of Still *et al.* using an automated system.¹ Analytical thin layer chromatography (t.I.c.) was conducted on aluminium-backed 2 mm thick silica gel 60 GF₂₅₄ and chromatograms were visualized under an ultraviolet lamp. High resolution mass spectra (HRMS) were obtained by ionizing samples using electrospray ionization (ESI) and a time of flight mass analyzer. Dry THF and CH₂Cl₂ were obtained by the method of Pangborn *et al.*² Pet. spirits refers to petroleum ether, boiling range 40-60 °C. All other commercially available reagents were used as received.

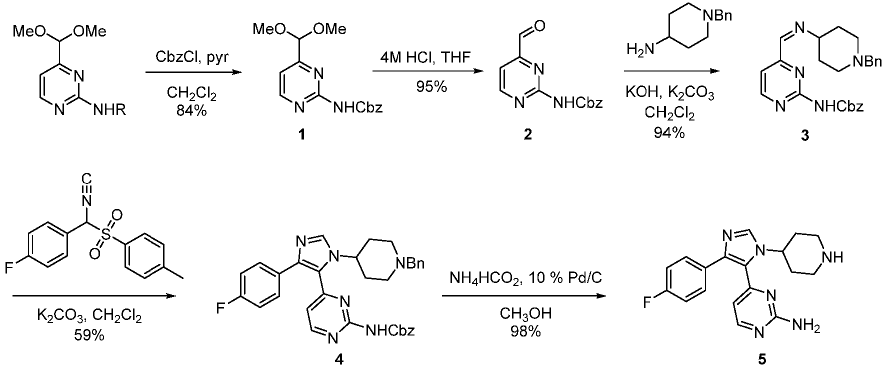

### Benzyl 4-(dimethoxymethyl)pyrimidin-2-ylcarbamate (1)

A solution of benzyl chloroformate (2.57 ml, 18.1 mmol) in CH₂Cl₂ (2.5 ml) was added to a mixture of 4-dimethoxymethyl pyrimidin-2-ylamine (0.330 g, 1.95 mmol) and pyridine (0.2 ml) in CH₂Cl₂ (7.5 ml) at -18 °C. The solution was gradually warmed to room temperature and stirring was continued overnight. TLC of the reaction mixture indicated starting material was not completely consumed. Hence, the reaction mixture was recooled to -18 °C and additional benzyl chloroformate (0.250 ml, 1.81 mmol) was added. The reaction mixture was diluted with CH₂Cl₂, and washed with water, dried (MgSO₄), filtered and concentrated. Flash chromatography of the residue (EtOAc/pet. spirits 2:1) afforded **1** as a white solid (0.496 g, 84%). ¹H NMR (CDCl₃, 400 MHz) δ 3.39 (2 × 3 H, s), 5.21 (1 H, s), 5.24 (2 H, s), 7.17 (1 H, d, *J* = 5.2 Hz), 7.32-7.41 (5 H, m), 8.33 (1 H, br s), 8.63 (1 H, d, *J* = 5.2 Hz); ¹³C NMR (CDCl₃, 100 MHz) δ 54.0, 67.5, 102.5, 113.4, 128.5, 128.6, 128.7, 135.8, 151.5, 157.4, 159.3, 167.0; HRMS (ESI⁺) calcd for C₁₅H₁₈N₃O₄ (M+H) 304.1297. Found 304.1292.

### Benzyl 4-formylpyrimidin-2-ylcarbamate hydrochloride (2)

Aqueous 4 M HCl (2 ml) was added to a solution of **1** (0.350 g, 1.15 mmol) in THF (1 ml). The resulting mixture was heated to 40 °C overnight and then cooled to room temperature. The reaction mixture containing the hydrochloride salt of the aldehyde was used directly in the next step without isolation.

### Benzyl (4-(((1-benzylpiperidin-4-yl)imino)-methyl)piperidine-2-yl)carbamate (3)

45% aq. KOH (0.448 g, 7.98 mmol) was added to an ice-cold solution of crude aldehyde **2** (0.290 g, 1.12 mmol) in aq. HCl (2 ml, 7.90 mmol), while the temperature was maintained below 15 °C. To the neutralized solution, CH₂Cl₂ (5 ml) and K₂CO₃ (0.158 g, 1.14 mmol) were added followed by 4-amino-1-benzylpiperidine (0.141 g, 0.838 mmol). The reaction mixture was gradually warmed to room temperature and stirring was continued for 2 h. ¹H NMR analysis indicated -10% aldehyde remaining hence an additional amount of 4-amino-1-benzylpiperidine (0.035 g, 0.025 mmol) was added, and stirring was continued further for 1 h. The reaction mixture was diluted with CH₂Cl₂, washed with water, dried (MgSO₄), filtered and concentrated. The crude material was used in the next step without purification (0.450 g, 94%). ¹H NMR (CDCl₃, 400 MHz) δ 1.71-1.74 (2 H, m), 1.83-1.93 (2 H, m), 2.13-2.18 (2 H, m), 2.92-2.95 (2 H, m), 3.33-3.38 (1 H, m), 5.25 (2 H, s), 7.30-7.43 (10 H, m), 7.59 (1 H, d, *J* = 5.2 Hz), 8.26 (1 H, br s), 8.61 (1 H, d, *J* = 4.8 Hz); ¹³C NMR (CDCl₃, 100 MHz) δ 33.2, 51.9, 63.2, 67.6, 112.6, 127.2, 128.4, 128.6, 128.7, 128.8, 129.3, 135.7, 151.5, 157.7, 158.7, 159.1, 163.0; HRMS (ESI⁺) calcd for C₂₅H₂₈N₅O₂ (M+H) 430.2243. Found 430.2239.

### Benzyl (4-(1-(1-benzylpiperidin-4-yl)-4-(4-fluorophenyl)-1H-imidazol-5-yl)pyrimidin-2-yl)carbamate (4)

α-(*p*-Toluenesulfonyl)-4-fluorobenzylisonitrile (0.262 g, 0.905 mmol) and 20% aq. K₂CO₃ (0.250 g, 1.808 mmol) were added to a solution of imine **3** (0.350 g, 0.815 mmol) in CH₂Cl₂ at 20 °C whilst stirring was continued overnight. At this point, additional imine (0.038 g, 0.091 mmol) and K₂CO₃ (0.090 g, 0.651 mmol) were added and the reaction mixture was stirred at 30 °C overnight. The reaction mixture was diluted with CH₂Cl₂, and washed with water, dried (MgSO₄), filtered and concentrated. Flash chromatography of the residue (MeOH/EtOAc 1:4) afforded **4** as a light yellow solid (0.345 g, 59%). ¹H NMR (DMSO-d₆, 400 MHz) δ 1.91-1.94 (4 H, m), 2.01-2.08 (2 H, m), 2.82 (2 H, br d, *J* = 10.8 Hz), 3.45 (2 H, s), 4.84-4.88 (1 H, m), 5.20 (2 H, s), 6.86 (1 H, d, *J* = 5.2 Hz), 7.13-7.17 (2 H, m), 7.22-7.45 (2 H, m), 8.14 (1 H, s), 8.51 (1 H, d, *J* = 5.2 Hz), 10.73 (1 H, s); ¹³C NMR (DMSO-d₆, 100 MHz) δ 32.8, 52.0, 53.1, 61.9, 65.8, 115.2, 116.4, 124.0, 126.9, 127.9, 129.0, 128.1, 128.4, 128.8, 129.9, 13.09, 136.6, 137.3, 138.5, 141.2, 151.8, 157.9, 158.2, 158.6, 161.5; HRMS (ESI⁺) calcd for C₃₂H₃₂FN₆O₂ (M+H) 563.2571. Found 563.2565.

### 4-(4-(4-Fluorophenyl)-1-(piperidine-4-yl)-1H-imidazol-5-yl)pyrimidin-2-amine (5)

10% Pd/C (0.1 g) and ammonium formate (0.390 g,6.22 mmol) were added to a solution of **4** (0.350 g, 0.622 mmol) in methanol (5 ml).The resulting reaction mixture was heated to 50 °C and stirred overnight. The reaction mixture was filtered through Celite and concentrated under reduced pressure to afford a residue, which was used without purification (0.206 g, 98%). ¹H NMR (MeOH-d₄, 400 MHz) δ 2.08-2.17 (2 H, m), 2.37-2.40 (2 H, m), 3.02-3.09 (2 H, m), 3.41-3.47 (2 H, m), 4.85-4.94 (1 H, m), 6.40 (1 H, d, *J* = 5.2 Hz), 7.06-7.11 (2 H, m), 7.39-7.43 (2 H, m), 8.03 (1 H, s), 8.12 (1 H, d, *J* = 5.2 Hz), 8.53 (1 H, s, NH); ¹³C NMR (MeOH-d₄, 100 MHz) δ 27.6, 28.3, 36.3, 36.5, 40.6, 42.9, 59.7, 113.3, 115.3, 115.5, 129.9, 130.0, 135.5, 142.2, 151.6, 158.6, 159.7, 161.2, 163.2, 163.7; HRMS (ESI⁺) calcd for C₁₈H₂₀FN₆ (M+H) 339.1733. Found 339.1731.

### Octanal (6)

A solution of 1-octanol (0.50 g, 3.83 mmol) in CH₂Cl₂ (5 ml) was added dropwise to a stirring suspension of pyridinium dichromate (2.16 g, 5.75 mmol) and Celite (0.5 g) in CH₂Cl₂ (5 ml). The resulting mixture was stirred at room temperature for 3 h. The solvent was removed under vacuum and the crude obtained was purified by flash chromatography (EtOAc/petspirits 1:9) to give **6** as a colorless oil (0.350 g, 71%). ¹H NMR (CDCl₃, 400 MHz) δ 0.88 (3 H, t, *J* = 8 Hz), 1.20-1.30 (8 H, m), 1.59-1.64 (2 H, m), 2.41 (2 H, t, *J* = 8 Hz), 9.76 (1 H, s).

### 4-Phenyl 1-butanal (8)

Dess-Martin periodinane (1.69 g, 3.98 mmol) was added to a solution of 4-phenyl-1-butanol (0.50 g, 3.32 mmol) in CH₂Cl₂ (10 ml) at 0 °C. the resulting reaction mixture was stirred at 0 °C for 30 min, followed by stirring at room temperature for 1.5 h. The reaction mixture was quenched with 10% sodium thiosulfate (50 ml) and the product was extracted into CH₂Cl₂. The organic layer was separated, and washed with 1 M aq NaOH (50 ml) followed by water (50 ml), dried (Na₂SO₄), filtered and concentrated. The crude material was used directly for the next reaction (0.485 g, 98%). ¹H NMR (CDCl₃, 400 MHz) δ 1.93-2.01 (2 H, pent, *J* = 7.6 Hz), 2.46 (2 H, td, *J* = 1.2, 7.2 Hz), 2.66 (2 H, t, *J* = 8 Hz), 7.17-7.22 (2 H, m), 7.26-7.31 (3 H, m), 9.76 (1 H, t, *J =* 1.2 Hz); ¹³C NMR (CDCl₃, 100 MHz) δ 23.8, 35.2, 43.3, 126.2, 128.4, 128.6, 141.3, 202.5.

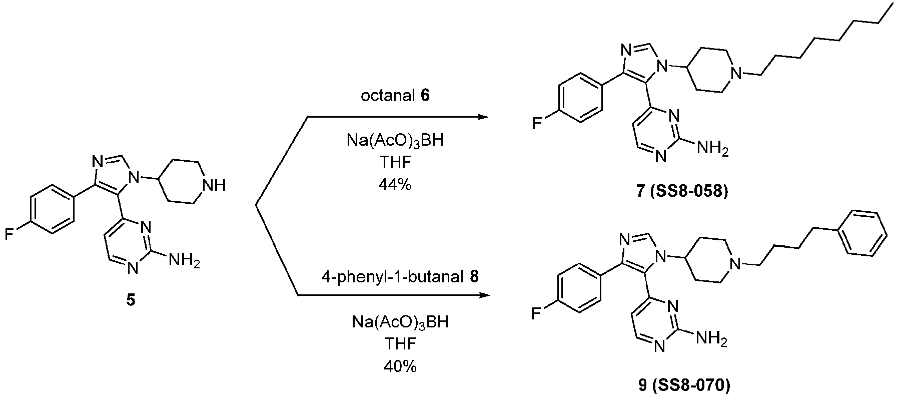

### 4-(4-(4-Fluorophenyl)-1-[1-octylpiperidin-4-yl]-1H-imidazol-5-yl)pyrimidin-2-amine (7; SS8-058)

Octanal **6** (0.133 g, 1.04 mmol) was added to a solution of **5** (0.250 g, 0.739 mmol) in dry THF (5 ml). The resulting reaction mixture was stirred at room temperature for 15 min. Sodium triacetoxyborohydride (0.454 g, 2.14 mmol) was added and the mixture was cooled to maintain the temperature below 37 °C. Stirring was continued for 2 h, at which point TLC indicated presence of ~40% amine. Hence, additional aldehyde **6** (0.095 g, 0.721 mmol) and sodium triacetoxyborohydride (0.313 g, 1.42 mmol) were added and stirring was continued overnight. The reaction mixture was slowly quenched with sat. aq. NaHCO₃ solution, followed by dilution with EtOAc. The organic layer was separated, and washed with water, dried (MgSO₄), filtered and concentrated. Flash chromatography of the residue (MeOH/EtOAc/Et₃N 6:93:1) afforded **7** as a light yellow solid (0.145 g, 44%). ¹H NMR (CDCl₃, 400 MHz) δ 0.88 (3 H, t, *J* = 6.8 Hz), 1.21-1.36 (10 H, m), 1.49-1.68 (2 H, m), 2.01-2.11 (4 H, m), 2.33-2.37 (2 H, m), 3.05-3.06 (2 H, m), 4.50-4.57 (1 H, m), 5.08 (2 H, br s), 6.50 (1 H, d, *J* = 4.8 Hz), 6.97-7.02 (2 H, m), 7.43-7.46 (2 H, m), 7.77 (1 H, s), 8.18 (1 H, d, *J* = 5.2 Hz); ¹³C NMR (CDCl₃, 150 MHz) δ 14.3, 22.8, 27.4, 27.8, 29.4, 29.7, 32.0, 33.7, 53.2, 54.3, 58.7, 113.1, 115.4, 115.6, 124.9, 130.06, 130.12, 130.7, 136.0, 138.9, 142.1, 158.7, 159.4, 161.5, 163.1, 163.5; HRMS (ESI⁺) calcd for C₂₆H₃₆FN₆ (M+H) 451.2985. Found 451.2978.

### 4-(4-(4-Fluorophenyl)-1-[1-(4-phenylbutyl)piperidin-4-yl)-1H-imidazol-5-yl)pyrimidin-2-amine (9; SS8-070)

A mixture of 4-phenyl-1-butanal 8 (0.061 g, 0.404 mmol) and **5** (0.10 g, 0.296 mmol) in dry THF (5 ml) was stirred at room temperature for 15 min. Sodium triacetoxyborohydride (0.182 g, 0.859 mmol) was added and the mixture was cooled to maintain the temperature below 37 °C. Stirring was continued for 2 h, at which point TLC indicated presence of ~50% amine. Hence, additional aldehyde 8 (0.043 g, 0.289 mmol) and sodium triacetoxyborohydride (0.090 g, 0.429 mmol) were added and stirring was continued overnight. The reaction mixture was slowly quenched with sat. aq. NaHCO₃ solution, followed by dilution with EtOAc. The organic layer was separated, and washed with water, dried (MgSO₄), filtered and concentrated. Flash chromatography of the residue (MeOH/EtOAc/Et₃N 5:94:1) afforded **9** as a white solid (0.028 g, 40%). ¹H NMR (CDCl₃, 400 MHz) δ ¹H NMR (CDCl₃, 400 MHz) δ 1.24-1.27 (4 H, m), 1.50-1.68 (4 H, m), 1.99-2.11 (2 H, m), 2.36-2.40 (2 H, t, *J* = 8 Hz), 2.62-2.65 (2 H, t, *J* = 8 Hz), 3.02-3.04 (2 H, m), 4.49-4.58 (1 H, m), 5.06 (2 H, br s), 6.50 (1 H, d, *J* = 4.8 Hz), 6.97-7.02 (2 H, m), 7.17-7.19 (3 H, m), 7.26-7.29 (2 H, m), 7.43-7.46 (2 H, m), 7.76 (1 H, s), 8.18 (1 H, d, *J* = 4.8 Hz); ¹³C NMR (CDCl₃, 100 MHz) δ 27.0, 29.5, 29.9, 33.6, 35.9, 53.2, 54.2, 58.4, 113.1, 115.4, 115.6, 124.8, 125.9, 128.45, 128.53, 130.07, 130.13, 130.6, 136.0, 142.2, 142.5, 158.7, 159.3, 161.5, 163.1, 163.5; HRMS (ESI⁺) calcd for C₂₈H₃₂FN₆ (M+H) 471.2672. Found 472.2681.

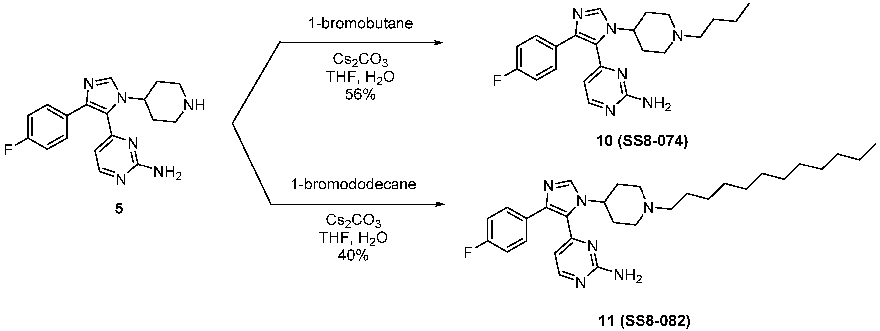

### 4-(1-(1-Butylpiperidin-4-yl)-4-(4-fluorophenyl)-1H-imidazol-5-yl)pyrimidin-2-amine (10; SS8-074)

A mixture of 1-bromobutane (0.020 g, 0.147 mmol), **5** (0.050 g, 0.147 mmol) and cesium carbonate (0.192 g, 0.589 mmol) in THF (3 ml) and water (1 ml) was heated to 70 °C and stirred overnight. TLC indicated presence of ~50% amine, hence additional 1-bromobutane (0.020 g, 0.147 mmol) and cesium carbonate (0.96 g, 0.295 mmol) were added and stirring was continued overnight. The reaction mixture was diluted with EtOAc, and washed with water and brine, dried (MgSO₄), filtered and concentrated. Flash chromatography of the residue (MeOH/EtOAc/Et₃N 5:94:1) afforded **10** as a white solid (0.028 g, 56%). ¹H NMR (CDCl₃, 400 MHz) δ 0.92 (3 H, t, *J* = 7.2 Hz), 1.25-1.37 (4 H, m), 1.42-1.53 (2 H, m), 2.03-2.11 (4 H, m), 2.36-2.40 (2 H, m), 3.06-3.08 (2 H, m), 4.50-4.61 (1 H, m), 5.12 (2 H, br s), 6.50 (1 H, d, *J* = 5.2 Hz), 6.97-7.01 (2 H, m), 7.42-7.46 (2 H, m), 7.77 (1 H, s), 8.18 (1 H, d, *J* = 4.8 Hz); ¹³C NMR (CDCl₃, 150 MHz) δ 14.2, 21.0, 29.9, 33.5, 53.2, 54.1, 58.3, 113.1, 115.4, 115.6, 124.8, 130.06, 130.13, 130.6, 136.0, 142.2, 158.7, 159.3, 161.5, 163.1, 163.5; HRMS (ESI⁺) calcd for C₂₂H₂₈FN₆ (M+H) 395.2359. Found 395.2348.

### 4-(1-(1-Dodecylpiperidin-4-yl)-4-(4-fluorophenyl)-1H-imidazol-5-yl)pyrimidin-2-amine (11; SS8-070)

A mixture of 1-bromododecane (0.043 g, 0.147 mmol), **5** (0.050 g, 0.147 mmol), and cesium carbonate (0.192 g, 0.589 mmol) in THF (3 ml) and water (1 ml) was heated to 70 °C and stirred overnight. TLC indicated presence of ~10% amine, hence additional 1-bromododecane (0.008 g, 0.029 mmol) and cesium carbonate (0.025 g, 0.074 mmol) were added and stirring was continued for 3 h. The reaction mixture was diluted with EtOAc, and washed with water and brine, dried (MgSO₄), filtered and concentrated. Flash chromatography of the residue (MeOH/EtOAc/Et₃N 5:94:1) afforded **11** as a white solid (0.031 g, 40%). ¹H NMR (CDCl₃, 400 MHz) δ 0.88 (3 H, t, *J* = 7.2 Hz), 1.09-1.28 (18 H, m), 1.42-1.49 (2 H, m), 1.96-2.15 (4 H, m), 2.35-2.38 (2 H, m), 3.04-3.11 (2 H, m), 4.51-4.59 (1 H, m), 5.07 (2 H, s), 6.50 (1 H, d, *J* = 5.2 Hz), 6.97-7.01 (2 H, m), 7.43-7.46 (2 H, m), 7.77 (1 H, s), 8.18 (1 H, d, *J* = 5.2 Hz); ¹³C NMR (CDCl₃, 150 MHz) δ 14.3, 22.9, 27.4, 27.8, 29.5, 29.7, 29.78, 29.80, 29.83, 32.1, 33.7, 52.2, 52.3, 58.7, 113.1, 115.4, 115.6, 124.9, 130.06, 130.13, 130.6, 136.0, 142.1, 158.7, 159.4, 161.5, 163.1, 163.5; HRMS (ESI⁺) calcd for C₃₀H₄₄FN₆ (M+H) 507.3611. Found 507.3613.

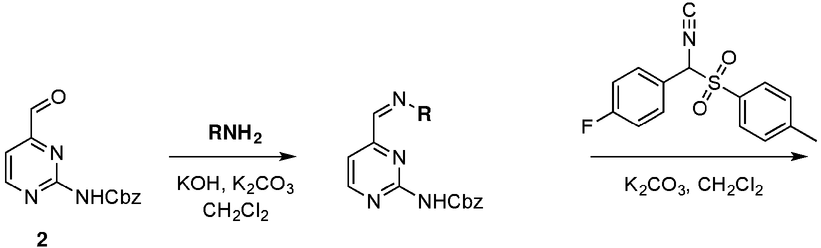

### RNH₂

| | |
|---|---|
| cyclohexylamine | **12** (85%) |
| 1-adamantyl amine | **15** (94%) |
| cycloheptylamine | **18** (91%) |
| exo-2-aminonorbornane | **21** (71%) |
| methylamine | **24** * |
| ethylamine | **27** * |
| *tert*-butylamine | **30** * |
| cyclopropylamine | **33** * |
| cyclobutylamine | **36** * |
| 3-oxetanamine | **39** * |
| cyclohexylmethylamine | **42** * |

| | |
|---|---|
| ** not isolated* | |

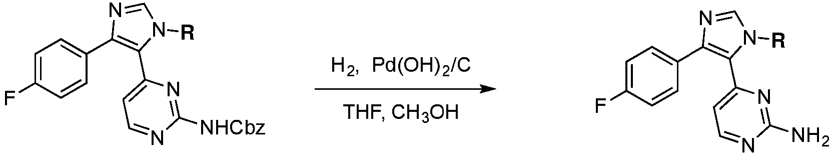

### R

| | |
|---|---|
| cyclohexyl | **13** (77%) |
| adamantan-1-yl | **16** (15%) |
| cycloheptyl | **19** (91%) |
| exo-2-norbornyl | **22** (23%) |
| methyl | **25** (37%) |
| ethyl | **28** (33%) |
| *tert-*butyl | **31** (31%) |
| cyclopropyl | **34** (22%) |
| cyclobutyl | **37** (39%) |
| 3-oxetanyl | **40** (47%) |
| cyclohexylmethyl | **43** (50%) |

### R

| | | |
|---|---|---|
| cyclohexyl | **14 (PF-670462)** | (83%) |
| adamantan-1-yl | **17 (SS8-122)** | (91%) |
| cycloheptyl | **20 (SS8-138)** | (92%) |
| exo-2-norbornyl | **23 (SS8-154)** | (60%) |
| methyl | **26 (ZH-142)** | (72%) |
| ethyl | **29 (ZH-6)** | (84%) |
| *tert-*butyl | **32 (ZH9-190)** | (69%) |
| cyclopropyl | **35 (ZH-58)** | (99%) |
| cyclobutyl | **38 (ZH-62)** | (51%) |
| 3-oxetanyl | **41 (ZH-78)** | (88%) |
| cyclohexylmethyl | **44 (ZH-138)** | (34%) |

### Benzyl (4-((cyclohexylimino)-methyl)pyrimidin-2-yl)carbamate (12)

45% aq. KOH (0.88 g, 15.8 mmol) was added to an ice-cold solution of crude aldehyde **2** (1.4 g, 5.44 mmol) in aq. HCl (4 ml, 15.8 mmol), while the temperature was maintained below 15 °C. To the neutralized mixture, CH₂Cl₂ (5 ml) and K₂CO₃ (0.76 g, 5.44 mmol) were added followed by cyclohexylamine (0.63 g, 6.36 mmol). The reaction mixture was gradually warmed to room temperature and stirring was continued for 2 h. ¹H NMR analysis indicated ~20% aldehyde remained hence additional cyclohexylamine (0.06 g, 0.63 mmol) was added, and stirring was continued overnight. The reaction mixture was diluted with CH₂Cl₂, washed with water, dried (MgSO₄), filtered and concentrated. The crude material obtained was used in the next step without purification (1.59 g, 85%). ¹H NMR (CDCl₃, 400 MHz) δ 1.24-1.42 (4 H, m), 1.51-1.60 (2 H, m), 1.67-1.85 (4 H, m), 3.28-3.34 (1 H, m), 5.25 (2 H, s, CH₂Ph), 7.34-7.42 (5 H, m), 7.54 (1 H, d, *J* = 4.8 Hz), 8.25 (1 H, s, NH), 8.56 (1 H, d, *J* = 5.2 Hz); ¹³C NMR (CDCl₃, 100 MHz) δ 24.6, 25.6, 34.0, 67.6, 69.8, 112.4, 128.6, 128.7, 128.8, 135.7, 151.6, 157.8, 158.2, 158.9, 163.2; HRMS (ESI⁺) calcd for C₁₉H₂₃N₄O₂ (M+H) 339.1821. Found 339.1817.

### Benzyl (4-(1-cyclohexyl-4-(4-fluorophenyl)-1H-imidazol-5-yl)pyrimidin-2-yl)carbamate (13)

A mixture of α-(*p*-toluenesulfonyl)-4-fluorobenzylisonitrile (1.42 g, 4.90 mmol), imine **12** (1.5 g, 4.43 mmol) and 20% aq. K₂CO₃ (1.35 g, 9.77 mmol) in CH₂Cl₂ at 20 °C was stirred overnight. At this point, additional imine (0.14 g, 0.49 mmol) and K₂CO₃ (0.34 g, 1.04 mmol) were added and the reaction mixture was stirred at 30 °C for a second overnight. The reaction mixture was diluted with CH₂Cl₂, and washed with water, dried (MgSO₄), filtered and concentrated. Flash chromatography of the residue (EtOAc/pet. spirits 6:4) afforded **13** as a white solid (1.62 g, 77%). ¹H NMR (CDCl₃, 400 MHz) δ 1.20-1.28 (2 H, m), 1.38-1.47 (2 H, m), 1.55-1.65 (2 H, m), 1.71-1.85 (2 H, m), 2.10-2.13 (2 H, m), 5.04-5.11 (1 H, m), 5.26 (2 H, s), 6.76 (1 H, d, *J* = 5.2 Hz), 6.99-7.04 (2 H, m), 7.34-7.45 (7 H, m), 7.78 (1 H, s), 8.06 (1 H, br s, NH), 8.33 (1 H, d, *J* = 5.2 Hz); ¹³C NMR (CDCl₃, 100 MHz) δ 25.6, 34.7, 55.5, 67.6, 115.6, 115.8, 116.7, 124.1, 128.7, 128.8, 130.4, 130.56, 130.8, 133.0, 135.8, 136.8, 143.8, 151.2, 157.5, 158.2, 159.5, 161.4, 163.9; HRMS (ESI⁺) calcd for C₂₇H₂₇FN₅O₂ (M+H) 472.2149. Found 472.2150

### 4-(1-Cyclohexyl-4-(4-fluorophenyl)-1H-imidazol-5-yl)pyrimidin-2-amine hydrochloride (14; PF670462)

A mixture of **13** (0.30 g, 0.64 mmol) and 20 wt% Pd(OH)₂/C (0.13 g) in THF (5 ml) and methanol (0.5 ml) was hydrogenated at 200 psi for 1 h. The mixture was filtered through Celite and concentrated under reduced pressure to afford the crude product, which was stirred in 4 M aq HCl (2 ml) and methanol (2 ml) for 1 h to give the HCl salt. The solvent was evaporated and recrystallisation from a combination of methanol, acetone and pet. spirits afforded **14** as a white solid (0.19 g, 83%). ¹H NMR (MeOH-d₄, 400 MHz) δ 1.28-1.40 (2 H, m), 1.48-1.58 (2 H, m), 1.78-1.88 (2 H, m), 1.94-1.98 (2 H, m), 2.27-2.30 (2 H, m), 4.95-5.02 (1 H, m), 6.67 (1 H, d, *J* = 6.4 Hz), 7.29-7.33 (2 H, m), 7.59-7.62 (2 H, m), 8.26 (1 H, d, *J* = 6 Hz), 9.48 (1 H, s); ¹³C NMR (MeOH-d₄, 100 MHz) δ 24.6, 25.1, 33.4, 59.1, 110.4, 116.3, 116.6, 121.8, 121.9, 124.6, 136.1, 136.2, 148.1, 156.3, 162.7, 163.1, 165.6; HRMS (ESI⁺) calcd for C₁₉H₂₁FN₅ (M+H) 338.1781. Found 339.1778.

### Benzyl (4-((adamantan-1-ylimino)-methyl)pyrimidin-2-yl)carbamate (15)

45% aq. KOH (0.448 g, 7.98 mmol) was added to an ice cold solution of crude aldehyde **2** (0.250 g, 0.972 mmol) in aq. HCl (2 ml, 7.94 mmol), while the temperature was maintained below 15 °C. To the neutralized mixture, CH₂Cl₂ (5 ml) and K₂CO₃ (0.158 g, 1.14 mmol) were added followed by 1-adamantylamine (0.175 g, 1.17 mmol). The reaction mixture was gradually warmed to room temperature and stirring was continued for 2 h. ¹H NMR analysis indicated ~20% aldehyde remaining hence additional 1-adamantylamine (0.030 g, 0.19 mmol) was added, and stirring was continued overnight. The reaction mixture was diluted with CH₂Cl₂, washed with water, dried (MgSO₄), filtered and concentrated. The crude material was used in the next step without purification (0.36 g, 94%). ¹H NMR (CDCl₃, 400 MHz) δ 1.57-1.79 (12 H, m), 2.01-2.08 (3 H, m), 5.26 (2 H, s), 7.34-7.43 (5 H, m), 7.61 (1 H, d, *J* = 4.8 Hz), 8.18 (1 H, br s, NH), 8.57 (1 H, d, *J* = 4.8 Hz); ¹³C NMR (CDCl₃, 100 MHz) δ 29.4, 36.4, 42.7, 59.2, 67.4, 112.1, 128.4, 128.5, 128.6, 135.6, 151.4, 157.5, 158.6, 163.7; HRMS (ESI⁺) calcd for C₂₃H₂₇N₄O₂ (M+H) 391.4867. Found 391.4872.

### Benzyl (4-(adamantan-1-yl-4-(4-fluorophenyl)-1H-imidazol-5-yl)pyrimidin-2-yl)carbamate (16)

α-(*p*-Toluenesulfonyl)-4-fluorobenzylisonitrile (0.300 g, 1.04 mmol) and 20% aq. K₂CO₃ (0.286 g, 2.07 mmol) were added to a solution of imine **15** (0.363 g, 0.936 mmol) in CH₂Cl₂ at 20 °C, and the mixture was stirred overnight. At this point, additional imine (0.036 g, 0.094 mmol) and K₂CO₃ (0.143 g, 1.03 mmol) were added and the reaction mixture was stirred at 30 °C for a second overnight. The reaction mixture was diluted with CH₂Cl₂, and washed with water, dried (MgSO₄), filtered and concentrated. Flash chromatography of the residue (MeOH/CHCl₃/Et₃N 39:60:1), followed by recrystallisation using CH₃CN/ H₂O afforded **16** as a white solid (0.070 g, 15%). ¹H NMR (CDCl₃, 400 MHz) δ 1.57-1.70 (7 H, m), 2.10-2.17 (3 H, m), 2.18-2.23 (5 H, m), 5.26 (2 H, s), 6.79 (1 H, d, *J* = 4.8 Hz), 6.88-6.92 (2 H, m), 7.20-7.26 (2 H, m), 7.35-7.46 (5 H, m), 7.73 (1 H, br s, NH), 7.80 (1 H, s), 8.50 (1 H, d, *J* = 5.2 Hz); ¹³C NMR (CDCl₃, 100 MHz) δ 29.7, 35.8, 43.1, 59.4, 67.6, 115.1, 115.3, 119.5, 128.6, 128.7, 128.8, 129.6, 129.7, 135.5, 138.8, 151.1, 157.3, 159.0, 163.0; HRMS (ESI⁺) calcd for C₃₁H₃₁FN₅O₂ (M+H) 524.2462. Found 524.2457.

### 4-(Adamantan-1-yl -4-(4-fluorophenyl)-1H-imidazol-5-yl)pyrimidin-2-amine (17; SS8-122)

A mixture of **16** (0.06 g, 0.15 mmol) and 20 wt% Pd(OH)₂/C (0.20 g) in THF (5 ml) and methanol (0.5 ml) was treated with hydrogen at 200 psi for 1 h. The mixture was filtered through Celite and concentrated under reduced pressure to afford the crude product. Flash chromatography of the residue (MeOH/CHCl₃/Et₃N 5:94:1) afforded **17** as a white solid (0.038 g, 86%). ¹H NMR (CDCl₃, 400 MHz) δ 1.60-1.72 (7 H, m), 2.12-2.24 (8 H, m), 5.16 (2 H, br s), 6.52 (1 H, d, *J* = 5.2 Hz), 6.89-6.94 (2 H, m), 7.26-7.33 (2 H, m), 7.77 (1 H, s), 8.24 (1 H, d, *J* = 4.8 Hz); ¹³C NMR (CDCl₃, 150 MHz) δ 29.9, 36.0, 43.4, 59.5, 115.1, 115.28, 115.3, 125.7, 129.4, 129.5, 130.6, 135.2, 141.5, 159.1, 161.1, 162.9, 163.0, 163.1; HRMS (ESI⁺) calcd for C₂₃H₂₅FN₅ (M+H) 390.2094. Found 391.2102.

### Benzyl (4-((cycloheptylimino)-methyl)pyrimidin-2-yl)carbamate (18)

45% aq. KOH (0.448 g, 7.98 mmol) was added to an ice cold solution of crude aldehyde **2** (0.250 g, 0.972 mmol) in aq. HCl (2 ml, 7.94 mmol), while the temperature was maintained below 15 °C. To the resulting neutralized solution, CH₂Cl₂ (5 ml) and K₂CO₃ (0.158 g, 1.14 mmol) were added followed by cycloheptylamine (0.132 g, 1.16 mmol). The reaction mixture was gradually warmed to room temperature and stirring was continued for 2 h. ¹H NMR analysis indicated ~20% aldehyde remaining hence additional cycloheptylamine (0.015 g, 0.19 mmol) was added, and stirring was continued further for overnight. The reaction mixture was diluted with CH₂Cl₂, washed with water, dried (MgSO₄), filtered and concentrated. The crude thus obtained was used in the next step without purification (0.31 g, 91%). ¹H NMR (CDCl₃, 400 MHz) δ 1.50-1.80 (12 H, m), 3.45-3.48 (1 H, m), 5.25 (2 H, s, CH₂Ph), 7.33-7.41 (5 H, m), 7.54 (1 H, d, *J =* 5.2 Hz), 8.20 (1 H, s, NH), 8.54 (1 H, d, *J* = 5.2 Hz); ¹³C NMR (CDCl₃, 100 MHz) δ 24.7, 28.6, 36.0, 67.5, 72.3, 112.4, 128.6, 128.7, 128.8, 135.7, 151.7, 157.4, 157.9, 158.8, 163.3; HRMS (ESI⁺) calcd for C₂₀H₂₅N₄O₂ (M+H) 353.1978. Found 353.1981.

### Benzyl (4-(1-cycloheptyl-4-(4-fluorophenyl)-1H-imidazol-5-yl)pyrimidin-2-yl)carbamate (19)

α-(*p*-Toluenesulfonyl)-4-fluorobenzylisonitrile (0.250 g, 0.861 mmol) and 20% aq. K₂CO₃ (0.238 g, 1.73 mmol) were added to a solution of imine **18** (0.274 g, 0.778 mmol) in CH₂Cl₂ at 20 °C whilst stirring was continued overnight. At this point, additional imine (0.027 g, 0.078 mmol) and K₂CO₃ (0.119 g, 0.865 mmol) were added and the reaction mixture was stirred at 30 °C overnight. The reaction mixture was diluted with CH₂Cl₂, and washed with water, dried (MgSO₄), filtered and concentrated. Flash chromatography of the residue (MeOH/CHCl₃/Et₃N 6:93:1) afforded **19** as a white solid (0.24 g, 70%). ¹H NMR (CDCl₃, 400 MHz) δ 1.52-1.87 (10 H, m), 2.14-2.18 (2 H, m), 5.24-5.30 (1 H, m), 5.26 (2 H, s), 6.74 (1 H, d, *J* = 5.2 Hz), 6.99-7.03 (2 H, m), 7.33-7.44 (7 H, m), 7.77 (1 H, s), 8.31 (1 H, d, *J =* 5.2 Hz), 8.38 (1 H, s, NH); ¹³C NMR (CDCl₃, 100 MHz) δ 24.7, 27.5, 36.7, 57.5, 67.7, 115.5, 115.7, 116.7, 124.0, 128.7, 128.77, 128.80, 130.4, 130.5, 130.8, 135.8, 137.1, 143.6, 151.3, 157.6, 158.1, 159.6, 161.4, 163.9; HRMS (ESI⁺) calcd for C₂₈H₂₈FN₅O₂ (M+H) 485.2227. Found 486.2235.

### 4-(1-Cycloheptyl-4-(4-fluorophenyl)-1H-imidazol-5-yl)pyrimidin-2-amine (20; SS8-138)

A mixture of **19** (0.235 g, 0.485 mmol) and 20 wt% Pd(OH)₂/C (0.25 g) in THF (5 ml) and methanol (0.5 ml) was treated with hydrogen at 200 psi for 1 h. The mixture was filtered through Celite and concentrated under reduced pressure to afford the crude product. Flash chromatography of the residue (MeOH/CHCl₃/Et₃N 5:94:1) afforded **20** as a white solid (0.155 g, 92%). ¹H NMR (CDCl₃, 400 MHz) δ 1.44-1.90 (10 H, m), 2.16-2.21 (2 H, m), 4.64-4.71 (1 H, m), 5.06 (2 H, br s), 6.50 (1 H, d, *J =* 5.2 Hz), 6.97-7.01 (2 H, m), 7.43-7.46 (2 H, m), 7.73 (1 H, s), 8.19 (1 H, d, *J* = 5.2 Hz); ¹³C NMR (CDCl₃, 150 MHz) δ 24.8, 27.4, 36.5, 57.5, 113.0, 115.1, 115.3, 124.5, 128.8, 129.9, 130.6, 136.0, 141.4, 158.4, 159.3, 161.0, 163.0, 163.5; HRMS (ESI⁺) calcd for C₂₀H₂₃FN₅ (M+H) 352.1937. Found 352.1934.

### Benzyl (4-((exo-2-aminonorbornane)-methyl)pyrimidin-2-yl)carbamate (21)

45% aq. KOH (0.448 g, 7.98 mmol) was added to an ice-cooled solution of crude aldehyde **2** (0.250 g, 0.972 mmol) in aq. HCl (2 ml, 7.94 mmol), while the temperature was maintained below 15 °C. To the neustralized solution, CH₂Cl₂ (5 ml) and K₂CO₃ (0.158 g, 1.14 mmol) were added followed by exo-2-aminonorbornane (0.129 g, 1.16 mmol). The reaction mixture was gradually warmed to room temperature and stirring was continued for 2 h. ¹H NMR analysis indicated complete consumption of aldehyde. The reaction mixture was diluted with CH₂Cl₂, washed with water, dried (MgSO₄), filtered and concentrated. The crude thus obtained was used in the next step without purification (0.24 g, 71%). ¹H NMR (CDCl₃, 400 MHz) 1.20-1.27 (3 H, m), 1.45-1.64 (4 H, m), 1.85-1.88 (1 H, m), 2.12-2.19 (1 H, m), 2.29-2.38 (1 H, m), 3.38-3.43 (1 H, m), 5.25 (2 H, m), 7.35-7.41 (5 H, m), 7.54 (1 H, d, *J* = 5.2 Hz), 8.16 (1 H, s, NH), 8.55 (1 H, d, *J* = 5.2 Hz); ¹³C NMR (CDCl₃, 100 MHz) δ 26.8, 29.2, 35.7, 36.4, 39.6, 44.4, 67.5, 74.0, 112.2, 128.6, 128.66, 128.73, 135.7, 151.7, 157.2, 157.8, 158.7, 163.4; HRMS (ESI⁺) calcd for C₂₀H₂₃N₄O₂ (M+H) 351.1821. Found 351.1815.

### Benzyl (4-(1-(exo-2-aminonorbornane)-4-(4-fluorophenyl)-1H-imidazol-5-yl)pyrimidin-2-yl)carbamate (22)

α-(*p*-Toluenesulfonyl)-4-fluorobenzylisonitrile (0.200 g, 0.691 mmol) and 20% aq. K₂CO₃ (0.191 g, 1.38 mmol) were added to a solution of imine **21** (0.218 g, 0.622 mmol) in CH₂Cl₂ (5 ml) at 20 °C whilst stirring was continued overnight. At this point, additional imine (0.024 g, 0.062 mmol) and K₂CO₃ (0.047 g, 0.345 mmol) were added and the reaction mixture was stirred at 30 °C overnight. The reaction mixture was diluted with CH₂Cl₂, and washed with water, dried (MgSO₄), filtered and concentrated. Flash chromatography of the residue (MeOH/CHCl₃/Et₃N 6:93:1), followed by recrystallisation using CH₃OH/ CH₂Cl₂ afforded **22** as a white solid (0.071 g, 23%). ¹H NMR (CDCl₃, 400 MHz) δ 1.18-1.23 (1 H, m), 1.35-1.43 (2 H, m), 1.51-1.68 (4 H, m), 1.76-1.81 (1 H, m), 2.35-2.40 (1 H, m), 2.61-2.63 (1 H, m), 5.12-5.15 (1 H, m), 5.20 (2 H, m), 6.75 (1 H, d, *J =* 5.2 Hz), 6.99-7.03 (2 H, m), 7.34-7.45 (7 H, m), 7.81 (1 H, s), 8.04 (1 H, s, NH), 8.33 (1 H, d, *J* = 5.2 Hz); ¹³C NMR (CDCl₃, 100 MHz) δ 27.5, 28.4, 36.7, 36.8, 41.0, 42.3, 59.8, 67.6, 115.6, 115.8, 116.7, 124.4, 128.7, 128.8, 130.31, 130.4, 130.7, 135.8, 136.5, 144.2, 151.3, 157.4, 158.2, 159.7, 161.4; HRMS (ESI⁺) calcd for C₂₈H₂₇FN₅O₂ (M+H) 484.2149. Found 484.2138.

### 4-(1-(1-(Exo-2-aminonorbornane)-4-(4-fluorophenyl)-1H-imidazol-5-yl)pyrimidin-2-amine (23; SS8-154)

A mixture of **22** (0.070 g, 0.147 mmol) and 20 wt% Pd(OH)₂/C (0.020 g) in THF (5 ml) and methanol (0.5 ml) was treated with hydrogen at 200 psi for 1 h. The mixture was filtered through Celite and concentrated under reduced pressure to afford the crude product. Flash chromatography of the residue (MeOH/CHCl₃/Et₃N 5:94:1) afforded **23** as a white solid (0.030 g, 60%). ¹H NMR (CDCl₃, 400 MHz) δ 1.18-1.37 (3 H, m), 1.53-1.82 (5 H, m), 2.39-2.44 (1 H, m), 2.56-2.61 (1 H, m), 4.55-4.58 (1 H, m), 5.10 (2 H, br s), 6.51 (1 H, d, *J* = 5.2 Hz), 6.96-7.01 (2 H, m), 7.43-7.46 (2 H, m), 7.76 (1 H, s), 8.19 (1 H, d, *J* = 5.2 Hz); ¹³C NMR (CDCl₃, 100 MHz) δ 27.6, 28.3, 36.3, 36.5, 40.6, 42.9, 59.7, 113.3, 115.3, 115.5, 129.9, 130.0, 135.5, 136.5, 142.2, 158.6, 159.7, 161.2, 163.2, 163.7; HRMS (ESI⁺) calcd for C₂ₒH₂₁FN₅ (M+H) 350.1781. Found 350.1785.

### Benzyl-(4-((methylimino)methyl)pyrimidin-2-yl)carbamate (24)

45% aq. KOH (0.449 g, 8.00 mmol) was added to an ice-cold solution of crude aldehyde **2** (0.137 g, 0.531 mmol) in aq. HCl (2 ml, 8.00 mmol), while the temperature was maintained below 15 °C. To the neutralized solution, CH₂Cl₂ (5 ml) and K₂CO₃ (0.069 g, 0.502 mmol) were added followed by methylamine solution in THF (530 µL, 1.06 mmol, 2.0 M). The reaction mixture was gradually warmed to room temperature and stirring was continued for 18 h. ¹H NMR analysis of the reaction mixture showed incomplete consumption of the aldehyde thus additional methylamine was added (265 µL, 0.531 mmol) and the reaction stirred for an additional 2 h. After complete consumption of the aldehyde, the crude material was used in the next step without isolation.

### Benzyl (4-(4-(4-fluorophenyl)-1-methyl-1H-imidazol-5-yl)pyrimidin-2-yl)carbamate (25)

A mixture of α-(*p*-toluenesulfonyl)-4-fluorobenzylisonitrile (0.184 g, 0.637 mmol), imine (0.144 g, 0.531 mmol) and aq. 20% K₂CO₃ (0.440 mL, 0.637 mmol) in CH₂Cl₂ (10 ml) was stirred at r.t. for 72 h. The reaction mixture was diluted with CH₂Cl₂, and washed with water, dried (MgSO₄), filtered and concentrated. Flash chromatography of the residue (EtOAc/pet. spirits 3:2) afforded the methyl carbamate as a colourless solid (79.2 mg, 37%), m.p. 190-198°C.¹H-NMR (500 MHz; CDCl₃): δ 4.03 (3 H, s), 5.24 (2 H, s), 6.67 (1 H, dt, *J* = 5.3, 0.7 Hz), 7.07-7.03 (2 H, m), 7.46-7.31 (7 H, m), 7.59 (1 H, s), 8.16-8.14 (1 H, m), 9.54 (1 H, s); ¹³C-NMR (126 MHz; CDCl₃): δ 35.1, 67.6, 115.2, 115.6, 115.8, 125.1, 128.7, 128.7, 128.8, 130.5 (d, *J*_{C-F} = 8.1 Hz), 130.8 (d, *c* = 3.3 Hz), 135.6, 141.1, 144.5, 151.6, 151.6, 157.4, 157.7, 158.8, 162.8 (d, *J*_{C-F} = 248 Hz). HRMS (ESI⁺) calcd for C₂₂H₁₉FN₅O₂ (M+H) 404.1523. Found 404.1518.

### 4-(4-(4-Fluorophenyl)-1-methyl-1H-imidazol-5-yl)pyrimidin-2-amine (26; ZH-142)

20% wt Pd(OH)₂/C (6.50 mg) was added to a solution of the methyl carbamate (47.0 mg, 0.117 mmol) in THF/MeOH 5:3 (8 mL). The resulting mixture was stirred under an atmosphere of hydrogen at 200 psi for 2 h. The mixture was filtered through a short pad of celite, concentrated and purified by flash chromatography (CHCl₃/MeOH/NEt₃ 97:2:1) to afford a colourless solid (31.4 mg, 72%). ¹H-NMR (500 MHz; CD₃OD): δ 3.9 (3 H, s), 6.41 (1 H, d, *J* = 5.2 Hz), 7.10-7.06 (2 H, m), 7.45-7.43 (2 H, m), 7.79 (1 H, s), 8.10 (1 H, d, *J* = 5.2 Hz); ¹³C-NMR (126 MHz; CD₃OD): δ 34.4, 112.1, 116.2, 116.4, 127.3, 128.6, 128.8, 129.0, 131.4 (d, *J*_{C-F} = 8.2 Hz), 131.6 (d, *J*_{C-F} = 3.4 Hz), 141.4, 142.6, 159.2, 159.9, 163.9 (d, *J*_{C-F} = 247 Hz), 164.8. HRMS (ESI⁺) calcd for C₁₄H₁₃FN₅ (M+H) 270.1155. Found 270.1149.

### Benzyl-(4-((ethylimino)methyl)pyrimidin-2-yl)carbamate (27)

45% aq. KOH (0.449 g, 8.00 mmol) was added to an ice-cold solution of crude aldehyde **2** (0.148 g, 0.574 mmol) in aq. HCl (2 ml, 8.00 mmol), while the temperature was maintained below 15 °C. To the neutralized solution, CH₂Cl₂ (5 ml) and K₂CO₃ (0.095 g, 0.688 mmol) were added followed by ethylamine solution in THF (575 µL, 1.15 mmol, 2.0 M). The reaction mixture was gradually warmed to room temperature and stirring was continued for 18 h. ¹H NMR analysis of the reaction mixture showed complete consumption of the aldehyde. The crude material was used in the next step without isolation.

### Benzyl (4-(1-(ethyl)-4-(4-fluorophenyl)-1H-imidazol-5-yl)pyrimidin-2-yl)carbamate (28)

A mixture of α-(*p*-toluenesulfonyl)-4-fluorobenzylisonitrile (0.199 g, 0.689 mmol), imine (0.163 g, 0.574 mmol) and aq. 20% K₂CO₃ (0.475 mL, 0.689 mmol) in CH₂Cl₂ (10 ml) was stirred at r.t. for 20 h. The reaction mixture was diluted with CH₂Cl₂, and washed with water, dried (MgSO₄), filtered and concentrated. Flash chromatography of the residue (EtOAc/pet. spirits 3:2) afforded the ethyl carbamate as an off-white solid (79.4 mg, 33%), m.p. 164-171°C. ¹H-NMR (500 MHz; CDCl₃): δ 1.28 (3 H, t, *J* = 7.2 Hz, 4.62 (2 H, q, *J* = 7.2 Hz), 5.23 (2 H, s), 6.62 (1 H, d, *J* = 5.4 Hz), 7.04 (2 H, t, *J* = 8.8 Hz), 7.45-7.29 (7 H, m), 7.64 (1 H, s), 8.12 (1 H, d, *J* = 5.4 Hz), 9.87 (1 H, s); ¹³C-NMR (101 MHz; CDCl₃): δ 16.9, 42.3, 67.6, 115.2, 115.5, 115.8, 124.1, 128.7, 128.4, 128.8, 130.6 (d, *J*_{C-F} = 8.0 Hz), 130.8 (d, *J*_{C-F} = 3.2 Hz), 135.6, 139.9, 144.7, 151.6, 157.6, 157.7, 159.0, 162.7 (d, *J*_{C-F} = 248 Hz). HRMS (ESI⁺) calcd for C₂₃H₂₁FN₅O₂ (M+H) 418.1679. Found 418.1678.

### 4-(1-(Ethyl)-4-(4-fluorophenyl)-1H-imidazol-5-yl)pyrimidin-2-amine(29; ZH-6)

20% wt Pd(OH)₂/C (37.0 mg) was added to a solution of the ethyl carbamate (79.0 mg, 0.189 mmol) in THF/MeOH 10:1 (5 mL). The resulting mixture was stirred under an atmosphere of hydrogen at 200 psi for 2 h. The mixture was filtered through celite, concentrated and purified by flash chromatography (CHCl₃/MeOH/NEt₃ 97:2:1) to afford a colourless solid (54.0 mg, 84%), m.p. 204-212°C.¹H-NMR (500 MHz; CD₃OD): δ 1.32 (3 H, t, *J* = 7.2 Hz), 4.38 (2 H, q, *J* = 7.2 Hz), 4.86 (3 H, s), 6.40 (1 H, d, *J* = 5.2 Hz), 7.10-7.06 (2 H, m), 7.45-7.42 (2 H, m), 7.87 (1 H, s), 8.11 (1 H, d, *J =* 5.2 Hz); ¹³C-NMR (126 MHz; CD₃OD): δ 16.9, 42.8, 112.2, 116.2, 116.4, 126.5, 131.4 (d, *J*_{C-F} = 8.3 Hz), 131.7 (d, *J*_{C-F} = 3.2 Hz), 140.2, 142.7 (s, 1C), 159.3, 160.1, 163.9 (d, *J*_{C-F} = 247 Hz), 164.9. HRMS (ESI⁺) calcd for C₁₅H₁₅FN₅ (M+H) 284.1311. Found 284.1307.

### Benzyl-(4-((tert-butylimino)methyl)pyrimidin-2-yl)carbamate (30)

45% aq. KOH (0.449 g, 8.00 mmol) was added to an ice-cold solution of crude aldehyde 2 (0.120 g, 0.465 mmol) in aq. HCl (2 ml, 8.00 mmol), while the temperature was maintained below 15 °C. To the neutralized solution, CH₂Cl₂ (5 ml) and K₂CO₃ (0.071 g, 0.511 mmol) were added followed by ethylamine solution in THF (73.0 µL, 0.968 mmol). The reaction mixture was gradually warmed to room temperature and stirring was continued for 18 h. ¹H NMR analysis of the reaction mixture showed complete consumption of the aldehyde. The crude material was used in the next step without isolation.

### Benzyl (4-(1-(tert-butyl)-4-(4-fluorophenyl)-1H-imidazol-5-yl)pyrimidin-2-yl)carbamate (31)

A mixture of α-(*p*-toluenesulfonyl)-4-fluorobenzylisonitrile (0.161 g, 0.558 mmol), imine (0.145 g, 0.465 mmol) and aq. 20% K₂CO₃ (0.385 mL, 0.558 mmol) in CH₂Cl₂ (10 ml) was stirred at r.t. for 60 h. The reaction mixture was diluted with CH₂Cl₂, and washed with water, dried (MgSO₄), filtered and concentrated. Flash chromatography of the residue (EtOAc/pet. spirits 3:2) afforded the *tert-butyl* carbamate as a colourless solid (64.1 mg, 31%). ¹H-NMR (400 MHz; CDCl₃): δ 1.61 (9 H, s), 5.27 (2 H, s), 6.76 (1 H, d, *J =* 5.0 Hz), 6.92 (2 H, t, *J* = 8.7 Hz), 7.44-7.22 (7 H, m), 7.78 (1 H, s), 8.45 (1 H, d, *J* = 5.0 Hz), 8.52 (1 H, s); ¹³C-NMR (101 MHz; CDCl₃): δ 31.2, 58.4, 67.7, 115.2, 115.4, 119.5, 125.3, 128.7, 128.8, 128.8, 129.8 (d, *J*_{C-F} = 8.2Hz), 130.2 (d, *J*_{C-F} = 3.4 Hz), 135.6, 136.3, 142.3, 151.4, 157.7 (s, 1C), 159.0, 162.2 (d, *J*_{C-F} = 248 Hz), 162.8.

### 4-(1-(Tert-butyl)-4-(4-fluorophenyl)-1H-imidazol-5-yl)pyrimidin-2-amine (32; ZH9-190)

20% wt Pd(OH)₂/C (33.0 mg) was added to a solution of the *tert-butyl* carbamate (64.0 mg, 0.143 mmol) in THF/MeOH 10:1 (5 mL). The resulting mixture was stirred under an atmosphere of hydrogen at 200 psi for 2 h. The mixture was filtered through celite, concentrated and purified by flash chromatography (CHCl₃/MeOH/NEt₃ 97:2:1) to afford a colourless solid (30.8 mg, 69%), m.p. 189-197°C. ¹H-NMR (500 MHz; CD₃OD): δ 1.61 (9 H, s), 6.52 (1 H, d, *J* = 5.0 Hz), 7.00-6.97 (2 H, m), 7.32-7.29 (2 H, m), 7.93 (1 H, s), 8.21 (1 H, d, *J* = 5.0 Hz); ¹³C-NMR (126 MHz; CD₃OD): δ 31.3, 59.8, 115.3, 115.9, 116.1, 127.8, 130.6 (d, *J*_{C-F} = 8.1 Hz), 131.5 (d, *J*_{C-F} = 3.4 Hz), 137.1, 141.5, 160.1, 163.3, 163.5 (d, *J*_{C-F} = 246 Hz), 164.9. HRMS (ESI⁺) calcd for C₁₇H₁₉FN₅ (M+H) 312.1624. Found 312.1619.

### Benzyl-(4-((cyclopropylimino)methyl)pyrimidin-2-yl)carbamate (33)

45% aq. KOH (0.449 g, 8.00 mmol) was added to an ice-cold solution of crude aldehyde 2 (0.140 g, 0.544 mmol) in aq. HCl (2 ml, 8.00 mmol), while the temperature was maintained below 15 °C. To the neutralized solution, CH₂Cl₂ (5 ml) and K₂CO₃ (0.090 g, 0.653 mmol) were added followed by cyclopropylamine (75.0 µL, 1.09 mmol). The reaction mixture was gradually warmed to room temperature and stirring was continued for 18 h. ¹H NMR analysis of the reaction mixture showed complete consumption of the aldehyde. The crude material was used in the next step without isolation.

### Benzyl (4-(1-cyclopropyl-4-(4-fluorophenyl)-1H-imidazol-5-yl)pyrimidin-2-yl)carbamate (34)

A mixture of α-(*p*-toluenesulfonyl)-4-fluorobenzylisonitrile (0.189 g, 0.653 mmol), imine (0.161 g, 0.544 mmol) and aq. 20% K₂CO₃ (0.450 mL, 0.653 mmol) in CH₂Cl₂ (10 ml) was stirred at 30°C for 48 h. The reaction mixture was diluted with CH₂Cl₂, and washed with water, dried (MgSO₄), filtered and concentrated. Flash chromatography of the residue (EtOAc/pet. spirits 3:2) afforded the cyclopropyl carbamate as a colourless solid (50.7 mg, 22%), m.p. 178-188°C. ¹H-NMR (500 MHz; CDCl₃): δ 0.95-0.76 (4 H, m), 4.05 (1 H, dt, *J* = 7.4, 3.6 Hz), 5.23 (2 H, s), 6.73 (1 H, d, *J =* 5.3 Hz), 7.02 (2 H, t, *J =* 8.8 Hz), 7.46-7.31 (7 H, m), 7.66 (1 H, s), 8.27 (1 H, d, *J* = 5.3 Hz), 9.12 (1 H, s); ¹³C-NMR (126 MHz; CDCl₃): δ 7.4, 29.4, 67.6, 115.6, 115.7, 126.6, 128.7, 128.8, 130.3 (d, *J*_{C-F} = 8.1 Hz), 130.4 (d, *J*_{C-F} = 3.2 Hz), 135.7, 139.7, 143.4, 151.5, 157.5, 158.0, 159.0, 162.6 (d, *J* = 247 Hz). HRMS (ESI⁺) calcd for C₂₄H₂₁FN₅O₂ (M+H) 430.1679. Found 430.1676.

### 4-(1-Cyclopropyl-4-(4-fluorophenyl)-1H-imidazol-5-yl)pyrimidin-2-amine (35; ZH-58)

20% wt Pd(OH)₂/C (25.0 mg) was added to a solution of the cyclopropyl carbamate (50.7 mg, 0.118 mmol) in THF/MeOH 5:2 (7 mL). The resulting mixture was stirred under an atmosphere of hydrogen at 200 psi for 2.5 h. The mixture was filtered through celite, concentrated and purified by flash chromatography (CHCl₃/MeOH/NEt₃ 97:2:1) to afford a colourless solid (31.9 mg, 99%).¹H-NMR (500 MHz; CDCl₃): δ 0.95-0.82 (4 H, m), 3.63 (1 H, tt, *J* = 7.3, 3.8 Hz), 5.21 (2 H, s), 6.56 (1 H, d, *J* = 5.1 Hz), 7.01-6.97 (2 H, m), 7.49-7.46 (2 H, m), 7.63 (1 H, s), 8.21 (1 H, d, *J* = 5.1 Hz); ¹³C-NMR (126 MHz; CDCl₃): δ 7.2, 28.5, 112.4, 115.3, 115.5, 127.1, 129.8 (d, *J*_{C-F} = 8.1 Hz), 130.4 (d, *J*_{C-F} = 3.2 Hz), 138.9, 141.7, 158.5, 159.1, 162.5 (d, *J*_{C-F} = 247 Hz), 163.19. HRMS (ESI⁺) calcd for C₁₆H₁₅FN₅ (M+H) 296.1311. Found 296.1306.

### Benzyl-(4-((cyclobutylimino)methyl)pyrimidin-2-yl)carbamate (36)

45% aq. KOH (0.449 g, 8.00 mmol) was added to an ice-cold solution of crude aldehyde **2** (0.146 g, 0.570 mmol) in aq. HCl (2 ml, 8.00 mmol), while the temperature was maintained below 15 °C. To the neutralized solution, CH₂Cl₂ (5 ml) and K₂CO₃ (0.095 g, 0.684 mmol) were added followed by cyclobutylamine (97.0 µL, 1.14 mmol). The reaction mixture was gradually warmed to room temperature and stirring was continued for 18 h. ¹H NMR analysis of the reaction mixture showed complete consumption of the aldehyde. The crude material was used in the next step without isolation.

### Benzyl (4-(1-cyclobutyl-4-(4-fluorophenyl)-1H-imidazol-5-yl)pyrimidin-2-yl)carbamate (37)

**A** mixture of α-(*p*-toluenesulfonyl)-4-fluorobenzylisonitrile (0.197 g, 0.684 mmol), imine (0.177 g, 0.570 mmol) and aq. 20% K₂CO₃ (0.450 mL, 0.684 mmol) in CH₂Cl₂ (10 ml) was stirred at 30°C for 18 h. The reaction mixture was diluted with CH₂Cl₂, and washed with water, dried (MgSO₄), filtered and concentrated. Flash chromatography of the residue (EtOAc/pet. spirits 2:3) afforded the cyclobutyl carbamate as a colourless solid (98.5 mg, 39%), m.p. 191-204°C. ¹H-NMR (500 MHz; CDCl₃): δ 1.87-1.82 (2 H, m), 2.28-2.23 (2 H, m), 2.45-2.40 (2 H, m), 5.27 (2 H, s), 5.56-5.52 (1 H, m), 6.68 (1 H, d, *J* = 5.3 Hz), 7.04-7.01 (2 H, m), 7.44-7.32 (7 H, m), 7.81 (1 H, s), 8.23 (1 H, d, *J =* 5.3 Hz), 9.01 (1 H, s); ¹³C-NMR (126 MHz; CDCl₃): δ 15.2, 31.1, 50.9, 67.6, 115.6, 115.7, 116.0, 124.3, 128.7, 128.8, 130.4 (d, *J*_{C-F} = 8.1 Hz), 130.7 (d, *J*_{C-F} = 3.2 Hz), 135.7, 137.4, 144.1, 151.5, 157.5, 157.9, 159.1, 162.7 (d, *J*_{C-F} = 247.3 Hz). HRMS (ESI⁺) calcd for C₂₅H₂₃FN₅O₂ (M+H) 444.1836. Found 444.1834.

### 4-(1-Cyclobutyl-4-(4-fluorophenyl)-1H-imidazol-5-yl)pyrimidin-2-amine (38; ZH-62)

20% wt Pd(OH)₂/C (56.0 mg) was added to a solution of the cyclobutyl carbamate (96.0 mg, 0.216 mmol) in THF/MeOH 5:2 (7 mL). The resulting mixture was stirred under an atmosphere of hydrogen at 100 psi for 3 h. The mixture was filtered through celite, concentrated and purified by flash chromatography (CHCl₃/MeOH/NEt₃ 97:2:1) to afford a colourless solid (34.2 mg, 51%), m.p. 229-231°C. ¹H-NMR (500 MHz; CD₃OD): δ 1.91-1.83 (2 H, m), 2.45-2.35 (4 H, m), 5.09 (1 H, quintet, *J* = 8.4 Hz), 6.41 (1 H, d, *J* = 5.2 Hz), 7.09-7.05 (2 H, m), 7.44-7.41 (2 H, m), 8.04 (1 H, s), 8.13 (1 H, d, *J* = 5.1 Hz); ¹³C-NMR (126 MHz; CD₃OD): δ 15.8, 31.8, 52.1, 112.6, 116.2, 116.4, 126.7, 131.2 (d, *J*_{C-F} = 8.2 Hz), 131.5 (d, *J*_{C-F} = 3.2 Hz), 137.9, 142.1, 159.4, 160.1, 163.9 (d, *J*_{C-F} = 247 Hz), 164.9. HRMS (ESI⁺) calcd for C₁₇H₁₇FN₅ (M+H) 310.1468. Found 310.1464.

### Benzyl-(4-((oxetan-3-ylimino)methyl)pyrimidin-2-yl)carbamate (39)

45% aq. KOH (0.449 g, 8.00 mmol) was added to an ice-cold solution of crude aldehyde 2 (0.140 g, 0.544 mmol) in aq. HCl (2 ml, 8.00 mmol), while the temperature was maintained below 15 °C. To the neutralized solution, CH₂Cl₂ (5 ml) and K₂CO₃ (0.090 g, 0.653 mmol) were added followed by 3-oxetanamine (77.0 µL, 1.09 mmol). The reaction mixture was gradually warmed to room temperature and stirring was continued for 18 h. ¹H NMR analysis of the reaction mixture showed complete consumption of the aldehyde. The crude material was used in the next step without isolation.

### Benzyl (4-(4-(4-fluorophenyl)-1-(oxetan-3-yl)-1H-imidazol-5-yl)pyrimidin-2-yl)carbamate (40)

A mixture of α-(*p*-toluenesulfonyl)-4-fluorobenzylisonitrile (0.188 g, 0.653 mmol), imine (0.170 g, 0.544 mmol) and aq. 20% K₂CO₃ (0.450 mL, 0.653 mmol) in CH₂Cl₂ (10 ml) was stirred at r.t. for 72 h. The reaction mixture was diluted with CH₂Cl₂, and washed with water, dried (MgSO₄), filtered and concentrated. Flash chromatography of the residue (EtOAc/pet. spirits 3:2) afforded the oxetan-3-yl carbamate as a colourless solid (113 mg, 47%), m.p. 186-196°C. ¹H-NMR (500 MHz; CDCl₃): δ 4.85 (2 H, t, *J* = 6.5 Hz), 5.13 (2 H, t, *J* = 7.2 Hz), 5.28 (2 H, s), 6.31 (1 H, t, *J* = 6.7 Hz), 6.72 (1 H, d, *J =* 5.3 Hz), 7.07 (2 H, t, *J =* 8.6 Hz,), 7.46-7.34 (7 H, m), 8.09 (1 H, s), 8.21 (1 H, d, *J* = 5.3 Hz), 8.70 (1 H, s); ¹³C-NMR (126 MHz; CDCl₃): δ 51.4, 67.8, 77.9, 115.5, 115.8, 116.0, 124.5, 128.8, 128.9, 130.4 (d, *J*_{C-F} = 3.3 Hz), 130.7 (d, *J*_{C-F} = 8.2 Hz), 135.6, 137.4, 144.8, 151.4, 157.4, 158.2, 158.2, 163.0 (d, *J*_{C-F} = 248 Hz). HRMS (ESI⁺) calcd for C₂₄H₂₁FN₅O₃ (M+H) 446.1628. Found 446.1626.

### 4-(4-(4-Fluorophenyl)-1-(oxetan-3-yl)-1H-imidazol-5-yl)pyrimidin-2-amine (41; ZH-78)

20% wt Pd(OH)₂/C (65.0 mg) was added to a solution of the oxetan-3-yl carbamate (102 mg, 0.229 mmol) in THF/MeOH 1:1 (10 mL). The resulting mixture was stirred under an atmosphere of hydrogen at 200 psi for 4 h. The mixture was filtered through celite, concentrated and purified by flash chromatography (CHCl₃/MeOH/NEt₃ 97:2:1) to afford a pale yellow solid (62.7 mg, 88%). ¹H-NMR (500 MHz; CD₃OD): δ 4.93 (2 H, t, *J* = 6.7 Hz), 5.05 (2 H, t, *J =* 7.3 Hz), 5.73 (1 H, m, *J* = 6.8 Hz), 6.37-6.36 (1 H, m), 7.12-7.08 (2 H, m), 7.46-7.43 (2 H, m), 8.06 (1 H, d, *J* = 5.2 Hz), 8.27 (1 H, s); ¹³C-NMR (126 MHz; CD₃OD): δ 53.0, 54.8, 78.2, 111.7, 116.3, 116.5, 126.9, 131.4 (d, *J*_{C-F} = 3.3 Hz), 131.6 (d, *J*_{C-F} = 8.3 Hz), 138.2, 143.2, 159.2, 159.3, 164.1 (d, *J*_{C-F} = 247 Hz), 164.7. HRMS (ESI⁺) calcd for C₁₆H₁₅FN₅O₃ (M+H) 312.1261. Found 312.1256.

### Benzyl-(4-((cyclohexylmethylimino)methyl)pyrimidin-2-yl)carbamate (42)

45% aq. KOH (0.449 g, 8.00 mmol) was added to an ice-cold solution of crude aldehyde **2** (0.108 g, 0.419 mmol) in aq. HCl (2 ml, 8.00 mmol), while the temperature was maintained below 15 °C. To the neutralized solution, CH₂Cl₂ (5 ml) and K₂CO₃ (0.069 g, 0.502 mmol) were added followed by cyclohexylmethylamine (110 µL, 0.838 mmol). The reaction mixture was gradually warmed to room temperature and stirring was continued for 18 h. ¹H NMR analysis of the reaction mixture showed complete consumption of the aldehyde. The crude material was used in the next step without isolation.

### Benzyl (4-(1-(cyclohexylmethyl)-4-(4-fluorophenyl)-1H-imidazol-5-yl)pyrimidin-2-yl)carbamate (43)

A mixture of α-(*p*-toluenesulfonyl)-4-fluorobenzylisonitrile (0.145 g, 0.503 mmol), imine (0.153 g, 0.419 mmol) and aq. 20% K₂CO₃ (0.350 mL, 0.503 mmol) in CH₂Cl₂ (10 ml) was stirred at r.t. for 72 h. The reaction mixture was diluted with CH₂Cl₂, and washed with water, dried (MgSO₄), filtered and concentrated. Flash chromatography of the residue (EtOAc/pet. spirits 3:7) afforded the cyclohexylmethyl carbamate as a colourless solid (102 mg, 50%), m.p. 174-181°C. ¹H-NMR (500 MHz; CDCl₃): δ 1.64-0.83 (11 H, m), 4.46 (2 H, d, *J* = 7.2 Hz), 5.25 (2 H, s), 6.65 (1 H, d, *J* = 5.3 Hz), 7.04 (2 H, t, *J* = 8.7 Hz), 7.45-7.30 (7 H, m), 7.56 (1 H, s), 8.17 (1 H, d, *J* = 5.4 Hz), 9.57 (1 H, s); ¹³C-NMR (126 MHz; CDCl₃): δ 25.7, 26.3, 30.4, 38.9, 52.9, 67.6, 115.6, 115.7, 124.3, 128.7, 128.8, 128.8, 130.6 (d, *J*_{C-F} = 8.1 Hz), 130.7 (d, *J*_{C-F} = 3.3 Hz), 135.6, 141.2, 144.5, 151.5, 157.6, 157.8, 159.4, 162.6 (d, *J* = 247 Hz). HRMS (ESI⁺) calcd for C₂₈H₂₉FN₅O₂ (M+H) 486.2305. Found 486.2303.

### 4-(1-(Cyclohexylmethyl)-4-(4-fluorophenyl)-1H-imidazol-5-yl)pyrimidin-2-amine (44; ZH-138)

20% wt Pd(OH)₂/C (21.0 mg) was added to a solution of the cyclohexylmethyl carbamate (102 mg, 0.229 mmol) in THF/MeOH 5:2 (7 mL). The resulting mixture was stirred under an atmosphere of hydrogen at 200 psi for 2 h. The mixture was filtered through celite, concentrated and purified by flash chromatography (EtOAc/pet.spirits 9:1) to afford a colourless solid (25.1 mg, 34%), m.p. 180-199°C. ¹H-NMR (500 MHz; CDCl₃): δ 0.88-0.85 (2 H, m), 1.14-1.11 (2 H, m), 1.68-1.53 (7 H, m), 4.09 (2 H, d, *J* = 6.7 Hz), 5.18 (2 H, s), 6.50 (1 H, d, *J* = 5.2 Hz), 7.00 (2 H, t, *J* = 8.8 Hz), 7.48-7.45 (2 H, m), 7.54 (1 H, s), 8.15 (1 H, d, *J* = 5.2 Hz); ¹³C-NMR (126 MHz; CDCl₃): δ 25.7, 26.3, 30.7, 39.0, 52.9, 112.5, 115.4, 115.5, 125.0, 130.1 (d, *J*_{C-F} = 8.1 Hz), 130.7 (d, *J*_{C-F} = 3.2 Hz), 140.0, 142.5, 158.4, 159.3, 162.5 (d, *J*_{C-F} = 247 Hz), 163.1. HRMS (ESI⁺) calcd for C₂₀H₂₃FN₅ (M+H) 352.1937. Found 352.1932.

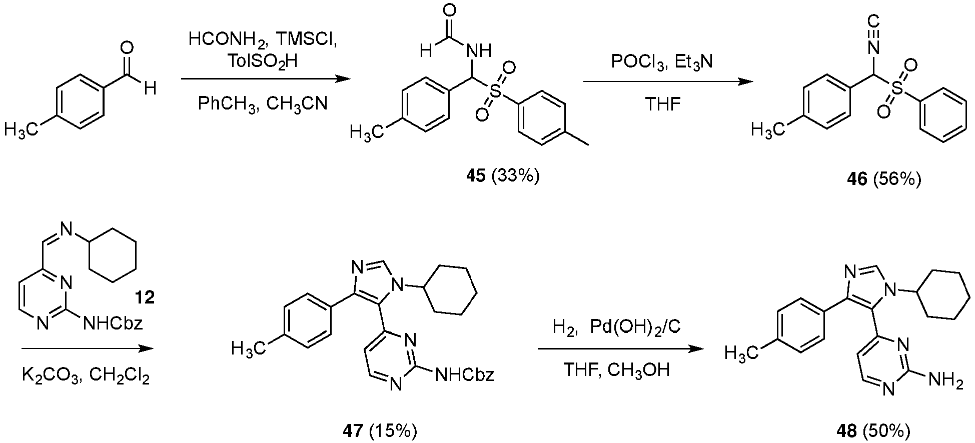

### p-Toluenesulfinic acid

H₂O (10 ml) was added to *p*-toulenesulfinic acid sodium salt (2.00 g, 10.3 mmol), and the resulting mixture was stirred for 30 min until a clear solution was obtained. Diethyl ether (10 ml) was added, followed by conc. HCl (0.7 ml) and stirring was continued for an additional 30 min. The organic layer was separated, diluted with toluene (10 ml) and concentrated on the rotary evaporator until around 90% of the solvent was removed. Heptane (5 ml) was added and the white solid obtained was filtered, washed with heptane and dried under vacuum to give to p-toulenesulfinic acid as a white solid (1.1 g, 69%). ¹H NMR (DMSO-d₆, 400 MHz) δ 2.37 (3 H, s), 7.36 (1 H, d, *J* = 8 Hz), 7.54 (1 H, d, *J* = 8 Hz); ¹³C NMR (DMSO-d₆, 100 MHz) δ 20.9, 124.5, 129.4, 141.3, 146.0.

### N-(p-Tolyl(tosyl)methyl)formamide (45)

Formamide (0.937 g, 20.8 mmol) was added to a solution of p-tolualdehyde (1.00 g, 8.32 mmol) in toluene (2 ml) and acetonitrile (2 ml), followed by addition of TMSCI (0.995 g, 9.15 mmol) and TolSO₂H (1.95 g, 12.5 mmol). The resulting suspension was heated at 50 °C for 5 h. The reaction mixture was cooled to 0 °C, diethyl ether (5 ml) and H₂O (10 ml) were added, the the mixture was stored in the fridge overnight. The solid white product thus obtained was collected by filtration and dried under vacuum to give **45** (0.810 g, 33%). Compound **45** exists as a 5:1 mixture of amide rotamers at room temperature in DMSO-d₆. Spectral data for the major isomer: ¹H NMR (DMSO-d₆, 400 MHz) δ 2.31 (3 H, s), 2.39 (3 H, s), 6.31 (1 H, d, *J =* 10.4 Hz), 7.21 (2 H, d, *J =* 7.6 Hz), 7.40-7.42 (4 H, m), 7.69 (2 H, d, *J =* 8.0 Hz), 7.93 (1 H, s), 9.70 (1 H, d, *J =* 10.4 Hz); ¹³C NMR (DMSO-d₆, 100 MHz) δ 21.3, 21.6, 70.4, 127.7, 129.3, 129.7, 129.8, 130.0, 134.0, 139.5, 145.2, 160.6.

### 1-(Isocyano(p-tolyl)methyl)sulfonyl)-4-methylbenzene (46)

Phosphorous oxychloride (0.493 ml, 0.527 mmol) was added to solution of **25** (0.80 g, 0.263 mmol) in dry THF (5 ml) and the resulting solution was stirred for 5 min at 25 °C. After cooling the solution to 0 °C, triethylamine (2.21 ml, 1.58 mmol) was slowly added. The reaction mixture was warmed to 5-10 °C and held there for 45 min. Ethyl acetate (5 ml) and water (5 ml) were added sequentially and the mixture was stirred for 5 min. The organic layer was separated, washed with water, sat. NaHCO₃, brine and evaporated to dryness. The residue was diluted with 1-propanol (5 ml) and this solution was concentrated on the rotary evaporator to approximately half (2.5 ml) of its original volume. The residue was cooled to 5-10 °C for 30 min and the beige solid that crystallized was filtered, rinsed twice with 1-propanol and dried under vacuum to give **46** (0.420 g, 56%). ¹H NMR (CDCl₃, 400 MHz) δ 2.39 (3 H, s), 2.47 (3 H, s), 5.57 (1 H, s), 7.18-7.24 (4 H, m), 7.33 (2 H, d, *J* = 8.4 Hz), 7.63 (2 H, d, *J =* 8 Hz); ¹³C NMR (CDCl₃, 100 MHz) δ 21.3, 21.8, 76.4, 123.5, 128.3, 129.5, 129.7, 130.2, 130.5, 141.1, 146.5, 165.8.

### Benzyl (4-(1-(cyclohexyl)-4-(4-methylphenyl)-1H-imidazol-5-yl)pyrimidin-2-yl)carbamate (47)

1-(Isocyano(*p*-tolyl)methyl)sulfonyl)-4-methylbenzene **46** (0.250 g, 0.876 mmol) and 20% aq. K₂CO₃ (1.2 mL, containing 0.242 g, 1.75 mmol) were added to a solution of imine **12** (0.267 g, 0.788 mmol) in CH₂Cl₂ (5 ml) at 20 °C whilst stirring was continued overnight. At this point, additional imine (0.029 g, 0.088 mmol) and K₂CO₃ (0.061 g, 0.086 mmol) were added and the reaction mixture was stirred at 30 °C overnight. The reaction mixture was diluted with CH₂Cl₂, and washed with water, dried (MgSO₄), filtered and concentrated. Flash chromatography of the residue (MeOH/CHCl₃/Et₃N 6:93:1) followed by recrystallization using methanol afforded **47** as a white solid (0.062 g, 15%). ¹H NMR (CDCl₃, 400 MHz) δ 1.21-1.28 (2 H, m), 1.37-1.47 (2 H, m), 1.58-1.74 (2 H, m), 1.82-1.85 (2 H, m), 2.10-2.13 (2 H, m), 2.35 (3 H, s), 4.99-5.02 (1 H, m), 5.27 (2 H, s), 6.85 (1 H, d, *J* = 5.2 Hz), 7.12 (1 H, d, *J =* 8.4 Hz), 7.32-7.46 (7 H, m), 7.60 (1 H, s), 7.77 (1 H, s), 8.35 (1 H, d, *J =* 5.2 Hz); ¹³C NMR (CDCl₃, 100 MHz) δ 21.4, 25.7, 34.7, 55.4, 67.6, 116.9, 123.9, 128.6, 128.7, 128.8, 129.3, 131.7, 135.6, 136.8, 137.7, 144.9, 151.3, 157.4, 158.0, 159.8; HRMS (ESI⁺) calcd for C₂₈H₃₀N₅O₂ (M+H) 468.2400. Found 468.2402.

### 4-(1-(1-(Cyclohexyl)-4-(4-methylphenyl)-1H-imidazol-5-yl)pyrimidin-2-amine (48; SS8-186)

A mixture of **47** (0.050 g, 0.012 mmol) and 20 wt% Pd(OH)₂/C (0.010 g) in THF (3 ml) and methanol (0.3 ml) was treated with hydrogen at 200 psi for 1 h. The mixture was filtered through Celite and concentrated under reduced pressure to afford the crude product. Flash chromatography of the residue (MeOH/CHCl₃/Et₃N 7:92:1) afforded **48** as a white solid (0.020 g, 50%). ¹H NMR (CDCl₃, 400 MHz) δ 1.26-1.40 (4 H, m), 1.56-1.75 (2 H, m), 1.86-1.90 (2 H, m), 2.15-2.17 (2 H, m), 2.35 (3 H, s), 4.49-4.57 (1 H, m), 5.09 (2 H, s), 6.54 (1 H, d, *J* = 5.2 Hz), 7.10 (2 H, d, *J =* 8.0 Hz), 7.37 (2 H, d, *J* = 8.0 Hz), 7.73 (1 H, s), 8.15 (1 H, d, *J* = 5.2 Hz); ¹³C NMR (CDCl₃, 100 MHz) δ 21.4, 25.5, 26.0, 55.7, 113.2, 124.6, 128.3, 129.2, 131.7, 135.9, 137.2, 143.0, 158.4, 159.8, 163.1; HRMS (ESI⁺) calcd for C₂₀H₂₄N₅ (M+H) 334.2032. Found 334.2028.

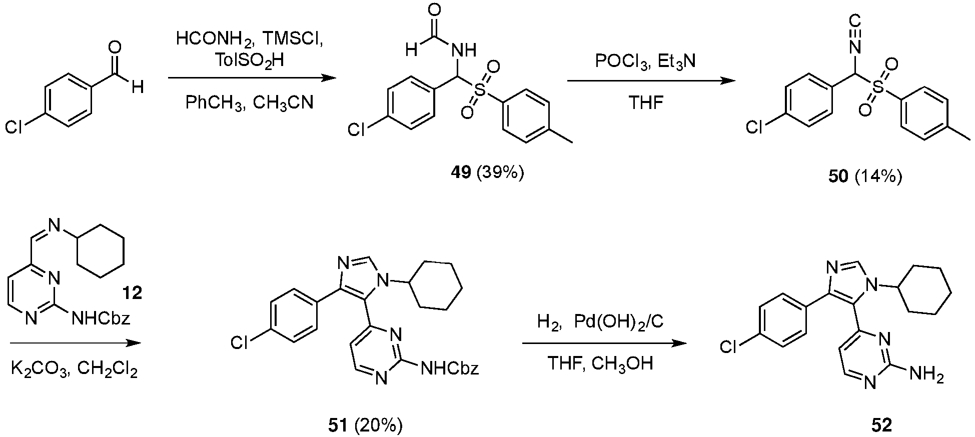

### N-((4-Chlorophenyl)(tosyl)methyl)formamide (49)

Formamide (0.801 g, 17.8 mmol) was added to a solution of p-tolualdehyde (1.00 g, 7.11 mmol) in toluene (2 ml) and acetonitrile (2 ml), followed by addition of TMSCI (0.850 g, 7.83 mmol) and TolSO₂H (1.66 g, 10.7 mmol). The resulting suspension was heated at 50 °C for 5 h. The reaction mixture was cooled to 0 °C, diethyl ether (5 ml) and H₂O (10 ml) were added and the mixture was stored in the fridge overnight. The solid white product thus obtained was collected by filtration and dried under high vacuum to give **49** (0.90 g, 39%). Compound **49** exists as a 3:1 mixture of amide rotamers at room temperature in DMSO-d₆. The spectra of the major isomer is as follows: ¹H NMR (DMSO-d₆, 400 MHz) δ 2.39 (3 H, s), 6.43 (1 H, d, *J* = 10.4 Hz), 7.41-7.51 (4 H, m), 7.57 (2 H, d, *J* = 8.4 Hz), 7.70 (2 H, d, *J* = 8.0 Hz), 7.94 (1 H, s), 9.77 (1 H, d, *J =* 10.8 Hz); ¹³C NMR (DMSO-d₆, 100 MHz) δ 21.6, 69.9, 124.9, 128.7, 129.6, 130.1, 131.7, 133.6, 134.9, 145.5, 160.7.

### 1-(Chloro-4-isocyano(tolyl)methyl)benzene (50)

Phosphorous oxychloride (0.462 ml, 0.494 mmol) was added to solution of **49** (0.800 g, 0.247 mmol) in dry THF (5 ml) and the resulting solution was stirred for 5 min at 25 °C. After cooling the solution to 0 °C, triethylamine (2.06 ml, 1.48 mmol) was slowly added. The reaction mixture was warmed to 5-10 °C and held there for 45 min. Ethyl acetate (5 ml) and water (5 ml) were added sequentially and the mixture for stirred for 5 min. The organic layer was separated, washed with water, sat. NaHCO₃, brine and evaporated to dryness. The residue was diluted with 1-propanol (5 ml) and the resulting solution was concentrated on the rotary evaporator to half of its original volume (2.5 ml). The residue was cooled to 5-10 °C for 30 min and the beige solid that crystallized was filtered, rinsed twice with 1-propanol and dried under high vacuum to give **50** (0.115 g, 14%). ¹H NMR (CDCl₃, 400 MHz) δ 2.48 (3 H, s), 5.58 (1 H, s), 7.28 (2 H, d, *J* = 8.4 Hz), 7.34-7.49 (4 H, m), 7.63 (2 H, d, *J* = 8.0 Hz); ¹³C NMR (CDCl₃, 100 MHz) δ 21.8, 75.8, 125.1, 129.1, 129.7, 129.8, 129.9, 130.5, 137.2, 146.9, 166.6.

### Benzyl (4-(1-(cyclohexyl)-4-(4-chlorophenyl)-1H-imidazol-5-yl)pyrimidin-2-yl)carbamate (51)

1-(Chloro-4-isocyano(tolyl)methyl)benzene **50** (0.120 g, 0.392 mmol) and 20% aq. K₂CO₃ (0.108 g, 0.785 mmol) were added to a solution of imine **12** (0.119 g, 0.353 mmol) in CH₂Cl₂ (5 ml) at 20 °C whilst stirring was continued overnight. At this point, additional imine (0.013 g, 0.062 mmol) and K₂CO₃ (0.047 g, 0.345 mmol) were added and the reaction mixture was stirred at 30 °C overnight. The reaction mixture was diluted with CH₂Cl₂, and washed with water, dried (MgSO₄), filtered and concentrated. Flash chromatography of the residue (MeOH/CHCl₃/Et₃N 6:93:1), followed by recrystallisation using CH₃OH/CH₂Cl₂ afforded **51** as a white solid (0.051 g, 20%). ¹H NMR (CDCl₃, 400 MHz) δ 1.21-1.28 (2 H, m), 1.36-1.46 (2 H, m), 1.56-1.74 (2 H, m), 1.82-1.86 (2 H, m), 2.10-2.13 (2 H, m), 4.96-5.02 (1 H, m), 5.27 (2 H, s), 6.81 (1 H, d, *J =* 5.6 Hz), 7.29 (1 H, d, *J* = 8.8 Hz), 7.32-7.46 (7 H, m), 7.69 (1 H, s), 7.78 (1 H, s), 8.38 (1 H, d, *J* = 5.2 Hz); ¹³C NMR (CDCl₃, 100 MHz) δ 25.7, 34.7, 55.5, 67.6, 116.8, 124.3, 128.7, 128.8, 129.9, 130.0, 133.1, 133.8, 135.8, 137.0, 143.3, 151.2, 157.6, 158.3, 159.4; HRMS (ESI⁺) calcd for C₂₇H₂₇³⁵ClN₅O₂ (M+H) 488.1853. Found 488.1861.

### 4-(1-(1-(Cyclohexyl)-4-(4-chlorophenyl)-1H-imidazol-5-yl)pyrimidin-2-amine (52; SS8-190)

A mixture of **50** (0.050 g, 0.102 mmol) and 20 wt% Pd(OH)₂/C (0.020 g) in THF (5 ml) and methanol (0.5 ml) was treated with hydrogen at 200 psi for 1 h. The mixture was filtered through Celite and concentrated under reduced pressure to afford the crude product. Flash chromatography of the residue (MeOH/CHCl₃/Et₃N 20:79:1) afforded **52,** contaminated with the dechlorinated compound. ¹H NMR (CDCl₃, 400 MHz) δ 1.23-1.42 (4 H, m), 1.56-1.76 (2 H, m), 1.87-1.91 (2 H, m), 2.15-2.17 (2 H, m), 4.49-4.55 (1 H, m), 5.06 (2 H, s), 6.54 (1 H, d, *J =* 5.2 Hz), 7.26-7.30 (2 H, m), 7.49 (2 H, d, *J =* 8.0 Hz), 7.75 (1 H, s), 8.16 (1 H, d, *J =* 5.2 Hz); HRMS (ESI⁺) calcd for C₁₉H₂₀³⁵ClN₅ (M+H) 354.1485. Found 354.1479.

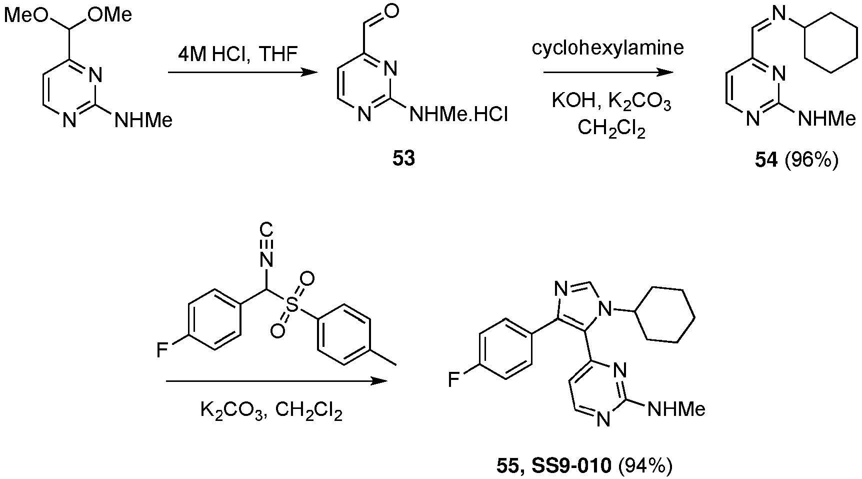

### 2-(Methylamino)pyrimidine-4-carbaldehyde hydrochloride (53)

Aqueous 4 M HCl (2 ml) was added to a solution of 4-(dimethoxymethyl)-N-methylpyrimidin-2-amine (0.060 g, 0.33 mmol) in THF (1 ml). The resulting mixture was heated to 40 °C overnight and then cooled to room temperature. The reaction mixture containing the hydrochloride salt of the aldehyde was used directly in the next step without isolation.

### 4-((Cyclohexylimino)-methyl)-N-methylpyrimidin-2-amine (54)

45% aq. KOH (0.448 g, 7.98 mmol) was added to an ice-cooled solution of crude aldehyde 53 (0.045 g, 0.33 mmol) in aq. HCl (2 ml, 7.94 mmol), while the temperature was maintained below 15 °C. To the neutralized mixture, CH₂Cl₂ (5 ml) and K₂CO₃ (0.55 g, 0.39 mmol) were added followed by cyclohexylamine (0.38 g, 0.39 mmol). The reaction mixture was gradually warmed to room temperature and stirring was continued for 2 h. ¹H NMR analysis indicated complete consumption of aldehyde. The reaction mixture was diluted with CH₂Cl₂, washed with water, dried (MgSO₄), filtered and concentrated. The crude material was used in the next step without purification (0.068 g, 48%). ¹H NMR (CDCl₃, 400 MHz) δ 1.25-1.43 (2 H, m), 1.53-1.67 (8 H, m), 5.10-5.14 (1 H, m), 7.12 (1 H, d, *J =* 4.8 Hz), 8.12 (1 H, s, NH), 8.34 (1 H, d, *J* = 5.2 Hz); ¹³C NMR (CDCl₃, 100 MHz) δ 24.6, 25.6, 34.0, 69.8, 112.4, 151.6, 157.8, 158.9, 163.2; HRMS (ESI⁺) calcd for C₁₂H₁₉N₄ (M+H) 219.1610. Found 219.1622.

### 4-(1-Cyclohexyl-4-(4-fluorophenyl)-1H-imidazol-5-yl)-N-methylpyrimidin-2-amine (55; SS9-010)

A mixture of α-(*p*-toluenesulfonyl)-4-fluorobenzylisonitrile (0.060 g, 0.275 mmol), imine **54** (0.088 g, 0.306 mmol) and 20% aq. K₂CO₃ (0.085 g, 0.612 mmol) in CH₂Cl₂ (5 ml) at 20 °C was stirred overnight. At this point, additional imine (5.0 mg, 0.027 mmol) and K₂CO₃ (7.0 mg, 0.056 mmol) were added and the reaction mixture was stirred at 30 °C for a second night. The reaction mixture was diluted with CH₂Cl₂, and washed with water, dried (MgSO₄), filtered and concentrated. Flash chromatography of the residue (EtOAc/pet. spirits 6:4) afforded **55** as a white solid (0.045g, 48%). ¹H NMR (CDCl₃, 400 MHz) δ 1.20-1.40 (4 H, m), 1.61-1.76 (2 H, m), 1.88-1.91 (2 H, m), 2.18-2.21 (2 H, m), 2.10-2.13 (2 H, m), 3.06 (3 H, d, *J* = 11.6 Hz), 4.57-4.65 (1 H, br s), 5.13-5.15 (1 H, m), 6.41 (1 H, d, *J =* 5.2 Hz), 6.96-7.01 (2 H, m), 7.44-7.49 (2 H, m), 7.73 (1 H, s), 8.16 (1 H, d, *J =* 5.2 Hz); ¹³C NMR (CDCl₃, 100 MHz) δ 25.5, 26.1, 28.6, 34.7, 55.7, 111.9, 115.2, 115.5, 130.1, 130.2, 135.9, 158.3, 159.0, 159.5, 163.2, 163.6; HRMS (ESI⁺) calcd for C₂₀H₂₃FN₅ (M+H) 352.1937. Found 352.1935.

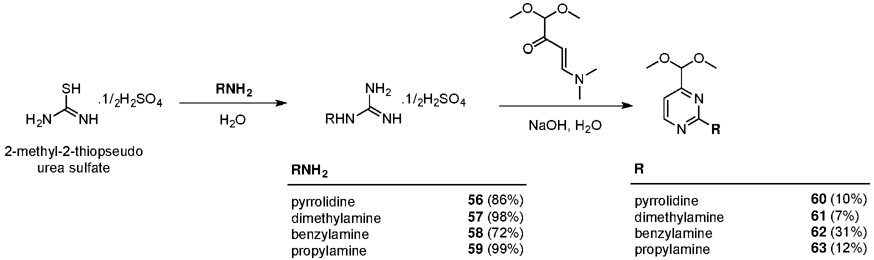

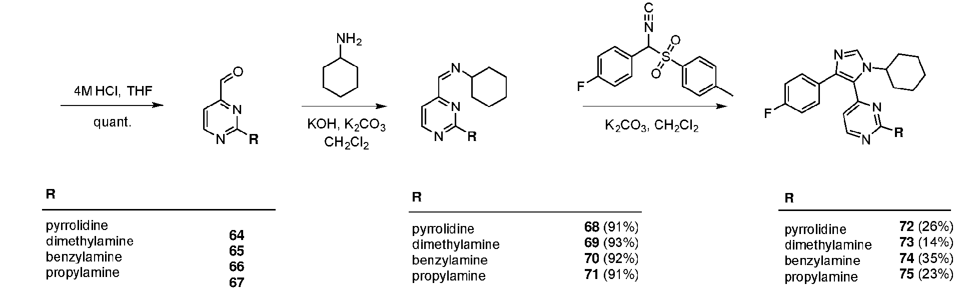

### Pyrrolidine-1-carboximidamide sulfate (56)

Equimolar amounts of pyrrolidine (1.77 ml, 21.5 mmol) and 2-methyl-2-thiopseudourea sulfate (3.00 g, 21.5 mmol) in water (10 ml) were heated under reflux for 2 h. The reaction mixture was allowed to cool to room temperature and conc. H₂SO₄ (2.25 ml, 21.5 mmol) was added. Solvent was evaporated and the solid obtained was filtered, washed with acetone and dried to afford **56** as a white solid (3.10 g, 86%). ¹H NMR (D₂O, 400 MHz) δ 1.98-2.01 (4 H, m), 3.38-3.40 (4 H, m); ¹³C NMR (D₂O, 100 MHz) δ 24.8, 46.8, 154.1; HRMS (ESI⁺) calcd for C₅H₁₂N₃ (M+H) 114.1031. Found 114.2028.

### 1,1-Dimethylguanidine sulfate (57)

Dimethylamine (40% w/v H2O) (3.08 ml, 28.7 mmol) and 2-methyl-2-thiopseudourea sulfate (2.00 g, 14.3 mmol) in water (10 ml) were stirred at room temperature for 3 days. The reaction mixture was allowed to cool to room temperature and conc. H₂SO₄ (0.76 ml, 14.3 mmol) was added. Solvent was evaporated and the solid obtained was filtered, washed with acetone and dried to afford **57** as a white solid (2.01 g, 98%), m.p. 280 °C. ¹H NMR (D₂O, 400 MHz) δ 3.03 (6 H, s); ¹³C NMR (D₂O, 100 MHz) δ 37.3, 156.7; HRMS (ESI⁺) calcd for C₃H₁₀N₃ (M+H) 88.0875. Found 88.0878.

### 1-Benzylguanidine sulfate (58)

Benzylamine (1.57 ml, 17.2 mmol) was added to a solution of 2-methyl-2-thiopseudourea sulfate (2.00 g, 14.3 mmol) in water (10 ml) and ethanol (10 ml). The resulting reaction mixture was heated under reflux for 12 h. The reaction mixture was allowed to cool to room temperature and conc. H₂SO₄ (0.76 ml, 14.3 mmol) was added. Solvent was evaporated and the solid obtained was filtered, washed with diethyl ether and dried to afford 58 as a white solid (2.36 g, 72%). ¹H NMR (D₂O, 400 MHz) δ 3.82 (2 H, s), 7.23-7.27 (5 H, m); ¹³C NMR (D₂O, 100 MHz) δ 44.4, 126.8, 127.9, 128.9, 136.1, 156.8; HRMS (ESI⁺) calcd for C₈H₁₂N₃ (M+H) 150.1031. Found 150.1037.

### 1-Propylguanidine sulfate (59)

Propylamine (1.77 ml, 22.1 mmol) and 2-methyl-2-thiopseudourea sulfate (2.00 g, 14.3 mmol) in water (10 ml) were stirred at room temperature for 3 days. The reaction mixture was allowed to cool to room temperature and conc. H₂SO₄ (0.76 ml, 14.3 mmol) was added. Solvent was evaporated and the solid obtained was filtered, washed with diethyl ether and dried to afford 59 as a white solid (2.22 g, 99%). ¹H NMR (D₂O, 400 MHz) δ 0.92 (3 H, t, *J* = 8 Hz), 1.56-1.61 (2 H, sex, *J =* 8 Hz), 3.12 (3 H, t, *J* = 8 Hz); ¹³C NMR (D₂O, 100 MHz) δ 10.3, 21.3, 42.7, 156.7; HRMS (ESI⁺) calcd for C₄H₁₂N₃ (M+H) 102.1031. Found 102.1037.

### 4-(Dimethoxymethyl)-2-(pyrrolidin-1-yl)pyrimidine (60)

Sodium hydroxide (0.706 g, 17.6 mmol) and **56** (1.00 g, 8.83 mmol) were added to a solution of *E*-1,1-dimethoxy-4-dimethylaminobut-2-en-2-one (2.29 g, 13.2 mmol) in water (10 ml). The resulting mixture was heated under reflux for 2 h. The reaction mixture was then allowed to cool to room temperature filtered to remove the precipitated sodium sulfate. The aqueous filtrate was extracted several times with CH₂Cl₂. Combined organic layers were dried (MgSO₄), filtered and concentrated. Flash chromatography of the residue (EtOAc/pet. spirits 1:1) afforded **60** as a light yellow oil (0.110 g, 10%). ¹H NMR (CDCl₃, 400 MHz) δ 1.96-2.00 (4 H, m), 3.42 (6 H, m), 3.57-3.60 (4 H, m), 5.08 (1 H, s), 6.68 (1 H, d, *J =* 5.2 Hz), 8.34 (1 H, d, *J =* 5.2 Hz); ¹³C NMR (CDCl₃, 100 MHz) δ 25.7, 46.8, 54.0, 103.8, 105.7, 158.4, 160.3, 165.9; HRMS (ESI⁺) calcd for C₁₁H₁₈N₃O₂ (M+H) 224.1399. Found 224.1398

### 4-(Dimethoxymethyl)-N,N-dimethylpyrimidin-2-amine (61)

Sodium hydroxide (0.867 g, 7.35 mmol) and **57** (1.00 g, 7.35 mmol) were added to a solution of *E*-1,1-dimethoxy-4-dimethylaminobut-2-en-2-one (1.91 g, 11.0 mmol) in water (10 ml). The resulting mixture was heated under reflux for 16 h. The reaction was diluted with CH₂Cl₂ and washed with water. After several extractions, the combined organic layers were dried (MgSO₄), filtered and concentrated. Flash chromatography of the residue (EtOAc/pet. spirits 1:1) afforded **61** as a light yellow oil (0.09 g, 7%). ¹H NMR (CDCl₃, 400 MHz) δ 3.18 (6 H, s), 3.41 (6 H, s), 5.08 (1 H, s), 6.66 (1 H, d, *J* = 5.2 Hz), 8.33 (1 H, d, *J* = 5.2 Hz); ¹³C NMR (CDCl₃, 100 MHz) δ 37.1, 54.0, 103.9, 105.7, 158.4, 162.3, 165.6; HRMS (ESI⁺) calcd for C₉H₁₆N₃O₂ (M+H) 198.1243. Found 198.1238.

### N-Benzyl-4-(dimethoxymethyl)pyrimidin-2-amine (62)

Sodium hydroxide (0.606 g, 15.2 mmol) and **58** (1.50 g, 7.57 mmol) were added to a solution of *E*-1,1-dimethoxy-4-dimethylaminobut-2-en-2-one (1.97 g, 11.3 mmol) in water (10 ml). The resulting mixture was heated under reflux for 16 h. The reaction was diluted with CH₂Cl₂ and washed with water. After several extractions, the combined organic layers were dried (MgSO₄), filtered and concentrated. Flash chromatography of the residue (EtOAc/pet. spirits 1:1) afforded **62** as a light yellow oil (0.6 g, 31%). ¹H NMR (CDCl₃, 400 MHz) δ 3.39 (6 H, s), 5.65 (2 H, d, *J* = 6Hz), 5.10 (1 H, s), 5.57 (1 H, br s), 6.77 (1 H, d, *J* = 5.2 Hz), 7.24-7.36 (5 H, m), 8.33 (1 H, d, *J* = 4.8 Hz); ¹³C NMR (CDCl₃, 100 MHz) δ 45.7, 53.8, 103.0, 108.0, 127.4, 127.7, 128.7, 139.2, 159.0, 162.4, 166.2; HRMS (ESI⁺) calcd for C₁₄H₁₇N₃O₂ (M+H) 260.1399. Found 260.1401.

### 4-(Dimethoxymethyl)-N-propylpyrimidin-2-amine (63)

Sodium hydroxide (0.80 g, 20.1 mmol) and **59** (1.50 g, 10.1 mmol) were added to a solution of *E*-1,1-dimethoxy-4-dimethylaminobut-2-en-2-one (2.07 g, 12.1 mmol) in water (10 ml). The resulting mixture was heated under reflux for 16 h. The reaction was diluted with CH₂Cl₂ and washed with water. After several extractions, the combined organic layers were dried (MgSO₄), filtered and concentrated. Flash chromatography of the residue (EtOAc/pet. spirits 1:1) afforded **63** as a light yellow oil (0.25 g, 12%). ¹H NMR (CDCl₃, 400 MHz) δ 0.97 (3 H, t, *J =* 8 Hz), 1.59-1.65 (2 H, m), 3.36-3.41 (2 H, m), 3.39 (6 H, s), 5.09 (1 H, s), 6.72 (1 H, d, *J* = 4.8 Hz), 8.33 (1 H, d, *J =* 5.2 Hz); ¹³C NMR (CDCl₃, 100 MHz) δ 11.6, 22.9, 43.5, 53.7, 103.0, 107.5, 158.9, 162.6, 166.1; HRMS (ESI⁺) calcd for C₁₀H₁₈N₃O₂ (M+H) 212.1399. Found 212.1401

### 1-(4-formylpyrimidin-2-yl)pyrrolidin hydrochloride (64)

Aqueous 4 M HCl (2 ml) was added to a solution of 60 (0.080 g, 0.358 mmol) in THF (1 ml). The resulting mixture was heated to 40 °C overnight and then cooled to room temperature. The reaction mixture containing the hydrochloride salt of the aldehyde was used directly in the next step without isolation.

### 2-(Dimethylamino)pyrimidin-4-carbaldehyde hydrochloride (65)

Aqueous 4 M HCl (2 ml) was added to a solution of **61** (0.080 g, 0.406 mmol) in THF (1 ml). The resulting mixture was heated to 40 °C overnight and then cooled to room temperature. The reaction mixture containing the hydrochloride salt of the aldehyde was used directly in the next step without isolation.

### 2-(Benzylamino)pyrimidin-4-carbaldehyde hydrochloride (66)

Aqueous 4 M HCl (2 ml) was added to a solution of **62** (0.200 g, 0.772 mmol) in THF (1 ml). The resulting mixture was heated to 40 °C overnight and then cooled to room temperature. The reaction mixture containing the hydrochloride salt of the aldehyde was used directly in the next step without isolation.

### 2-(Propylamino)pyrimidin-4-carbaldehyde hydrochloride (67)

Aqueous 4 M HCl (2 ml) was added to a solution of **63** (0.200 g, 0.948 mmol) in THF (1 ml). The resulting mixture was heated to 40 °C overnight and then cooled to room temperature. The reaction mixture containing the hydrochloride salt of the aldehyde was used directly in the next step without isolation.

### N-((2-(Pyrrolidin-1-yl)pyrimidin-4-yl)methylene)cyclohexanamine (68)

45% aq. KOH (0.448 g, 7.98 mmol) was added to an ice-cooled solution of crude aldehyde **64** (0.060 g, 0.338 mmol) in aq. HCl (2 ml, 7.94 mmol), while the temperature was maintained below 15 °C. To the neutralized mixture, CH₂Cl₂ (5 ml) and K₂CO₃ (0.047 g, 0.338 mmol) were added followed by cyclohexylamine (0.040 g, 0.406 mmol). The reaction mixture was gradually warmed to room temperature and stirring was continued overnight. ¹H NMR analysis indicated complete consumption of aldehyde. The reaction mixture was diluted with CH₂Cl₂, washed with water, dried (MgSO₄), filtered and concentrated. The crude material was used in the next step without purification (0.080 g, 91%). ¹H NMR (CDCl₃, 400 MHz) δ 1.25-1.42 (4 H, m), 1.66-1.84 (6 H, m), 1.98-2.01 (4 H, m), 3.26-3.31 (1 H, m), 3.58-3.61 (4 H, m), 7.08 (1 H, d, *J =* 4.8 Hz), 8.15 (1 H, s, NH), 8.34 (1 H, d, *J =* 5.2 Hz); ¹³C NMR (CDCl₃, 100 MHz) δ 24.6, 25.5, 25.6, 34.0, 46.6, 69.7, 104.8, 158.1, 158.3, 159.7, 162.2; HRMS (ESI⁺) calcd for C₁₅H₂₃N₄ (M+H) 259.1923. Found 259.1915

### 4-((Cyclohexylimino)-methyl)-N,N-dimethylpyrimidin-2-amine (69)

45% aq. KOH (0.448 g, 7.98 mmol) was added to an ice-cooled solution of crude aldehyde **65** (0.063 g, 0.417 mmol) in aq. HCl (2 ml, 7.94 mmol), while the temperature was maintained below 15 °C. To the neutralized mixture, CH₂Cl₂ (5 ml) and K₂CO₃ (0.057 g, 0.417 mmol) were added followed by cyclohexylamine (0.049 g, 0.534 mmol). The reaction mixture was gradually warmed to room temperature and stirring was continued for overnight. ¹H NMR analysis indicated complete consumption of aldehyde. The reaction mixture was diluted with CH₂Cl₂, washed with water, dried (MgSO₄), filtered and concentrated. The crude material was used in the next step without purification (0.091 g, 93%). ¹H NMR (CDCl₃, 400 MHz) δ 1.02-1.43 (4 H, m), 1.61-1.85 (6 H, m), 3.19 (3 H , s), 3.21 (3 H, s), 3.24-3.31 (1 H, m), 7.08 (1 H, d, *J =* 5.2 Hz), 8.14 (1 H, s, NH), 8.34 (1 H, d, *J* = 5.2 Hz); ¹³C NMR (CDCl₃, 100 MHz) δ 24.8, 25.7, 34.2, 37.2, 69.9, 105.0, 158.2, 158.4, 159.9, 162.3; HRMS (ESI⁺) calcd for C₁₃H₂₄N₄ (M+H) 233.1766. Found 233.1758

### N-benzyl-4-((Cyclohexylimino)-methyl)pyrimidin-2-amine (70)

45% aq. KOH (0.448 g, 7.98 mmol) was added to an ice-cooled solution of crude aldehyde **66** (0.2 g, 0.939 mmol) in aq. HCl (2 ml, 7.94 mmol), while the temperature was maintained below 15 °C. To the neutralized mixture, CH₂Cl₂ (5 ml) and K₂CO₃ (0.129 g, 0.939 mmol) were added followed by cyclohexylamine (0.112 g, 11.3 mmol). The reaction mixture was gradually warmed to room temperature and stirring was continued for overnight. ¹H NMR analysis indicated complete consumption of aldehyde. The reaction mixture was diluted with CH₂Cl₂, washed with water, dried (MgSO₄), filtered and concentrated. The crude material was used in the next step without purification (0.250 g, 92%). ¹H NMR (CDCl₃, 400 MHz) δ 1.24-1.38 (4 H, m), 1.56-1.68 (4 H, m), 1.81-1.84 (2 H, m), 3.24-3.31 (1 H, m), 3.39 (2 H, s), 6.73 (1 H, d, *J =* 4.8 Hz), 7.24-7.27 (5 H, m), 8.11 (1 H, s), 8.32 (1 H, d, *J* = 5.2 Hz); ¹³C NMR (CDCl₃, 100 MHz) δ 24.7, 25.6, 33.9, 45.5, 69.8, 107.8, 127.2, 127.4, 128.5, 139.08, 158.8, 158.9, 162.4, 166.0; HRMS (ESI⁺) calcd for C₁₂H₁₉N₄ (M+H) 295.1923. Found 295.1915.

### 4-((Cyclohexylimino)-methyl)-N-propylpyrimidin-2-amine (71)

45% aq. KOH (0.448 g, 7.98 mmol) was added to an ice-cooled solution of crude aldehyde **67** (0.15 g, 0.908 mmol) in aq. HCl (2 ml, 7.94 mmol), while the temperature was maintained below 15 °C. To the neutralized mixture, CH₂Cl₂ (5 ml) and K₂CO₃ (0.125 g, 0.908 mmol) were added followed by cyclohexylamine (0.108 g, 11.1 mmol). The reaction mixture was gradually warmed to room temperature and stirring was continued for overnight. ¹H NMR analysis indicated complete consumption of aldehyde. The reaction mixture was diluted with CH₂Cl₂, washed with water, dried (MgSO₄), filtered and concentrated. The crude material was used in the next step without purification (0.250 g, 91%). ¹H NMR (CDCl₃, 400 MHz) δ 0.96-1.00 (3 H, t, *J* = 8 Hz), 1.24-1.38 (4 H, m), 1.56-1.84 (8 H, m), 3.25-3.30 (1 H, m), 3.39 (2 H, t, *J* = 8.4 Hz), 5.09 (1 H, br s), 6.72 (1 H, d, *J* = 5.2 Hz), 8.11 (1 H, s, NH), 8.32 (1 H, d, *J* = 4.8 Hz); ¹³C NMR (CDCl₃, 100 MHz) δ 11.4, 22.8, 24.6, 25.1, 43.3, 69.9, 105.0, 158.2, 158.4, 159.9, 162.3; HRMS (ESI⁺) calcd for C₁₂H₁₉N₄ (M+H) 247.1923. Found 247.1915.

### 4-(1-Cyclohexyl-4-(4-fluorophenyl)-1H-imidazol-5-yl)-2-(pyrrolidin-1-yl)pyrimidine (72; SS9-098)

A mixture of α-(*p*-toluenesulfonyl)-4-fluorobenzylisonitrile (0.099 g, 0.344 mmol), crude imine **68** (0.070 g, 0.309 mmol) and 20% aq. K₂CO₃ (0.095 g, 0.688 mmol) in CH₂Cl₂ (5 ml) at 20 °C was stirred overnight. At this point, additional K₂CO₃ (0.024 g, 0.172 mmol) was added and the reaction mixture was stirred at 30 °C for a second night. The reaction mixture was diluted with CH₂Cl₂, and washed with water, dried (MgSO₄), filtered and concentrated. Flash chromatography of the residue (EtOAc/pet. spirits 4:6) afforded impure product which was re-purified by HPLC to give **72** (0.028g, 26%). ¹H NMR (MeOH, 400 MHz, HCl salt) δ 1.11-1.31 (4 H, m), 1.39-1.69 (4 H, m), 1.75-1.81 (2 H, m), 1.75-1.81 (2 H, m), 1.95-2.01 (2 H, m), 2.09-2.16 (2 H, m), 3.48-3.71 (4H, m), 4.75-4.81 (1 H, m), 6.49 (1 H, d, *J =* 6.4 Hz), 7.13-7.18 (2 H, m), 7.36-7.40 (2 H, m), 7.94 (1 H, d, *J =* 6.8 Hz), 9.03 (1 H, s); ¹³C NMR (CDCl₃, 100 MHz) δ 24.7, 25.7, 34.0, 47.3, 58.2, 109.7, 116.6, 116.8, 122.9, 130.7,130.8, 155.9, 157.6, 162.5, 165.0; HRMS (ESI⁺) calcd for C₂ₒH₂₃FN₅ (M+H) 392.2250. Found 392.2247.

### 4-(1-Cyclohexyl-4-(4-fluorophenyl)-1H-imidazol-5-yl)-N,N-dimethylpyrimidin-2-amine (73; SS9-078)

A mixture of α-(*p*-toluenesulfonyl)-4-fluorobenzylisonitrile (0.124 g, 0.430 mmol), crude imine **69** (0.090 g, 0.387 mmol) and 20% aq. K₂CO₃ (0.119 g, 0.861 mmol) in CH₂Cl₂ (5 ml) at 20 °C was stirred overnight. At this point, additional K₂CO₃ (0.030 g, 0.215 mmol) was added and the reaction mixture was stirred at 30 °C for a second night. The reaction mixture was diluted with CH₂Cl₂, and washed with water, dried (MgSO₄), filtered and concentrated. Flash chromatography of the residue (EtOAc/pet. spirits 6:4) afforded impure product which was re-purified by HPLC to give **73** (0.022 g, 14%).¹H NMR (CDCl₃, 400 MHz) δ 1.25-1.39 (4 H, m), 1.73-1.79 (2 H, m), 1.95-1.98 (2 H, m), 2.24-2.26 (2 H, m), 3.26 (6 H, s), 4.70-4.75 (1 H, m), 6.31 (1 H, d, *J* = 5.2 Hz), 7.09-7.12 (2 H, m), 7.46-7.48 (2 H, m), 8.32 (1 H, d, *J =* 5.2 Hz), 8.85 (1 H, s); ¹³C NMR (CDCl₃, 100 MHz) δ 25.0, 25.8, 34.3, 37.3, 58.0, 110.1, 116.5, 116.7, 123.3, 126.7, 130.66, 130.73, 133.8, 134.4, 155.1, 159.0, 162.2, 162.7, 164.7; HRMS (ESI⁺) calcd for C₂ₒH₂₃FN₅ (M+H) 366.2094. Found 365.2089.

### N-benzyl-4-(1-cyclohexyl-4-(4-fluorophenyl)-1H-imidazol-5-yl)pyrimidin-2-amine (74; SS9-102)

A mixture of α-(*p*-toluenesulfonyl)-4-fluorobenzylisonitrile (0.273 g, 0.945 mmol), crude imine **69** (0.230 g, 0.850 mmol) and 20% aq. K₂CO₃ (0.261 g, 1.891 mmol) in CH₂Cl₂ (5 ml) at 20 °C was stirred overnight. At this point, additional K₂CO₃ (0.065 g, 0.472 mmol) and imine (0.023 g, 0.085 mmol) were added and the reaction mixture was stirred at 30 °C for a second night. The reaction mixture was diluted with CH₂Cl₂, and washed with water, dried (MgSO₄), filtered and concentrated. Flash chromatography of the residue (EtOAc/pet. spirits 6:4) afforded **74** as a white solid (0.110 g, 35%). ¹H NMR (CDCl₃, 400 MHz) δ 1.19-1.25 (4 H, m), 1.56-1.62 (2 H, m), 1.81-1.86 (2 H, m), 2.11-2.14 (2 H, m), 4.54-4.57 (1 H, m), 4.71 (2 H, d, *J* = 6.0 Hz), 5.55 (1 H, br s), 6.44 (1 H, d, *J* = 5.2 Hz), 6.96-7.01 (2 H, m), 7.28-7.31 (1 H, m), 7.34-7.37 (4 H, m), 7.44-7.47 (2 H, m), 7.72 (1 H, s), 8.16 (1 H, d, *J =* 5.2 Hz); ¹³C NMR (CDCl₃, 100 MHz) δ 25.3, 25.7, 34.5, 45.3, 55.4, 112.3, 115.2, 115.3, 125.0, 127.2, 127.3, 128.7, 129.9, 130.0, 130.7, 135.7, 140.0, 141.6, 158.3, 160.0, 161.0, 62.4, 163.5; HRMS (ESI⁺) calcd for C₂₀H₂₃FN₅ (M+H) 428.2250. Found 428.2247.

### 4-(1-Cyclohexyl-4-(4-fluorophenyl)-1H-imidazol-5-yl)-propylpyrimidin-2-amine (75; SS9-106)

A mixture of α-(*p*-toluenesulfonyl)-4-fluorobenzylisonitrile (0.260 g, 0.902 mmol), crude imine **71** (0.200 g, 0.812 mmol) and 20% aq. K₂CO₃ (0.250 g, 1.812 mmol) in CH₂Cl₂ (5 ml) at 20 °C was stirred overnight. At this point, additional K₂CO₃ (0.064 g, 0.448 mmol) and imine (0.021 g, 0.081 mmol) were added and the reaction mixture was stirred at 30 °C for a second night. The reaction mixture was diluted with CH₂Cl₂, and washed with water, dried (MgSO₄), filtered and concentrated. Flash chromatography of the residue (EtOAc/pet. spirits 6:4) afforded 75 as a white solid (0.085 g, 23%). ¹H NMR (CDCl₃, 400 MHz) δ 1.01 (3 H, t, *J* = 7.2 Hz), 1.23-1.39 (4 H, m), 1.59-1.76 (4H, m), 1.88-1.91 (2 H, m), 2.16-2.19 (2 H, m), 3.43 (2 H, q, *J =* 7.2 Hz), 4.55-4.58 (1 H, br s), 5.19-5.35 (1 H, m), 6.38 (1 H, d, *J* = 5.2 Hz), 6.98-7.01 (2 H, m), 7.43-7.47 (2 H, m), 7.72 (1 H, s), 8.14 (1 H, d, *J* = 5.2 Hz); ¹³C NMR (CDCl₃, 100 MHz) δ 11.6, 23.1, 25.5, 26.0, 34.7, 43.5, 55.6, 111.9, 115.2, 115.4, 130.1, 130.2, 135.8, 141.5, 158.3, 161.2, 163.2, 163.6; HRMS (ESI⁺) calcd for C₂ₒH₂₃FN₅ (M+H) 380.2250. Found 380.2246.

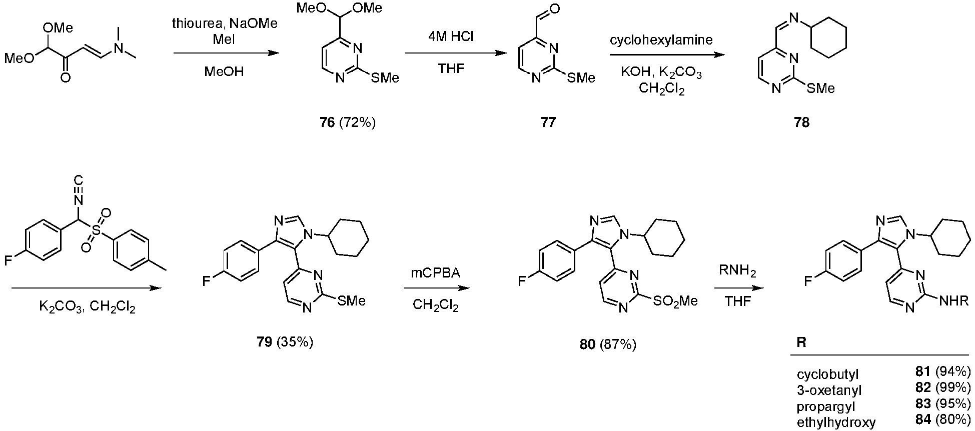

### 4-Dimethoxymethyl-2-methylsulfanyl-pyrimidine (76)

A mixture of 1,1-Dimethoxy-4-dimethylaminobut-2-en-2-one (5.00 g, 28.9 mmol), thiourea (3.30 g, 43.4 mmol) and 2.5 M NaOMe (2.34 g, 43.4 mmol) in MeOH (80 mL) was heated at reflux overnight. The mixture was cooled to r.t. then Mel (2.16 mL, 34.7 mmol) was added dropwise. The mixture was stirred at r.t. overnight. The mixture was concentrated in vacuum. The residue was extracted into EtOAc and washed with water, brine, dried with Na₂SO₄ and concentrated in vacuum. The residue was purified by flash chromatography (EtOAc/pet. spirits 1:5) to give the thioether (4.14 g, 72%) as a colourless oil. ¹H NMR (CDCl₃, 400 MHz) δ 2.91 (3 H, s), 5.67 (6 H, s), 5.18 (1 H, s), 7.18 (1 H, d, *J* = 4.0 Hz), 8.56 (1 H, d, *J* = 4.0 Hz); ¹³C NMR (CDCl₃, 100 MHz) δ 14.3, 54.2, 103.2, 113.4, 158.0, 165.7, 172.8.

### 2-(Methylthio)pyrimidine-4-carbaldehyde (77)

Aqueous 4 M HCl (13 ml) was added to a solution of 4-dimethoxymethyl-2-methylsulfanyl-pyrimidine (4.10 g, 20.5 mmol). The resulting mixture was heated to 50 °C overnight. ¹H NMR analysis indicated conversion to the carbaldehyde so the mixture was cooled to room temperature. The reaction mixture was diluted with EtOAc and neutralized with K₂CO₃ solution. The aqueous phase was extracted with EtOAc, dried with MgSO₄ and concentrated. The crude material was used in the next step without purification (2.74 g, 87%). ¹H NMR (CDCl₃, 400 MHz) δ 2.64 (3 H, s), 7.44 (1 H, d, *J =* 4.8 Hz), 8.77 (1 H, d, *J =* 4.8 Hz), 9.96 (1 H, s).

### 4-((Cyclohexylimino)-methyl)-N-methylthiopyrimidin-2-amine (78)

20%. Aqueous K₂CO₃ (0.55 g, 0.39 mmol) and cyclohexylamine (2.45 mL, 21.4 mmol) were added to a solution of the crude aldehyde (2.74 g, 17.8 mmol) in CH₂Cl₂ (15 mL). The reaction mixture was stirred at r.t. overnight. ¹H NMR analysis indicated complete consumption of the aldehyde. The reaction mixture containing the imine was used directly in the next step without isolation.

### 4-(1-Cyclohexyl-4-(4-fluorophenyl)-1H-imidazol-5-yl)-2-(methylthio)pyrimidine (79)

A mixture of α-(*p*-toluenesulfonyl)-4-fluorobenzylisonitrile (5.67 g, 19.6 mmol), imine (2.74 g, 17.8 mmol) and K₂CO₃ (2.71 g, 19.6 mmol) in CH₂Cl₂ (15 ml) was stirred at r.t. overnight. The reaction mixture was diluted with CH₂Cl₂, and washed with water, dried (MgSO₄), filtered and concentrated. Flash chromatography of the residue (EtOAc/pet. spirits 1:1) afforded a solid which was recrystallized from EtOAc/pet. spirits) to give the sulfide as pale yellow crystals, (2.32 g, 35%) m.p. 192-195°C. ¹H-NMR (400 MHz; CDCl₃): δ 1.41-1.19 (3 H, m), 1.75-1.59 (3 H, m), 1.88 (2 H, d, *J* = 13.3 Hz), 2.16 (2 H, d, *J* = 11.3 Hz), 2.58 (3 H, s), 4.62 (1 H, tt, *J* = 11.9, 3.4 Hz), 6.76 (1 H, d, *J* = 5.2 Hz), 6.99 (2 H, t, *J* = 8.6 Hz), 7.40 (2 H, dd, *J* = 8.5, 5.5 Hz), 7.76 (1 H, s), 8.31 (1 H, d, *J* = 5.2 Hz); ¹³C-NMR (101 MHz; CDCl₃): δ 14.2, 25.4, 26.0, 34.7, 55.9, 115.5, 115.71 (s, 1C), 117.2, 124.2, 130.3 (d, *J*_{C-F} = 8.0 Hz), 130.6 (d, *J*_{C-F} = 3.2 Hz), 136.6, 143.1, 157.1, 158.2, 162.6 (d, *J*_{C-F} = 247 Hz), 173.0. HRMS (ESI⁺) calcd for C₂₀H₂₂FN₄S (M+H) 369.1549. Found 369.1545.

### 4-(1-Cyclohexyl-4-(4-fluorophenyl)-1H-imidazol-5-yl)-2-methylsulfonylpyrimidin-2-amine (80)

To a mixture of sulfide (0.404 g, 1.10 mmol) in CH₂Cl₂ (20 mL) was added mCPBA (0.569g, 3.30 mmol) portionwise and the mixture was stirred at at r.t. overnight. TLC indicated conversion to a more polar compound. The mixture was quenched with aq. Na₂CO₃, water, brine, dried with Na₂SO₄, and concentrated in vacuum to give the sulfone as a colourless solid (0.440 g, 87%) m.p. 196-202°C. ¹H-NMR (400 MHz; CDCl₃): δ 1.93-1.21 (8 H, m), 2.23 (2 H, d, *J =* 11.3 Hz), 3.39 (3 H, s), 4.85 (1 H, tt, *J* = 11.8, 3.4 Hz), 7.08 (2 H, t, *J* = 8.6 Hz), 7.27 (1 H, m), 7.43 (2 H, dd, *J* = 8.5, 5.5 Hz), 7.86 (1 H, s), 8.59 (1 H, d, *J* = 5.4 Hz); ¹³C-NMR (101 MHz; CDCl₃): δ 25.5, 25.8, 34.9, 39.2, 56.9, 116.0, 116.3, 123.1, 130.3 (d, *J*_{C-F} = 3.6 Hz, 1C), 130.7 (d, *J*_{C-F} = 8.2 Hz), 138.2, 146.0, 157.5, 159.8, 163.1 (d, *J*_{C-F} = 249 Hz), 166.3. HRMS (ESI⁺) calcd for C₂₀H₂₂FN₄O₂S (M+H) 401.1447. Found 401.1444.

### N-Cyclobutyl-4-(1-cyclohexyl-4-(4-fluorophenyl)-1H-imidazol-5-yl)pyrimidin-2-amine (81)

Cyclobutylamine (35.0 µL, 0.569 mmol) was added to a mixture of **80** (38.0 mg, 0.095 mmol) in THF (2 mL) and the mixture was stirred at r.t. for 40 h. The mixture was concentrated under vacuum and the compound purified by flash chromatography (EtOAc/ pet. spirits 1:1) to give a pale yellow solid (37.0 mg, 99%), m.p. 185-189°C. ¹H-NMR (500 MHz; CDCl₃): δ 1.99-1.29 (13 H, m,), 2.17 (2 H, d, *J =* 11.2 Hz), 2.43 (2 H, qd, *J =* 7.7, 3.3 Hz,), 4.50 (1 H, dq, *J =* 16.1, 8.0 Hz), 4.60 (1 H, br s), 5.46 (1 H, br s), 6.38 (1 H, d, *J* = 5.1 Hz), 7.00-6.96 (2H, m), 7.46-7.44 (2 H, m), 7.73 (1 H, s), 8.13 (1 H, d, *J* = 4.3 Hz); ¹³C-NMR (126 MHz; CDCl₃): δ 15.2, 25.5, 26.0, 31.9, 34.7, 46.6, 55.5, 112.1, 115.2, 115.4, 125.2, 130.1 (d, *J*_{C-F} = 8.0 Hz), 130.8 (d, *J*_{C-F} = 3.1 Hz), 135.8, 141.6, 158.3, 159.1, 161.7, 162.4 (d, *J*_{C-F} = 247 Hz). HRMS (ESI⁺) calcd for C₂₃H₂₇FN₅ (M+H) 392.2250. Found 392.2247.

### 4-(1-Cyclohexyl-4-(4-fluorophenyl)-1H-imidazol-5-yl)-N-(oxetan-3-yl)pyrimidin-2-amine (82)

3-Oxetanamine (48.0 µL, 0.053 mmol) was added to a mixture of **80** (48.0 mg, 0.120 mmol) in THF (2 mL) and the mixture was stirred at r.t. for 24 h. TLC indicated the reaction was incomplete, so additional 3-oxetanamine (48 µL, 0.053 mmol) was added and stirring continued for a further 48 h. The mixture was concentrated under vacuum and the compound purified by flash chromatography (EtOAc/ pet. spirits 1:1) to give a pale yellow solid (44.9 mg, 95%). ¹H-NMR (500 MHz; CDCl₃): δ 1.92-1.27 (8 H, m), 2.15-2.12 (2 H, m), 4.48 (1 H. dd, *J* = 9.6, 6.0 Hz), 4.62 (2 H, t, *J* = 6.5 Hz), 4.98 (2 H, t, *J =* 7.0 Hz), 5.15 (1 H, q, *J* = 6.8 Hz), 6.46 (1 H, d, *J =* 5.1 Hz), 6.99-6.95 (2 H, m), 7.41 (2 H, td, *J* = 6.1, 2.6 Hz), 7.74 (1 H, s), 8.17 (1 H, d, *J* = 5.1 Hz); ¹³C NMR (126 MHz; CDCl₃) δ 21.2, 25.4, 26.0, 34.64, 46.6, 55.6, 76.9, 77.4, 79.2, 113.0, 115.3, 115.5, 124.9, 130.1 (d, *J*_{C-F} = 8.1 Hz), 130.6 (d, *J*_{C-F} = 3.0 Hz), 136.0, 141.9, 158.4, 159.2, 161.5 (s, 1C), 162.4 (d, *J*_{C-F} = 247.2 Hz). HRMS (ESI⁺) calcd for C₂₂H₂₅FN₅O (M+H) 394.2043. Found 394.2042.

### 4-(1-Cyclohexyl-4-(4-fluorophenyl)-1H-imidazol-5-yl)-N-(prop-2-yn-1-yl)pyrimidin-2-amine (83)

Propargylamine (56.0 µL, 0.869 mmol) was added to a mixture **80** (58.0 mg, 0.145 mmol) in THF (5 mL) and the mixture was stirred at r.t. for 24 h. TLC indicated the reaction was incomplete, so additional propargylamine (112 µL, 1.74 mmol) was added and stirring continued for a further 6 days. additional 3-oxetanamine (48 µL, 0.053 mmol) was added and stirring continued for a further 48 h. The mixture was concentrated under vacuum and the compound purified by flash chromatography (EtOAc/ pet. spirits 1:1) to give a pale orange solid (51.3 mg, 94%), m.p. 147-155°C. ¹H-NMR (500 MHz; CDCl₃): δ 2.19-1.20 (10 H, m), 2.24 (1 H, t, *J =* 2.5 Hz), 4.28 (2 H, dd, *J* = 5.8, 2.5 Hz), 4.61-4.59 (1 H, m), 5.63-5.59 (1 H, m), 6.48 (1 H, d, *J* = 5.1 Hz), 7.01-6.96 (2 H, m), 7.46-7.42 (2 H, m), 7.75 (1 H, s), 8.19 (1 H, d, *J* = 5.1 Hz); ¹³C NMR (126 MHz; CDCl₃) δ 25.5, 25.9, 31.3, 34.8, 55.6, 71.2, 81.0, 112.9, 115.3, 115.5, 125.0, 130.2 (d, *J*_{C-F} = 8.0 Hz), 130.8 (d, *J*_{C-F} = 3.3 Hz), 136.0, 142.0, 158.4, 159.1, 161.8, 162.4 (d, *J*_{C-F} = 247 Hz). HRMS (ESI⁺) calcd for C₂₂H₂₃FN₅ (M+H) 376.1937. Found 376.1934.

### 2-((4-(1-Cyclohexyl-4-(4-fluorophenyl)-1H-imidazol-5-yl)pyrimidin-2-yl)amino)ethan-1-ol (84)

Ethanolamine (93 µL, 1.56 mmol) was added to a mixture of 80 (0.104 g, 0.261 mmol) in THF (5 mL) and the mixture was stirred at r.t. for 18 h. TLC indicated complete consumption of the sulfone to a more polar compound. The mixture was concentrated under vacuum and the compound purified by flash chromatography (EtOAc/ pet. spirits 8:2) to give a pale yellow solid (79.6 mg, 80%), m.p. 180-184°C. ¹H NMR (CD₃OD, 400 MHz) δ 1.29-2.15 (10 H, m), 3.56 (2 H, m), 3.74 (2 H, m), 4.58 (1 H, m), 6.37 (1 H, d, *J* = 4 Hz), 7.06, (2 H, m), 7.39 (2 H, m), 8.00 (1 H, s), 8.14 (1 H, d, *J* = 4 Hz); ¹³C NMR (CD₃OD, 101 MHz) δ 26.3, 26.9 44.7, 49.5, 57.1, 61.9, 112.7, 116.1, 116.35, 123.8, 126.8, 131.3 (d, *J*_{C-F} = 8.1 Hz), 131.7 (d, *J*_{C-F} = 3.1 Hz), 137.4, 141.6, 159.5, 160.0, 163.8 (d, *J*_{C-F} = 246 Hz), 164.0. HRMS (ESI⁺) calcd for C₂₁H₂₅FN₅O (M+H) 382.2043. Found 382.2040.

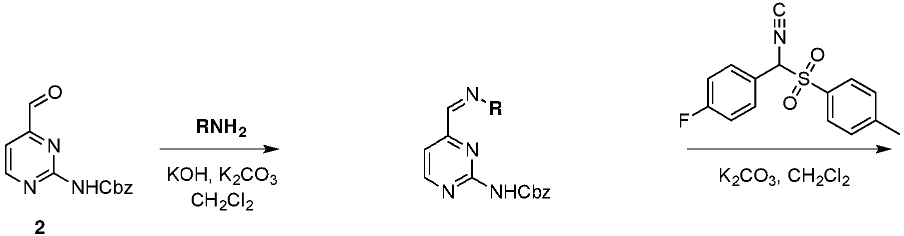

### RNH₂

| | |
|---|---|
| cyclopentylamine | **85 *** |
| cyclopentylmethylamine | **88 *** |

| | |
|---|---|
| ** not isolated* | |

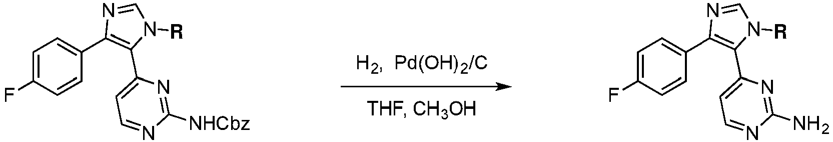

### R

| | |
|---|---|
| cyclopentyl | **86** ** |
| cyclopentylmethyl | **89** ** |
| | *** impure* |

### R

| | |
|---|---|
| cyclopentyl | **87** |
| cyclopentylmethyl | **90** (23%) |

### Benzyl-(4-((cyclopentylimino)methyl)pyrimidin-2-yl)carbamate

45% aq. KOH (0.449 g, 8.00 mmol) was added to an ice-cold solution of crude aldehyde **2** (0.070g, 0.274 mmol) in aq. HCl (2 ml, 8.00 mmol), while the temperature was maintained below 15 °C. To the neutralized solution, CH₂Cl₂ (5 ml) and K₂CO₃ (0.045 g, 0.328 mmol) were added followed by cyclopentylamine (54.0 µL, 0.547 mmol). The reaction mixture was gradually warmed to room temperature and stirring was continued for 18 h. ¹H NMR analysis of the reaction mixture showed complete consumption of the aldehyde. The crude material was used in the next step without isolation.

### Benzyl (4-(1-(cyclopentyl)-4-(4-fluorophenyl)-1H-imidazol-5-yl)pyrimidin-2-yl)carbamate

A mixture of α-(*p*-toluenesulfonyl)-4-fluorobenzylisonitrile (0.095 g, 0.329 mmol), imine (0.088 g, 0.274 mmol) and aq. 20% K₂CO₃ (0.255 mL, 0.329 mmol) in CH₂Cl₂ (10 ml) was stirred at r.t. for 60 h. The reaction mixture was diluted with CH₂Cl₂, and washed with water, dried (MgSO₄), filtered and concentrated. Flash chromatography of the residue (EtOAc/pet. spirits 3:2) afforded the cyclopentyl carbamate (0.103 mg, 82%).

### 4-(1-(cyclopentyl)-4-(4-fluorophenyl)-1H-imidazol-5-yl)pyrimidin-2-amine

Add 20% wt Pd(OH)₂/C to a solution of the cyclopentyl carbamate in THF/MeOH 10:1. Stir the resulting mixture under an atmosphere of hydrogen at 200 psi for 2 h. Filter the mixture through celite, concentrate and purify by flash chromatography (CHCl₃/MeOH/NEt₃ 97:2:1) to afford a colourless solid.

### Benzyl-(4-((cyclopentylmethylimino)methyl)pyrimidin-2-yl)carbamate

45% aq. KOH (0.449 g, 8.00 mmol) was added to an ice-cold solution of crude aldehyde **2** (0.147g, 0.571 mmol) in aq. HCl (2 ml, 8.00 mmol), while the temperature was maintained below 15 °C. To the neutralized solution, CH₂Cl₂ (5 ml) and K₂CO₃ (0.095 g, 0.685 mmol) were added followed by cyclopentylmethylamine (107 µL, 0.113 mmol). The reaction mixture was gradually warmed to room temperature and stirring was continued for 18 h. ¹H NMR analysis of the reaction mixture showed complete consumption of the aldehyde. The crude material was used in the next step without isolation.

### Benzyl (4-(1-(cyclopentylmethyl)-4-(4-fluorophenyl)-1H-imidazol-5-yl)pyrimidin-2-yl)carbamate

Stir a mixture of α-(*p*-toluenesulfonyl)-4-fluorobenzylisonitrile (0.198 g, 0.5685 mmol), imine (0.193 g, 0.571 mmol) and aq. 20% K₂CO₃ (0.475 mL, 0.685 mmol) in CH₂Cl₂ (10 ml) at r.t. for 72 h. Dilute the reaction mixture with CH₂Cl₂, and wash with water, dry (MgSO₄), filter and concentrate. Flash chromatography of the residue (EtOAc/pet. spirits 3:2) will afford the cyclopentylmethyl carbamate as a colourless solid.

### 4-(1-(cyclopentylmethyl)-4-(4-fluorophenyl)-1H-imidazol-5-yl)pyrimidin-2-amine

Add 20% wt Pd(OH)₂/C to a solution of the cyclopentylmethyl carbamate in THF/MeOH 10:1. Stir the resulting mixture under an atmosphere of hydrogen at 200 psi for 2 h. Filter the mixture through celite, concentrate and purify by flash chromatography (CHCl₃/MeOH/NEt₃ 97:2:1) to afford a colourless solid.
1 W. C. Still, M. Kahn and A. M. Mitra, *J. Org. Chem*., 1978, **43**, 2923.
2 A. B. Pangborn, M. A. Giardello, R. H. Grubbs, R. K. Rosen and F. J. Timmers, *Organometallics,* 1996, **15,** 1518.

## Claims

1. A compound of formula (I), or a salt, solvate, or polymorph thereof: 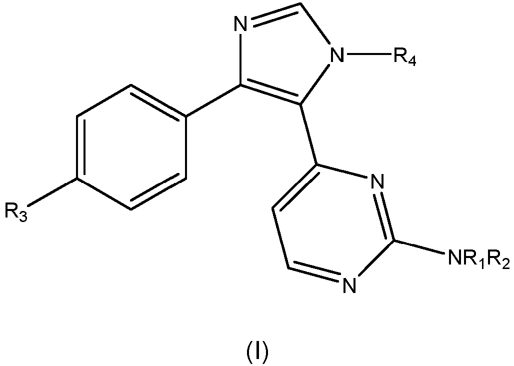 wherein:
R₁ is selected from the group consisting of:
C₂₋₆alkynyl, optionally substituted with one substituent independently selected from the group consisting of: C₁₋₆haloalkyl, preferably C₁₋₆ perfluoroalkyl, C₁₋₆ haloalkoxy, C₁₋₆hydroxyalkyl, C₃₋₇heterocyclyl, C₃₋₇cycloalkyl, C₁₋₆alkoxy, C₁₋₆alkylsulfanyl, C₁₋₆alkylsulfenyl, C₁₋₆alkylsulfonyl, C₁₋₆alkylsulfonylamino, arylsulfonylamino, alkylcarboxy, alkylcarboxyamide, oxo, hydroxy, mercapto, amino, carboxy, carbamoyl, aryl, aryloxy, heteroaryl, aminosulfonyl, aroyl, aroylamino, heteroaroyl, aroyloxy, heteroaroyloxy, alkoxycarbonyl, nitro, cyano, halo, and ureido;
C₁alkylC₆aryl, optionally substituted with one substituent independently selected from the group consisting of: C₁₋₆haloalkyl, preferably C₁₋₆ perfluoroalkyl, C₁₋₆ haloalkoxy, C₁₋₆hydroxyalkyl, C₃₋₇heterocyclyl, C₃₋₇cycloalkyl, C₁₋₆alkoxy, C₁₋₆alkylsulfanyl, C₁₋₆alkylsulfenyl, C₁₋₆alkylsulfonyl, C₁₋₆alkylsulfonylamino, arylsulfonylamino, alkylcarboxy, alkylcarboxyamide, hydroxy, mercapto, amino, carboxy, carbamoyl, aryl, aryloxy, heteroaryl, aminosulfonyl, aroyl, aroylamino, heteroaroyl, aroyloxy, heteroaroyloxy, alkoxycarbonyl, nitro, halo, and ureido;
C₃₋₆cycloalkyl, optionally substituted with one substituent independently selected from the group consisting of: C₁₋₆alkyl, C₁₋₆haloalkyl, preferably C₁₋₆ perfluoroalkyl, C₁₋₆ haloalkoxy, C₁₋₆hydroxyalkyl, C₃₋₇heterocyclyl, C₁₋₆alkoxy, C₁₋₆alkylsulfanyl, C₁₋₆alkylsulfenyl, C₁₋₆alkylsulfonyl, C₁₋₆alkylsulfonylamino, arylsulfonylamino, alkylcarboxy, alkylcarboxyamide, oxo, hydroxy, mercapto, amino, carboxy, carbamoyl, aryl, aryloxy, heteroaryl, aminosulfonyl, aroyl, aroylamino, heteroaroyl, aroyloxy, heteroaroyloxy, alkoxycarbonyl, nitro, cyano, halo, and ureido; and
C₃₋₅heterocyclyl, optionally substituted with one substituent independently selected from the group consisting of: C₁₋₆alkyl, C₁₋₆haloalkyl, preferably C₁₋₆ perfluoroalkyl, C₁₋₆ haloalkoxy, C₁₋₆hydroxyalkyl, C₃₋₇cycloalkyl, C₁₋₆alkoxy, C₁₋₆alkylsulfanyl, C₁₋₆alkylsulfenyl, C₁₋₆alkylsulfonyl, C₁₋₆alkylsulfonylamino, arylsulfonyloamino, alkylcarboxy, alkylcarboxyamide, oxo, hydroxy, mercapto, amino, carboxy, carbamoyl, aryl, aryloxy, heteroaryl, aminosulfonyl, aroyl, aroylamino, heteroaroyl, aroyloxy, heteroaroyloxy, alkoxycarbonyl, nitro, cyano, halo, and ureido;
R₂ is H;
R₃ is selected from the group consisting of F, Cl, Br, I, CH₃, OCH₃, OCF₂H, OCF₃, CO₂H, CO₂C₁₋₁₀alkyl;
R₄ is C₃₋₇cycloalkyl, optionally substituted with one substituent independently selected from the group consisting of: C₁₋₆alkyl, C₁₋₆haloalkyl, preferably C₁₋₆ perfluoroalkyl, C₁₋₆ haloalkoxy, C₁₋₆hydroxyalkyl, C₃₋₇heterocyclyl, C₁₋₆alkoxy, C₁₋₆alkylsulfanyl, C₁₋₆alkylsulfenyl, C₁₋₆alkylsulfonyl, C₁₋₆alkylsulfonylamino, arylsulfonylamino, alkylcarboxy, alkylcarboxyamide, oxo, hydroxy, mercapto, amino, carboxy, carbamoyl, aryl, aryloxy, heteroaryl, aminosulfonyl, aroyl, aroylamino, heteroaroyl, aroyloxy, heteroaroyloxy, alkoxycarbonyl, nitro, cyano, halo, and ureido.

2. A compound according to claim 1, or a salt, solvate, or polymorph thereof wherein R₁ is C₃₋₆cycloalkyl.

3. A compound according to claim 1 or 2, or a salt, solvate, or polymorph thereof wherein R₁ is C₆cycloalkyl.

4. A compound according to claim 1 or 2, or a salt, solvate, or polymorph thereof wherein R₁ is C₄cycloalkyl.

5. A compound according to claim 1, or a salt, solvate, or polymorph thereof wherein R₁ is C₁alkylC₆aryl.

6. A compound according to claim 1, or a salt, solvate or polymorph thereof wherein R₁ is C₂₋₆alkynyl.

7. A compound according to claim 1, or a salt, solvate or polymorph thereof wherein R₁ is C₃₋₅heterocyclyl.

8. A compound according to any one of the preceding claims, or a salt, solvate, or polymorph thereof wherein R₃ is selected from CH₃ and halo, preferably F or Cl.

9. A compound according to any one of the preceding claims, or a salt, solvate, or polymorph thereof wherein R₄ is C₃₋₇cycloalkyl.

10. A compound according to any one of claims 1 to 8, or a salt, solvate, or polymorph thereof wherein R₄ is C₄cycloalkyl.

11. A compound according to any one of claims 1 to 8, or a salt, solvate, or polymorph thereof wherein R₄ is C₆cycloalkyl.

12. A compound according to claim 1, or a salt, solvate, or polymorph thereof wherein:
R₁ is selected from the group consisting of C₂₋₆alkynyl, and C₃₋₅heterocyclyl;
R₂ is H;
R₃ is selected from the group consisting of F, CI, Br, I and CH₃;
R₄ is C₃₋₇cycloalkyl;
wherein each of R₁ and R₄ is optionally substituted.

13. A compound according to claim 1, or a salt, solvate or polymorph thereof wherein the compound is selected from the group consisting of: 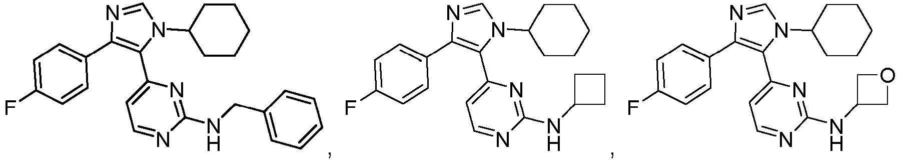 and

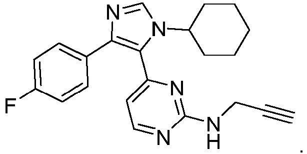

14. A composition comprising a compound of formula (I) according to any one of claims 1 to 13, or a salt, solvate, or polymorph thereof, and a pharmaceutically acceptable excipient.

15. A compound of formula (I) according to any one of claims 1 to 13, or a salt, solvate, or polymorph thereof, or the composition according to claim 14, for use in the treatment or prevention of a respiratory disease in a subject.

## Patentansprüche

1. Verbindung der Formel (I) oder ein Salz, Solvat oder Polymorph davon: 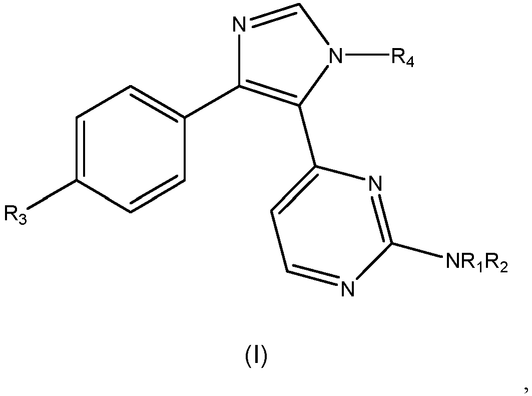 wobei:
R₁ aus der Gruppe ausgewählt ist, die aus Folgendem besteht:
C₂₋₆-Alkinyl, optional substituiert mit einem Substituenten, unabhängig ausgewählt aus der Gruppe bestehend aus: C₁₋₆-Halogenalkyl, vorzugsweise C₁₋₆-Perfluoralkyl, C₁₋₆-Halogenalkoxy, C₁₋₆-Hydroxyalkyl, C₃₋₇-Heterocyclyl, C₃₋₇-Cycloalkyl, C₁₋₆-Alkoxy, C₁₋₆-Alkylsulfanyl, C₁₋₆-Alkylsulfenyl, C₁₋₆-Alkylsulfonyl, C₁₋₆-Alkylsulfonylamino, Arylsulfonylamino, Alkylcarboxy, Alkylcarboxyamid, Oxo, Hydroxy, Mercapto, Amino, Carboxy, Carbamoyl, Aryl, Aryloxy, Heteroaryl, Aminosulfonyl, Aroyl, Aroylamino, Heteroaroyl, Aroyloxy, Heteroaroyloxy, Alkoxycarbonyl, Nitro, Cyano, Halo und Ureido;
C₁-Alkyl-C₆-Aryl, optional substituiert mit einem Substituenten, unabhängig ausgewählt aus der Gruppe bestehend aus: C₁₋₆-Halogenalkyl, vorzugsweise C₁₋₆-Perfluoralkyl, C₁₋₆-Halogenalkoxy, C₁₋₆-Hydroxyalkyl, C₃₋₇-Heterocyclyl, C₃₋₇-Cycloalkyl, C₁₋₆-Alkoxy, C₁₋₆-Alkylsulfanyl, C₁₋₆-Alkylsulfenyl, C₁₋₆-Alkylsulfonyl, C₁₋₆-Alkylsulfonylamino, Arylsulfonylamino, Alkylcarboxy, Alkylcarboxyamid, Hydroxy, Mercapto, Amino, Carboxy, Carbamoyl, Aryl, Aryloxy, Heteroaryl, Aminosulfonyl, Aroyl, Aroylamino, Heteroaroyl, Aroyloxy, Heteroaroyloxy, Alkoxycarbonyl, Nitro, Halo und Ureido;
C₃₋₆-Cycloalkyl, optional substituiert mit einem Substituenten, unabhängig ausgewählt aus der Gruppe bestehend aus: C₁₋₆-Alkyl, C₁₋₆-Halogenalkyl, vorzugsweise C₁₋₆-Perfluoralkyl, C₁₋₆-Halogenalkoxy, C₁₋₆-Hydroxyalkyl, C₃₋₇-Heterocyclyl, C₁₋₆-Alkoxy, C₁₋₆-Alkylsulfanyl, C₁₋₆-Alkylsulfenyl, C₁₋₆-Alkylsulfonyl, C₁₋₆-Alkylsulfonylamino, Arylsulfonylamino, Alkylcarboxy, Alkylcarboxyamid, Oxo, Hydroxy, Mercapto, Amino, Carboxy, Carbamoyl, Aryl, Aryloxy, Heteroaryl, Aminosulfonyl, Aroyl, Aroylamino, Heteroaroyl, Aroyloxy, Heteroaroyloxy, Alkoxycarbonyl, Nitro, Cyano, Halo und Ureido; und
C₃₋₅-Heterocyclyl, optional substituiert mit einem Substituenten, unabhängig ausgewählt aus der Gruppe bestehend aus: C₁₋₆-Alkyl, C₁₋₆-Halogenalkyl, vorzugsweise C₁₋₆-Perfluoralkyl, C₁₋₆-Halogenalkoxy, C₁₋₆-Hydroxyalkyl, C₃₋₇-Cycloalkyl, C₁₋₆-Alkoxy, C₁₋₆-Alkylsulfanyl, C₁₋₆-Alkylsulfenyl, C₁₋₆-Alkylsulfonyl, C₁₋₆-Alkylsulfonylamino, Arylsulfonyloamino, Alkylcarboxy, Alkylcarboxyamid, Oxo, Hydroxy, Mercapto, Amino, Carboxy, Carbamoyl, Aryl, Aryloxy, Heteroaryl, Aminosulfonyl, Aroyl, Aroylamino, Heteroaroyl, Aroyloxy, Heteroaroyloxy, Alkoxycarbonyl, Nitro, Cyano, Halo und Ureido;
R₂ H ist;
R₃ ausgewählt ist aus der Gruppe bestehend aus F, Cl, Br, I, CH₃, OCH₃, OCF₂H, OCF₃, CO₂H, CO₂C₁₋₁₀-Alkyl;
R₄ C₃₋₇-Cycloalkyl ist, optional substituiert mit einem Substituenten, unabhängig ausgewählt aus der Gruppe bestehend aus: C₁₋₆-Alkyl, C₁₋₆-Halogenalkyl, vorzugsweise C₁₋₆-Perfluoralkyl, C₁₋₆-Halogenalkoxy, C₁₋₆-Hydroxyalkyl, C₃₋₇-Heterocyclyl, C₁₋₆-Alkoxy, C₁₋₆-Alkylsulfanyl, C₁₋₆-Alkylsulfenyl, C₁₋₆-Alkylsulfonyl, C₁₋₆-Alkylsulfonylamino, Arylsulfonylamino, Alkylcarboxy, Alkylcarboxyamid, Oxo, Hydroxy, Mercapto, Amino, Carboxy, Carbamoyl, Aryl, Aryloxy, Heteroaryl, Aminosulfonyl, Aroyl, Aroylamino, Heteroaroyl, Aroyloxy, Heteroaroyloxy, Alkoxycarbonyl, Nitro, Cyano, Halo und Ureido.

2. Verbindung nach Anspruch 1 oder ein Salz, Solvat oder Polymorph davon, wobei R₁ C₃₋₆-Cycloalkyl ist.

3. Verbindung nach Anspruch 1 oder 2 oder ein Salz, Solvat oder Polymorph davon, wobei R₁ C₆-Cycloalkyl ist.

4. Verbindung nach Anspruch 1 oder 2 oder ein Salz, Solvat oder Polymorph davon, wobei R₁ C₄-Cycloalkyl ist.

5. Verbindung nach Anspruch 1 oder ein Salz, Solvat oder Polymorph davon, wobei R₁ C₁-Alkyl-C₆-Aryl ist.

6. Verbindung nach Anspruch 1 oder ein Salz, Solvat oder Polymorph davon, wobei R₁ C₂₋₆-Alkinyl ist.

7. Verbindung nach Anspruch 1 oder ein Salz, Solvat oder Polymorph davon, wobei R₁ C₃₋₅-Heterocyclyl ist.

8. Verbindung nach einem der vorhergehenden Ansprüche oder ein Salz, Solvat oder Polymorph davon, wobei R₃ ausgewählt ist aus CH₃ und Halo, vorzugsweise F oder Cl.

9. Verbindung nach einem der vorhergehenden Ansprüche oder ein Salz, Solvat oder Polymorph davon, wobei R₄ C₃₋₇-Cycloalkyl ist.

10. Verbindung nach einem der Ansprüche 1 bis 8 oder ein Salz, Solvat oder Polymorph davon, wobei R₄ C₄-Cycloalkyl ist.

11. Verbindung nach einem der Ansprüche 1 bis 8 oder ein Salz, Solvat oder Polymorph davon, wobei R₄ C₆-Cycloalkyl ist.

12. Verbindung nach Anspruch 1 oder ein Salz, Solvat oder Polymorph davon, wobei:
R₁ ausgewählt ist aus der Gruppe bestehend aus C₂₋₆-Alkinyl und C₃₋₅-Heterocyclyl;
R₂ H ist;
R₃ ausgewählt ist aus der Gruppe bestehend aus F, Cl, Br, I und CH₃;
R₄ C₃₋₇-Cycloalkyl ist;
wobei jedes von R₁ und R₄ optional substituiert ist.

13. Verbindung nach Anspruch 1 oder ein Salz, Solvat oder Polymorph davon, wobei die Verbindung aus der Gruppe ausgewählt ist, die aus Folgendem besteht: 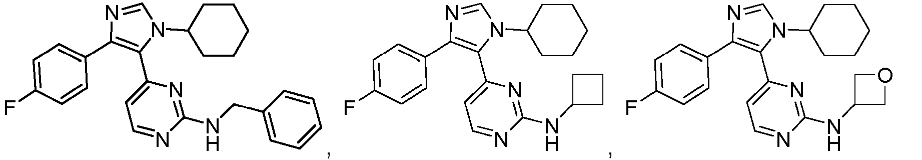 und

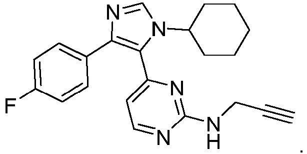

14. Zusammensetzung, umfassend eine Verbindung der Formel (I) nach einem der Ansprüche 1 bis 13 oder ein Salz, Solvat oder Polymorph davon und einen pharmazeutisch unbedenklichen Hilfsstoff.

15. Verbindung der Formel (I) nach einem der Ansprüche 1 bis 13 oder ein Salz, Solvat oder Polymorph davon oder die Zusammensetzung nach Anspruch 14 zur Verwendung bei der Behandlung oder Prävention einer Atemwegserkrankung bei einem Subjekt.

## Revendications

1. Composé de formule (I), ou sel, solvate ou polymorphe de celui-ci : 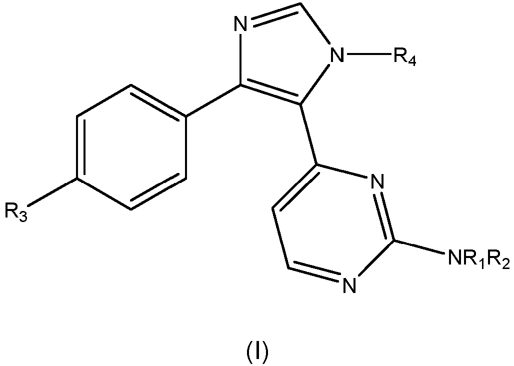 dans lequel :
R₁ est choisi dans le groupe constitué de :
un alcynyle en C₂₋₆, éventuellement substitué par un substituant indépendamment choisi dans le groupe constitué de : un halogénoalkyle en C₁₋₆, de préférence un perfluoroalkyle en C₁₋₆, un halogénoalcoxy en C₁₋₆, un hydroxyalkyle en C₁₋₆, un hétérocyclyle en C₃₋₇, un cycloalkyle en C₃₋₇, un alcoxy en C₁₋₆, un alkylsulfanyle en C₁₋₆, un alkylsulfényle en C₁₋₆, un alkylsulfonyle en C₁₋₆, un alkylsulfonylamino en C₁₋₆, un arylsulfonylamino, un alkylcarboxy, un alkylcarboxyamide, un oxo, un hydroxy, un mercapto, un amino, un carboxy, un carbamoyle, un aryle, un aryloxy, un hétéroaryle, un aminosulfonyle, un aroyle, un aroylamino, un hétéroaroyle, un aroyloxy, un hétéroaroyloxy, un alcoxycarbonyle, un nitro, un cyano, un halogéno et un uréido ;
un alkyle en Ci-aryle en C₆, éventuellement substitué par un substituant indépendamment choisi dans le groupe constitué de : un halogénoalkyle en C₁₋₆, de préférence un perfluoroalkyle en C₁₋₆, un halogénoalcoxy en C₁₋₆, un hydroxyalkyle en C₁₋₆, un hétérocyclyle en C₃₋₇, un cycloalkyle en C₃₋₇, un alcoxy en C₁₋₆, un alkylsulfanyle en C₁₋₆, un alkylsulfényle en C₁₋₆, un alkylsulfonyle en C₁₋₆, un alkylsulfonylamino en C₁₋₆, un arylsulfonylamino, un alkylcarboxy, un alkylcarboxyamide, un hydroxy, un mercapto, un amino, un carboxy, un carbamoyle, un aryle, un aryloxy, un hétéroaryle, un aminosulfonyle, un aroyle, un aroylamino, un hétéroaroyle, un aroyloxy, un hétéroaroyloxy, un alcoxycarbonyle, un nitro, un halogéno et un uréido ;
un cycloalkyle en C₃₋₆, éventuellement substitué par un substituant indépendamment choisi dans le groupe constitué de : un alkyle en C₁₋₆, un halogénoalkyle en C₁₋₆, de préférence un perfluoroalkyle en C₁₋₆, un halogénoalcoxy en C₁₋₆, un hydroxyalkyle en C₁₋₆, un hétérocyclyle en C₃₋₇, un alcoxy en C₁₋₆, un alkylsulfanyle en C₁₋₆, un alkylsulfényle en C₁₋₆, un alkylsulfonyle en C₁₋₆, un alkylsulfonylamino en C₁₋₆, un arylsulfonylamino, un alkylcarboxy, un alkylcarboxyamide, un oxo, un hydroxy, un mercapto, un amino, un carboxy, un carbamoyle, un aryle, un aryloxy, un hétéroaryle, un aminosulfonyle, un aroyle, un aroylamino, un hétéroaroyle, un aroyloxy, un hétéroaroyloxy, un alcoxycarbonyle, un nitro, un cyano, un halogéno et un uréido ; et
un hétérocyclyle en C₃₋₅, éventuellement substitué par un substituant indépendamment choisi dans le groupe constitué de : un alkyle en C₁₋₆, un halogénoalkyle en C₁₋₆, de préférence un perfluoroalkyle en C₁₋₆, un halogénoalcoxy en C₁₋₆, un hydroxyalkyle en C₁₋₆, un cycloalkyle en C₃₋₇, un alcoxy en C₁₋₆, un alkylsulfanyle en C₁₋₆, un alkylsulfényle en C₁₋₆, un alkylsulfonyle en C₁₋₆, un alkylsulfonylamino en C₁₋₆, un arylsulfonylamino, un alkylcarboxy, un alkylcarboxyamide, un oxo, un hydroxy, un mercapto, un amino, un carboxy, un carbamoyle, un aryle, un aryloxy, un hétéroaryle, un aminosulfonyle, un aroyle, un aroylamino, un hétéroaroyle, un aroyloxy, un hétéroaroyloxy, un alcoxycarbonyle, un nitro, un cyano, un halogéno et un uréido ;
R₂ est H ;
R₃ est choisi dans le groupe constitué de F, Cl, Br, I, CH₃, OCH₃, OCF₂H, OCF₃, CO₂H, CO₂-alkyle en C₁₋₁₀ ;
R₄ est un cycloalkyle en C₃₋₇, éventuellement substitué par un substituant indépendamment choisi dans le groupe constitué de : un alkyle en C₁₋₆, un halogénoalkyle en C₁₋₆, de préférence un perfluoroalkyle en C₁₋₆, un halogénoalcoxy en C₁₋₆, un hydroxyalkyle en C₁₋₆, un hétérocyclyle en C₃₋₇, un alcoxy en C₁₋₆, un alkylsulfanyle en C₁₋₆, un alkylsulfényle en C₁₋₆, un alkylsulfonyle en C₁₋₆, un alkylsulfonylamino en C₁₋₆, un arylsulfonylamino, un alkylcarboxy, un alkylcarboxyamide, un oxo, un hydroxy, un mercapto, un amino, un carboxy, un carbamoyle, un aryle, un aryloxy, un hétéroaryle, un aminosulfonyle, un aroyle, un aroylamino, un hétéroaroyle, un aroyloxy, un hétéroaroyloxy, un alcoxycarbonyle, un nitro, un cyano, un halogéno et un uréido.

2. Composé selon la revendication 1, ou sel, solvate ou polymorphe de celui-ci, dans lequel R₁ est un cycloalkyle en C₃₋₆.

3. Composé selon l'une des revendications 1 ou 2, ou sel, solvate ou polymorphe de celui-ci, dans lequel R₁ est un cycloalkyle en C₆.

4. Composé selon l'une des revendications 1 ou 2, ou sel, solvate ou polymorphe de celui-ci, dans lequel R₁ est un cycloalkyle en C₄.

5. Composé selon la revendication 1, ou sel, solvate ou polymorphe de celui-ci, dans lequel R₁ est un alkyle en C₁-aryle en C₆.

6. Composé selon la revendication 1, ou sel, solvate ou polymorphe de celui-ci, dans lequel R₁ est un alcynyle en C₂₋₆.

7. Composé selon la revendication 1, ou sel, solvate ou polymorphe de celui-ci, dans lequel R₁ est un hétérocyclyle en C₃₋₅.

8. Composé selon l'une quelconque des revendications précédentes, ou sel, solvate ou polymorphe de celui-ci, dans lequel R₃ est choisi parmi CH₃ et un halogéno, de préférence F ou Cl.

9. Composé selon l'une quelconque des revendications précédentes, ou sel, solvate ou polymorphe de celui-ci, dans lequel R₄ est un cycloalkyle en C₃₋₇.

10. Composé selon l'une quelconque des revendications 1 à 8, ou sel, solvate ou polymorphe de celui-ci, dans lequel R₄ est un cycloalkyle en C₄.

11. Composé selon l'une quelconque des revendications 1 à 8, ou sel, solvate ou polymorphe de celui-ci, dans lequel R₄ est un cycloalkyle en C₆.

12. Composé selon la revendication 1, ou sel, solvate ou polymorphe de celui-ci, dans lequel :
R₁ est choisi dans le groupe constitué d'un alcynyle en C₂₋₆ et d'un hétérocyclyle en C₃₋₅ ;
R₂ est H ;
R₃ est choisi dans le groupe constitué de F, Cl, Br, I et CH₃ ;
R₄ est un cycloalkyle en C₃₋₇ ;
dans lequel chacun de R₁ et R₄ est éventuellement substitué.

13. Composé selon la revendication 1, ou sel, solvate ou polymorphe de celui-ci, ledit composé étant choisi dans le groupe constitué de : 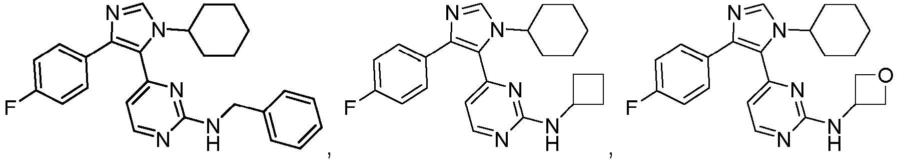 et

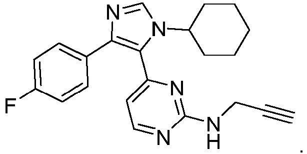

14. Composition comprenant un composé de formule (I) selon l'une quelconque des revendications 1 à 13, ou un sel, solvate ou polymorphe de celui-ci, et un excipient acceptable pharmaceutiquement.

15. Composé de formule (I) selon l'une quelconque des revendications 1 à 13, ou sel, solvate ou polymorphe de celui-ci, ou composition selon la revendication 14, destiné(e) à être utilisé(e) dans le traitement ou la prévention d'une maladie respiratoire chez un sujet.
